# EUROPEAN PATENT APPLICATION

(11) **EP 4 223 328 A2**
(43) Date of publication of application: **09.08.2023**
(21) Application number: 23164800.7
(22) Date of filing: 26.04.2017
(51) Int. Cl.: A61L 27/46, A61L 27/52, A61L 27/56, A61L 27/58, A61L 24/00, A61P 19/02, A61P 19/08

(54) **METHODS AND COMPOSITIONS FOR THE TREATMENT OF DEGENERATE BONE**

(30) Priority: 27.04.2016 US 201662328313 P
(62) Divisional of application: 17722590.1
(71) Applicant: Anika Therapeutics, Inc., Bedford, MA 01730 (US)
(72) Inventor: AHN, Edward S., Dover, MA 02030 (US); LIN, Chia-En, Concord, MA 01742 (US); WHITE, Colin D., North Reading, MA 01864 (US)
(74) Representative: Harris, Jennifer Lucy

(57) **Abstract**

The present disclosure relates to methods and compositions for the treatment of degenerate bone in a patient. In some embodiments, the methods and compositions disclosed herein are useful in the treatment, prevention, or in delaying the progression of a bone disease linked to bone degeneration, such as osteoarthritis ("OA"), rheumatoid arthritis, and avascular necrosis.

## Description

### CROSS REFERENCE TO RELATED APPLICATIONS

This application claims the benefit of priority of U.S. Provisional Application No. 62/328,313, filed April 27, 2016, the contents of which are hereby incorporated by reference.

### FIELD OF INVENTION

The present disclosure relates to methods and compositions for the treatment of degenerate bone in a patient. In some embodiments, the methods and compositions disclosed herein are useful in the treatment, prevention, or in delaying the progression of a bone disease linked to bone degeneration, such as osteoarthritis ("OA"), rheumatoid arthritis, and avascular necrosis.

### BACKGROUND

Areas of degenerate bone can lead to a host of issues for patients. For example, the onset and progression of symptomatic OA, rheumatoid arthritis, and avascular necrosis are thought to be linked to areas of degenerate bone in or adjacent to the affected area. While the etiologies of these diseases are different, each is often associated with significant pain and loss of function. Slowing, arresting, and repairing bone degeneration can reduce pain and slow, prevent, or reverse disease progression.

One example of a pathology thought to be linked to degenerate bone is OA. Osteoarthritis is the most common form of arthritis, affecting the hands, knees, hips, spine, and other joints, and is a leading cause of lost productivity, estimated to affect approximately 27 million Americans. Arthritis Foundation: What is Osteoarthritis, available at http://www.arthritis.org/about-arthritis/types/osteoarthritis/what-is-osteoarthritis.php (last visited April 12, 2017), the contents of which are incorporated herein by reference in their entirety. OA results in damage to cartilage in the joint, pain, swelling, and movement problems. As OA progresses, bone in the region begins to degenerate, resulting in bone spurs and further inflammation. The etiology of OA is not fully understood, but is thought to include causes such as trauma (*e.g.*, fractures), degeneration, inflammation, ischemia, congenital joint abnormalities, metabolic defects, endocrine and neuropathic diseases, and infections.

Patients who initially present with painful bone disease linked to bone degeneration are usually treated non-surgically. Non-surgical treatments are modestly effective at temporarily relieving pain, but are not risk free. For example, pharmacologic intervention (*e.g*., non-steroidal anti-inflammatory drugs) has been reported to be associated with significant complications, such as gastric ulcers, strokes and heart attacks. Generally speaking, non-surgical interventions are only efficacious for alleviating the pain caused by bone disease and do not slow or prevent disease progression.

When patients fail non-surgical treatment for bone disease, surgical intervention, whether invasive or minimally invasive, is often recommended. Current invasive surgical approaches aim to alter the biomechanical forces on areas of the affected joint, either by shifting weight from an area of damaged cartilage to an area of healthy cartilage by osteotomy or other means, or by completely replacing the joint and restoring biomechanical function with the use of joint replacement hardware. Minimally invasive surgical approaches include the treatment of areas of degenerate bone, such as bone marrow lesions ("BMLs"), whose presence has been associated with the onset and progression of OA. *See, e.g.,* Sharkey, P. F. et al. Am. J. Orthop. (Belle Mead NJ) 2012, 41(9), 413-17, the contents of which are incorporated herein by reference in their entirety. Minimally invasive prior art treatments for bone degeneration include the injection of a variety of calcium phosphate cements ("CPCs") into the area of degenerate bone, such that the CPCs biomechanically stabilize the joint. *See, e.g.,* Hisatome, T. et al., J. Biomed. Mater. Res. 2002, 59(3), 490-98 (creating subchondral access into the femoral condyle while preserving articular cartilage by using an augmentation material such as a CPC because of its mechanical strength and using the CPC to fill a large defect to prevent collapse and provide the necessary support to preserve the articular cartilage); Chatterjee, D. et al. Clin. Orthop. Relat. Res. 2015, 473(7), 2334-42 (disclosing injection of a CPC with a pore size of 150-500 µm into the subchondral bone in order to improve its structural integrity and biomechanical strength), the contents of all of the foregoing of which are incorporated herein by reference in their entireties. Other prior art CPCs have been used for fracture fixation or to fill bony voids or gaps of the skeletal system (*e.g.,* extremities, craniofacial, spine, and pelvis). *See, e.g.,* Nishizuka, T. et al. PLoS One 2014, 9(8), e104603, the contents of which are incorporated herein by reference in their entirety.

Importantly, these prior art treatments have significant drawbacks when used to treat bone diseases such as OA. For example, invasive surgical approaches carry considerable risk, including infection, deep vein thrombosis, and - in extreme cases - death. Moreover, total joint replacements are effective for only approximately 20 years. Prior art minimally invasive treatments for bone disease have also been shown to be ineffective in patients with more advanced bone degeneration. *See, e.g.,* Chatterjee, D. et al. Clin. Orthop. Relat. Res. 2015, 473(1), 2334-42, the contents of which are incorporated herein by reference in their entirety. Finally, use of both invasive and non-invasive prior art treatments that provide for biomechanical stabilization of bone result in significant pain post-operatively.

Furthermore, prior art treatments that provide for biomechanical stabilization of bone also do not address the causative factors of bone disease characterized by bone degeneration. During the onset and progression of bone disease, bone in the affected area is subject to insult by inflammatory and/or non-inflammatory mediators. These mediators emanate from the joint space and pass through channels in the subchondral bone plate that link the joint space and the affected area of bone. The influx of these mediators causes degeneration of the bone and fluid accumulation within the weakened trabecular structure, and results in intense pain due to activation of nociceptors in the subchondral bone. The insult to the bone in the affected area is worsened in more advanced cases of bone disease because of the destruction of at least a portion of the articular cartilage, which in turn results in an increased flow of mediators from the joint space, through the cortical bone plate, and into the affected area of bone.

CPCs used in the treatment of bone disease require several features in order to effectively treat the affected area of bone, including injectability, flowability, settability, cohesion, and adhesion to bone. Unfortunately, conventional CPCs typically are lacking with respect to one or more of the desired characteristics, which has hindered the development of CPCs capable of being administered to a desired anatomical location in a minimally invasive manner. CPCs are typically formed by mixing a solid and a liquid to obtain a paste suitable for injection which later sets and cures after administration into the affected area of bone. Prior art CPCs are designed to have a high compressive strength and elastic modulus so as to provide biomechanical stabilization to the affected area of bone. Such CPCs are generally made with high solid-to-liquid ratios, which results in high compressive strengths and elastic moduli and generally lower porosity, but these CPCs offer poor injectability due to the required high injection pressures and poor flowability such that the materials do not adequately fill the space in the affected area. CPCs made with these high solid-to-liquid ratios can also dewater during injection, leaving cement solid in the instrumentation and preventing the CPC from setting and curing *in situ.* Attempts to address these issues by preparing CPCs with lower solid-to-liquid ratios have resulted in poor cohesiveness and a lack of setting and/or curing post-administration due to the hydrophilic nature of the CPC and its tendency to mix with body fluids. Additionally, even when materials made with these lower solid-to-liquid ratios are capable of setting, they do not maintain cohesion or adhesion to bone, such that that they do not remain in the affected area after administration, and instead flow through the porous bone structure.

As a result of these challenges, a very limited number of CPCs are available that have the desired combination of providing biomechanical stability to the affected area while maintaining injectability and flowability to fill the affected area of bone. *See, e.g.,* Subchondroplasty® Procedure AccuFill® Bone Substitute Material (BSM), available at http://subchondroplasty.com/healthcare-professionals-bsm.html (last visited April 18, 2017); *see also* Tofighi, A. et al. J. Biomimetics Biomat. Tissue Eng'g 2009, 2, 39-28, the contents of all of the foregoing of which are incorporated herein by reference in their entireties. Unfortunately, these CPCs, which are used to treat bone disease, cure to form biomaterials with a high degree of porosity, resulting in significant pain post-operatively. *See, e.g.,* Farr, J.; Cohen, S. B. Oper. Tech. Sports Med. 2013, 21(2), 138-43; Eliaz, N.; Metoki, N. Materials 2017, 10, 334, the contents of all of the foregoing of which are incorporated herein by reference in their entireties.

While the prior art has provided certain CPCs that include a carbohydrate, these materials have short setting times or are made with high powder-to-liquid ratios and are accordingly insufficiently intermixable to provide for facile preparation and administration directly from syringes. *See, e.g.,* Pek, Y. S. et al. Biomat. 2009, 30, 822-28; Ahmadzadeh-Asl, S. et al. Adv. Applied Ceramics 2011, 110(6), 340-45; the contents of all of the foregoing of which are incorporated herein by reference in their entireties.

Accordingly, there is a need in the art for more safe and efficacious treatment options that address the underlying causes of bone disease associated with degenerate bone with less risk and side effects than prior art methods.

### SUMMARY OF INVENTION

Methods and compositions for the treatment of degenerate bone in a patient in need thereof are disclosed herein.

In one aspect, disclosed herein is an injectable biomaterial comprising a solid component and a liquid component comprising a carbohydrate, wherein the injectable biomaterial sets and cures to form an apatitic crystal structure after mixing of the solid component and the liquid component.

In another aspect, disclosed herein is a method for making an injectable biomaterial comprising creating the liquid component by providing a liquid solution, adjusting the pH of the liquid solution with a pH adjusting agent, and dissolving the carbohydrate in the liquid solution to form a the liquid component; providing the solid component; and mixing the liquid component and the solid component to form the injectable biomaterial.

In some embodiments, the injectable biomaterial sets over a period of time. In some embodiments, the injectable biomaterial cures over a period of time. In some embodiments, the injectable biomaterial sets prior to completely curing.

In some embodiments, the solid component comprises at least one of a metal phosphate and a metal carbonate. In some embodiments, the solid component comprises a reactive calcium phosphate. In some embodiments, the solid component comprises at least one of α-tricalcium phosphate (Ca₃(PO₄)₂), calcium carbonate (CaCO₃), and monocalcium phosphate monohydrate (Ca(H₂PO₄)₂ H₂O). In some embodiments, the solid component comprises 70-90% alpha tricalcium phosphate, 10-20% calcium carbonate, and 0.5-2% calcium phosphate monobasic monohydrate (mass/mass). In some embodiments, the solid component comprises 80-89% alpha tricalcium phosphate, 11-19% calcium carbonate, and 0.75-1.5% calcium phosphate monobasic monohydrate (mass/mass). In some embodiments, the solid component comprises 82-86% alpha tricalcium phosphate, 13-16% calcium carbonate, and 0.9-1.2% calcium phosphate monobasic monohydrate (mass/mass). In some embodiments, the solid component comprises 84.3% alpha tricalcium phosphate, 14.7% calcium carbonate, and 1.02% calcium phosphate monobasic monohydrate (mass/mass). In some embodiments, the solid component further comprises at least one ionic compound of at least one oligoelement occurring naturally in a human body. In some embodiments, the at least one ionic compound comprises a cation selected from the group consisting of Na⁺, K⁺, Mg²⁺, Ca²⁺, Sr²⁺, H⁺, and mixtures thereof. In further embodiments, the at least one ionic compound comprises an anion selected from the group consisting of PO₄³⁻, HPO₄²⁻, H₂PO₄⁻, P₂O₇⁴⁻, CO₃²⁻, HCO₃⁻, SO₄²⁻, HSO₄⁻, Cl⁻, OH⁻, F⁻, SiO₄⁴⁻, and mixtures hereof.

In some embodiments, the liquid component further comprises a salt. In some embodiments, the salt is a metal salt. In some embodiments, the salt is selected from a phosphate salt, a silicate salt, a chloride salt, a hydroxide salt, and mixtures thereof. In some embodiments, the salt comprises at least one of sodium phosphate dibasic, sodium silicate, sodium chloride, and calcium hydroxide.

In some embodiments, the carbohydrate is selected from the group consisting of dextran, alginate, carboxymethylcellulose, and hyaluronic acid. In some embodiments, the carbohydrate is hyaluronic acid, or an ester, acylurea, acyl isourea, disulfide, or amide thereof. In some embodiments, the hyaluronic acid is selected from the group consisting of hyaluronan, sodium hyaluronate, potassium hyaluronate, magnesium hyaluronate, calcium hyaluronate, ammonium hyaluronate, and combinations thereof. In some embodiments, the hyaluronic acid comprises at least one cross-link. In some embodiments, the hyaluronic acid is derived from bacteria or animals. In some embodiments, the hyaluronic acid comprises a sulfated hyaluronic acid, or ester, acylurea, acyl isourea, carbomer, disulfide, or amide thereof. In some embodiments, the hyaluronic acid comprises an N-sulfated hyaluronic acid, or ester, acylurea, acyl isourea, carbomer, disulfide, or amide thereof. In some embodiments, the hyaluronic acid comprises a hyaluronic ester. In some embodiments, the hyaluronic ester is a esterified in an amount from about 20 to 100%. In some embodiments, the non-esterified hyaluronic acid is salified with an organic or an inorganic base.

In some embodiments, the carbohydrate is water-soluble. In some embodiments, the liquid component is in the form of a hydrogel.

In some embodiments, the carbohydrate is present in the injectable biomaterial at a concentration of about 0.1 to about 100 mg/mL. In some embodiments, the carbohydrate is present in the injectable biomaterial at a concentration of about 0.1 to about 50 mg/mL. In some embodiments, the carbohydrate is present in the injectable biomaterial at a concentration of about 0.1 to about 10 mg/mL. In some embodiments, the carbohydrate is present in the injectable biomaterial at a concentration of about 1 to about 10 mg/mL. In some embodiments, the carbohydrate is present in the injectable biomaterial at a concentration of about 2 to about 10 mg/mL. In some embodiments, the carbohydrate is present in the injectable biomaterial at a concentration of about 4 to about 8 mg/mL. In some embodiments, the carbohydrate is present in the injectable biomaterial at a concentration of about 5 to about 7 mg/mL.

In some embodiments, the carbohydrate has a molecular weight of from about 0.90 × 10⁶ Da to about 1.0 × 10⁷ Da. In some embodiments, the carbohydrate has a molecular weight of from about 0.90 × 10⁶ Da to about 5.0 × 10⁶ Da. In some embodiments, the carbohydrate has a molecular weight of from about 0.90 × 10⁶ Da to about 4.0 × 10⁶ Da. In some embodiments, the carbohydrate has a molecular weight of from about 0.90 × 10⁶ Da to about 3.0 × 10⁶ Da. In some embodiments, the carbohydrate has a molecular weight of from about 1.5 × 10⁶ Da to about 3.0 ×10⁶ Da. In some embodiments, the carbohydrate has a molecular weight of from about 1.7 × 10⁶ Da to about 2.5 × 10⁶ Da. In some embodiments, the carbohydrate is hyaluronic acid having a molecular weight of about 0.90 × 10⁶ Da and is present at a concentration of about 6.0 mg/mL. In some embodiments, the carbohydrate is hyaluronic acid having a molecular weight of about 1.7 × 10⁶ Da and is present at a concentration of about 6.0 mg/mL. In some embodiments, the carbohydrate is hyaluronic acid having a molecular weight of about 2.6 × 10⁶ Da and is present at a concentration of about 6.0 mg/mL.

In some embodiments, the molecular weight of the carbohydrate is stable for at least 3 months. In some embodiments, the molecular weight of the carbohydrate is stable for at least 6 months. In some embodiments, the molecular weight of the carbohydrate is stable for at least 1 year. In some embodiments, the molecular weight of the carbohydrate is stable for at least 2 years. In some embodiments, the molecular weight of the carbohydrate is stable for at least 3 years. In some embodiments, the molecular weight of the carbohydrate is stable for at least 4 years. In some embodiments, the molecular weight of the carbohydrate is stable for at least 5 years.

In some embodiments, the ratio of solid component to liquid component is about 3 to about 1 by mass. In some embodiments, the ratio of solid component to liquid component is about 2 to about 1 by mass. In some embodiments, the ratio of solid component to liquid component is about 1.5 to about 1 by mass. In some embodiments, the ratio of solid component to liquid component is about 1 to about 1 by mass.

In some embodiments, the injectable biomaterial is injectable through a needle or cannula prior to initially setting. In some embodiments, the needle or cannula has a size of at least 21 gauge. In some embodiments, the needle or cannula has a size of at least 20 gauge. In some embodiments, the needle or cannula has a size of at least 18 gauge. In some embodiments, the needle or cannula has a size of at least 16 gauge. In some embodiments, the needle or cannula has a size of at least 15 gauge. In some embodiments, the needle or cannula has a size of at least 14 gauge. In some embodiments, the needle or cannula has a size of at least 12 gauge. In some embodiments, the needle or cannula has a size of at least 10 gauge.

In some embodiments, the injectable biomaterial does not dewater when being dispensed through a needle or cannula. In some embodiments, the injectable biomaterial does not seize when being dispensed through a needle or cannula.

In some embodiments, the injectable biomaterial is cohesive. In some embodiments, the injectable biomaterial remains cohesive during its initial setting time.

In some embodiments, the injectable biomaterial adheres to bone. In some embodiments, the injectable biomaterial remains adhesive to the bone during its initial setting time.

In some embodiments, the injectable biomaterial is workable for less than about 60 minutes after the mixing of the solid component and the liquid component. In some embodiments, the injectable biomaterial is workable for less than about 50 minutes after the mixing of the solid component and the liquid component. In some embodiments, the injectable biomaterial is workable for less than about 40 minutes after the mixing of the solid component and the liquid component. In some embodiments, the injectable biomaterial is workable for less than about 30 minutes after the mixing of the solid component and the liquid component. In some embodiments, the injectable biomaterial is workable for less than about 20 minutes after the mixing of the solid component and the liquid component. In some embodiments, the injectable biomaterial is workable for less than about 10 minutes after the mixing of the solid component and the liquid component. In some embodiments, the injectable biomaterial is workable for less than about 5 minutes after the mixing of the solid component and the liquid component. In some embodiments, the injectable biomaterial is workable for less than about 4 minutes after the mixing of the solid component and the liquid component. In some embodiments, the injectable biomaterial is workable for less than about 3 minutes after the mixing of the solid component and the liquid component. In some embodiments, the injectable biomaterial is workable for less than about 2 minutes after the mixing of the solid component and the liquid component. In some embodiments, the injectable biomaterial is workable for less than about 1 minute after the mixing of the solid component and the liquid component.

In some embodiments, the injectable biomaterial initially sets in less than about 60 minutes after mixing the solid component and the liquid component. In some embodiments, the injectable biomaterial initially sets in less than in less than about 50 minutes after mixing the solid component and the liquid component. In some embodiments, the injectable biomaterial initially sets in less than in less than about 40 minutes after mixing the solid component and the liquid component. In some embodiments, the injectable biomaterial initially sets in less than in less than about 30 minutes after mixing the solid component and the liquid component. In some embodiments, the injectable biomaterial initially sets in less than in less than about 20 minutes after mixing the solid component and the liquid component. In some embodiments, the injectable biomaterial initially sets in less than in less than about 10 minutes after mixing the solid component and the liquid component. In some embodiments, the injectable biomaterial initially sets in less than in less than about 5 minutes after mixing the solid component and the liquid component. In some embodiments, the injectable biomaterial initially sets in less than in less than about 4 minutes after mixing the solid component and the liquid component. In some embodiments, the injectable biomaterial initially sets in less than in less than about 3 minutes after mixing the solid component and the liquid component. In some embodiments, the injectable biomaterial initially sets in less than in less than about 2 minutes after mixing the solid component and the liquid component. In some embodiments, the injectable biomaterial initially sets in less than in less than about 1 minute after mixing the solid component and the liquid component.

In some embodiments, the injectable biomaterial cures completely in less than about 96 hours after the mixing of the solid component and the liquid component. In some embodiments, the injectable biomaterial cures completely in less than about 72 hours after the mixing of the solid component and the liquid component. In some embodiments, the injectable biomaterial cures completely in less than about 48 hours after the mixing of the solid component and the liquid component. In some embodiments, the injectable biomaterial cures completely in less than about 24 hours after the mixing of the solid component and the liquid component. In some embodiments, the injectable biomaterial cures completely in less than about 12 hours after the mixing of the solid component and the liquid component. In some embodiments, the injectable biomaterial cures completely in less than about 6 hours after the mixing of the solid component and the liquid component. In some embodiments, the injectable biomaterial cures completely in less than about 5 hours after the mixing of the solid component and the liquid component. In some embodiments, the injectable biomaterial cures completely in less than about 4 hours after the mixing of the solid component and the liquid component. In some embodiments, the injectable biomaterial cures completely in less than about 3 hours after the mixing of the solid component and the liquid component. In some embodiments, the injectable biomaterial cures completely in less than about 2 hours after the mixing of the solid component and the liquid component. In some embodiments, the injectable biomaterial cures completely in less than about 1 hour after the mixing of the solid component and the liquid component.

In some embodiments, the initial setting and curing of the injectable biomaterial does not result in a gaseous release.

In some embodiments, the injectable biomaterial does not significantly alter the pH of the adjacent fluids when disposed in a patient.

In some embodiments, the initial setting curing of the injectable biomaterial does not significantly alter the temperature of the adjacent fluids when disposed in a patient.

In some embodiments, the curing of the injectable biomaterial yields an apatitic crystal structure substantially consistent with that of hydroxyapatite. In some embodiments, the curing of the injectable biomaterial yields an apatitic crystal structure that is at least about 90% hydroxyapatite. In some embodiments, the curing of the injectable biomaterial yields an apatitic crystal structure that is at least about 95% hydroxyapatite. In some embodiments, the curing of the injectable biomaterial yields an apatitic crystal structure that is at least about 96% hydroxyapatite. In some embodiments, the curing of the injectable biomaterial yields an apatitic crystal structure that is at least about 97% hydroxyapatite. In some embodiments, the injectable biomaterial yields an apatitic crystal structure that is at least about 98% hydroxyapatite. In some embodiments, the curing of the injectable biomaterial yields an apatitic crystal structure that is at least about 99% hydroxyapatite. In some embodiments, the curing of the injectable biomaterial yields an apatitic crystal structure that is greater than about 99% hydroxyapatite.

In some embodiments, the fully set and cured injectable biomaterial has a molar Ca/P ratio of about 1 to about 2. In some embodiments, the fully set and cured injectable biomaterial has a molar Ca/P ratio of about 1.3 to about 1.8. In some embodiments, the fully set and cured injectable biomaterial has a molar Ca/P ratio of about 1.4 to about 1.7. In some embodiments, the fully set and cured injectable biomaterial has a molar Ca/P ratio of about 1.5 to about 1.7. In some embodiments, the fully set and cured injectable biomaterial has a molar Ca/P ratio of about 1.5 to about 1.667.

In some embodiments, the fully set and cured injectable biomaterial has a compressive strength of less about 20 MPa. In some embodiments, the fully set and cured injectable biomaterial has a compressive strength of less about 15 MPa. In some embodiments, the fully set and cured injectable biomaterial has a compressive strength of less about 10 MPa. In some embodiments, the fully set and cured injectable biomaterial has a compressive strength of less about 9 MPa. In some embodiments, the fully set and cured injectable biomaterial has a compressive strength of less about 8 MPa. In some embodiments, the fully set and cured injectable biomaterial has a compressive strength of less about 7 MPa. In some embodiments, the fully set and cured injectable biomaterial has a compressive strength of less about 6 MPa. In some embodiments, the fully set and cured injectable biomaterial has a compressive strength of less about 5 MPa. In some embodiments, the fully set and cured injectable biomaterial has a compressive strength of less about 4 MPa. In some embodiments, the fully set and cured injectable biomaterial has a compressive strength of less about 3 MPa. In some embodiments, the fully set and cured injectable biomaterial has a compressive strength of less about 2 MPa. In some embodiments, the fully set and cured injectable biomaterial has a compressive strength of less about 1 MPa.

In some embodiments, the fully set and cured injectable biomaterial has an elastic modulus of less than about 5 GPa. In some embodiments, the fully set and cured injectable biomaterial has an elastic modulus of less than about 4 GPa. In some embodiments, the fully set and cured injectable biomaterial has an elastic modulus of less than about 3 GPa. In some embodiments, the fully set and cured injectable biomaterial has an elastic modulus of less than about 2 GPa. In some embodiments, the fully set and cured injectable biomaterial has an elastic modulus of less than about 1 GPa. In some embodiments, the fully set and cured injectable biomaterial has an elastic modulus of less than about 0.5 GPa. In some embodiments, the fully set and cured injectable biomaterial has an elastic modulus of less than about 0.25 GPa.

In some embodiments, the injectable biomaterial has a viscosity of about 5 Pa·s and about 30 Pa s immediately after mixing the solid component and the liquid component, when measured at room temperature. In some embodiments, the injectable biomaterial has a viscosity of about 5 Pa s and about 20 Pa s immediately after mixing the solid component and the liquid component, when measured at room temperature. In some embodiments, the injectable biomaterial has a viscosity of about 5 Pa s and about 18 Pa s immediately after mixing the solid component and the liquid component, when measured at room temperature. In some embodiments, the injectable biomaterial does not biomechanically stabilize bone.

In some embodiments, the fully set and cured injectable biomaterial has a true density of about 1 g/cm³ to about 4 g/cm³. In some embodiments, the fully set and cured injectable biomaterial has a true density of about 1.5 g/cm³ to about 3.5 g/cm³. In some embodiments, the fully set and cured injectable biomaterial has a true density of about 1.83 g/cm³ to about 3.14 g/cm³. In some embodiments, the fully set and cured injectable biomaterial has a true density of about 2 g/cm³ to about 3 g/cm³.

In some embodiments, the fully set and cured injectable biomaterial comprises a median pore diameter of less than about 1 µm. In some embodiments, the fully set and cured injectable biomaterial comprises a median pore diameter of less than about 0.8 µm. In some embodiments, the fully set and cured injectable biomaterial comprises a median pore diameter of less than about 0.6 µm. In some embodiments, the fully set and cured injectable biomaterial comprises a median pore diameter of less than about 0.5 µm. In some embodiments, the fully set and cured injectable biomaterial comprises a median pore diameter of less than about 0.4 µm. In some embodiments, the fully set and cured injectable biomaterial comprises a median pore diameter of less than about 0.2 µm. In some embodiments, the fully set and cured injectable biomaterial comprises a median pore diameter of less than about 0.15 µm.

In some embodiments, the fully set and cured injectable biomaterial comprises a total porous area of less than about 4 m²/g. In some embodiments, the fully set and cured injectable biomaterial comprises a total porous area of less than about 3 m²/g. In some embodiments, the fully set and cured injectable biomaterial comprises a total porous area of less than about 2 m²/g.

In some embodiments, the fully set and cured injectable biomaterial comprises a porosity sufficient to prevent diffusional passage of at least one of inflammatory mediators and non-inflammatory mediators.

In some embodiments, the fully set and cured injectable biomaterial is osteoinductive.

In some embodiments, the fully set and cured injectable biomaterial is osteoconductive.

In some embodiments, the fully set and cured injectable biomaterial is resorbable.

In some embodiments, the curing of the injectable biomaterial yields less than about 5% calcium oxide. In some embodiments, the curing of the injectable biomaterial yields less than about 4% calcium oxide. In some embodiments, the curing of the injectable biomaterial yields less than about 3% calcium oxide. In some embodiments, the curing of the injectable biomaterial yields less than about 2% calcium oxide. In some embodiments, the curing of the injectable biomaterial yields less than about 1% calcium oxide.

In some embodiments, the liquid component is sterile. In some embodiments, the solid component is sterile.

In some embodiments, the injectable biomaterial is intermixable.

In some embodiments, the pH adjusting agent is selected from an organic acid and an inorganic acid. In some embodiments, the pH adjusting agent is selected from the group consisting of citric acid, formic acid, acetic acid, and mixtures thereof. In some embodiments, the pH adjusting agent s selected from the group consisting of hydrochloric acid, phosphoric acid, nitric acid, and mixtures thereof.

In some embodiments, providing the solid component further comprises drying the solid component. In some embodiments, the drying comprises exposing the solid component to heat over a period of time. In some embodiments, the heat comprises at least about 165 °C. In some embodiments, the period of time comprises at least about 12 hours.

In another aspect, disclosed herein is a method of treating an affected area of a bone in a patient in need thereof, the method comprising identifying the affected area in the bone of the patient; creating in the bone an incision through a cortical wall of the bone to provide access to a degenerate cancellous space in the affected area of the bone; administering a volume of an injectable biomaterial of any preceding claim through the incision through the cortical wall of the bone and into the degenerate cancellous space.

In some embodiments, the affected area of bone is adjacent to a joint of the patient in which the patient is experiencing a joint pathology. In some embodiments, the joint pathology is a pathology of the knee, shoulder, wrist, hand, spine, ankle, elbow, or hip. In some embodiments, the joint pathology is selected from the group consisting of pain, osteoarthritis, rheumatoid arthritis, avascular necrosis, and combinations thereof. In some embodiments, the method is for the treatment of osteoarthritis in a joint of the patient. In some embodiments, the osteoarthritis has a Kellgren Lawrence (KL) grade of 1-3. In some embodiments, the joint pathology is not related to joint instability.

In some embodiments, the affected area exhibits at least one of inflammatory or degradative changes as a result of at least one of inflammatory mediators and non-inflammatory mediators.

In some embodiments, the inflammatory or degradative changes are identified by MRI. In some embodiments, the MRI is a T2 MRI.

In some embodiments, the inflammatory or degradative changes are disposed in cancellous bone.

In some embodiments, the affected area is disposed between about 0 inches and about 5 inches from the joint of the patient. In some embodiments, the affected area is disposed between about 0 inches and about 4 inches from the joint of the patient. In some embodiments, the affected area is disposed between about 0 inches and about 3 inches from the joint of the patient. In some embodiments, the affected area is disposed between about 0 inches and about 2 inches from the joint of the pat In some embodiments, the affected area is disposed between about 0 inches and about 1 inch from the joint of the patient. In some embodiments, the affected area is disposed between about 0 inches and about 20 mm from the joint of the patient. In some embodiments, the affected area is disposed between about 0 mm and about 10 mm from the joint of the patient. In some embodiments, the affected area is disposed between about 0 mm and about 5 mm from the joint of the patient. In some embodiments, the affected area is disposed between about 0 mm and about 1 mm from the joint of the patient.

In some embodiments, the incision is percutaneous.

In some embodiments, providing the access to the cancellous space comprises creating a channel in the bone of the patient to couple the incision in the cortical wall of the bone to the cancellous space comprising the affected area. In some embodiments, the channel is perpendicular to the long axis of the bone. In some embodiments, the channel is not perpendicular to the long axis of the bone. In some embodiments, the channel is within about 5 inches from the proximal subchondral plate. In some embodiments, the channel is within about 4 inches from the proximal subchondral plate. In some embodiments, the channel is within about 3 inches from the proximal subchondral plate. In some embodiments, the channel is within about 2 inches from the proximal subchondral plate. In some embodiments, the channel is within about 1 inches from the proximal subchondral plate. In some embodiments, the channel is within about 20 mm of the proximal subchondral plate. In some embodiments, the channel is within about 10 mm of the proximal subchondral plate. In some embodiments, the channel is within about 5 mm of the proximal subchondral plate. In some embodiments, the channel is within about 1 mm of the proximal subchondral plate. In some embodiments, the channel is accessed by a cannula that is positioned and inserted without the need for additional targeting instrumentation.

In some embodiments, the method further comprises decompressing and aspirating the contents of the affected area prior to administration of the injectable biomaterial to the affected area. In some embodiments, the decompression and aspiration reduces localized inflammation in the affected area. In some embodiments, the decompression and aspiration reduces intraosseous pressure in the affected area. In some embodiments, the contents comprise a fluid. In some embodiments, the fluid comprises at least one of inflammatory mediators and non-inflammatory mediators.

In some embodiments, the at least one inflammatory mediator comprises at least one of bradykinin, histamine, prostaglandins, lactic acid, substance P, vasoactive intestinal peptide, calcitonin gene related peptide (CGRP), and mixtures thereof. In some embodiments, the at least one inflammatory mediator comprises an inflammatory cytokine. In some embodiments, the inflammatory cytokine is selected from the group consisting of AIMP1 (SCYE1), BMP2, CD40LG (TNFSF5), CSF1 (MCSF), CSF2 (GM-CSF), CSF3 (GCSF), FASLG (TNFSF6), GM-CSF, IFNA2, IFNG, IL-1, IL-6, IL-8, IL-15, IL-16, IL-17, IL-18, IFN-γ, LTA (TNFB), LTB, MIF, NAMPT, OSM, SPP1, TGF-β, TNF, TNF-α, TNFSF10 (TRAIL), TNFSF11 (RANKL), TNFSF13, TNFSF13B, TNFSF4 (OX40L), VEGFA, and mixtures thereof. In some embodiments, the at least one non-inflammatory mediator comprises a proteolytic enzyme. In some embodiments, the proteolytic enzyme is selected from the group consisting of matrix metalloproteinases (MMPs), tissue inhibitors of metalloproteinases (TIMPs), a disintegrin and metalloproteinase with thrombospondin motifs (ADAM-TS), and mixtures thereof. In some embodiments, the inflammatory mediator comprises an inflammatory chemokine. In some embodiments, the inflammatory chemokine is selected from the group consisting of C5, CCL1 (I-309), CCL11 (eotaxin), CCL13 (MCP-4), CCL15 (MIP-1d), CCL16 (HCC-4), CCL17 (TARC), CCL2 (MCP-1), CCL20 (MIP-3a), CCL22 (MDC), CCL23 (MPIF-1), CCL24 (MPIF-2, Eotaxin-2, MPIF-2, Eotaxin-2), CCL26 (eotaxin-3), CCL3 (MIP-1A), CCL4 (MIP-1B), CCL5 (RANTES), CCL7 (MCP-3), CCL8 (MCP-2), CX3CL1, CXCL1 (GRO1, GRO-alpha, SCYB1), CXCL10 (INP10), CXCL11 (I-TAC, IP-9), CXCL12 (SDF1), CXCL13, CXCL2 (GRO2, GRO-beta, SCYB2), CXCL3, CXCL5 (ENA-78, LIX), CXCL6 (GCP-2), CXCL9 (MIG), and mixtures thereof. In some embodiments, the inflammatory mediator comprises an interleukin. In some embodiments, the interleukin is selected from the group consisting of IL13, IL15, IL16, IL17A, IL17C, IL17F, IL1A, IL1B, IL1RN, IL21, IL27, IL3, IL33, IL5, IL7, CXCL8, IL9, and mixtures thereof. In some embodiments, the inflammatory mediator comprises an inflammatory mediator selected from the group consisting of bradykinin, calcitonin gene related peptide (CGRP), histamine, lactic acid, nerve growth factor (NGF), prostaglandins, substance P, vasoactive intestinal peptide, and mixtures thereof.

In some embodiments, the injectable biomaterial is administered through a cannula or needle. In some embodiments, the needle or cannula has a size of at least 21 gauge. In some embodiments, the needle or cannula has a size of at least 20 gauge. In some embodiments, the needle or cannula has a size of at least 18 gauge. In some embodiments, the needle or cannula has a size of at least 16 gauge. In some embodiments, the needle or cannula has a size of at least 15 gauge. In some embodiments, the needle or cannula has a size of at least 14 gauge. In some embodiments, the needle or cannula has a size of at least 12 gauge. In some embodiments, the needle or cannula has a size of at least 10 gauge.

In some embodiments, the injectable biomaterial does not dewater when being dispensed through the needle or cannula.

In some embodiments, the injectable biomaterial does not seize when being dispensed through the needle or cannula.

In some embodiments, the injectable biomaterial is administered through a steerable cannula to minimize surgical damage.

In some embodiments, the injectable biomaterial is injected into the affected area while minimally disrupting the subchondral plate.

In some embodiments, the injectable biomaterial is injected into a layer between about 0 mm and about 20 mm above or below the affected area while minimally disrupting the subchondral plate. In some embodiments, the injectable biomaterial is injected into a layer between about 0 mm and about 10 mm above or below the affected area while minimally disrupting the subchondral plate. In some embodiments, the injectable biomaterial is injected into a layer between about 0 mm and about 5 mm above or below the affected area while minimally disrupting the subchondral plate. In some embodiments, the injectable biomaterial is injected into a layer between about 0 mm and about 1 mm above or below the affected area while minimally disrupting the subchondral plate.

In some embodiments, the injectable biomaterial is administered to an area that is not intrinsic to the structural stability of the bone.

In some embodiments, the method further comprises arthroscopically examining the joint space post-injection to ensure an absence of the injectable biomaterial in the joint.

In some embodiments, the injectable biomaterial flows into the porosity of cancellous bone during administration into the affected area.

In some embodiments, the injectable biomaterial remains cohesive and substantially fills bone voids during administration into the affected area.

In some embodiments, the injectable biomaterial at least partially coats the interface between the cancellous space and an adjacent joint to provide a protective layer upon setting.

In some embodiments, the injectable biomaterial prevents diffusional passage of at least one of inflammatory mediators and non-inflammatory mediators from the adjacent joint space into the affected area.

In some embodiments, the protective layer provides a sacrificial layer for osteoclasts to consume during bone remodeling.

In some embodiments, the administration of the injectable biomaterial does not cause stress shielding resulting in the weakening of the unloaded bone.

In some embodiments, the method does not cause substantial post-operative pain.

In some embodiments, the method decreases pain in the joint.

In some embodiments, the method slows the progression of osteoarthritis in the joint.

In some embodiments, the method is for the treatment of rheumatoid arthritis in a joint of the patient. In some embodiments, the method slows the progression of rheumatoid arthritis in the joint.

In some embodiments, the method slows the progression of avascular necrosis in the joint.

In another aspect, disclosed herein is a kit comprising a solid component and a liquid component for preparing an injectable biomaterial as disclosed herein and instructions for use of the same.

In some embodiments, the instructions are for a method of treating an affected area of a bone in a patient in need thereof.

In some embodiments, the treatment is for pain, osteoarthritis, rheumatoid arthritis, avascular necrosis, or combinations thereof.

In some embodiments, the solid component and the liquid component are disposed in separate sterile containers.

In some embodiments, the packaging configuration allows the solid component and the liquid component to remain stable at 2 °C - 25 °C for at least about three months. In some embodiments, the packaging configuration allows the solid component and the liquid component to remain stable at 2 °C - 25 °C for at least about six months. In some embodiments, the packaging configuration allows the solid component and the liquid component to remain stable at 2 °C - 25 °C for at least about one year. In some embodiments, the packaging configuration allows the solid component and the liquid component to remain stable at 2 °C - 25 °C for at least about two years. In some embodiments, the packaging configuration allows the solid component and the liquid component to remain stable at 2 °C - 25 °C for at least about three years. In some embodiments, the packaging configuration allows the solid component and the liquid component to remain stable at 2 °C - 25 °C for at least about four years. In some embodiments, the packaging configuration allows the solid component and the liquid component to remain stable at 2 °C - 25 °C for at least about five years.

In some embodiments, the liquid component is disposed in a sterile syringe.

In some embodiments, the solid component is disposed in a syringe possessing an integrated mixing device for *in situ* mixing of premeasured portions of the solid component and the liquid component to form the injectable biomaterial. In some embodiments, the syringe is sterile.

In some embodiments, the kit further comprises a Luer-Lock.

In some embodiments, the kit further comprises an end cap.

### BRIEF DESCRIPTION OF THE DRAWINGS

FIG. 1 shows a schematic of a human knee comprising an area of degenerate bone.
FIGs. 2A-E show a schematic callout of an area comprising a portion of the degenerate bone of FIG. 1
FIGs. 3A-B show an injectable biomaterial according to the present disclosure as compared to an injectable biomaterial lacking a carbohydrate, each in phosphate buffered saline.
FIGs. 4A-D show injectable biomaterials according to the present disclosure as compared to an injectable biomaterial lacking a carbohydrate, each in phosphate buffered saline.
FIGs. 5A-D show an injectable biomaterial according to the present disclosure as compared to an injectable biomaterial lacking a carbohydrate, after removal of excess phosphate buffered saline.
FIGs. 6A-D show cross-sections of sawbone injected with injectable biomaterials according to the present disclosure as contrasted with sawbone injected with an injectable biomaterial lacking a carbohydrate.
FIGs. 7A-B show cross-sections of sawbone injected with injectable biomaterials according to the present disclosure as contrasted with sawbone injected with an injectable biomaterial lacking a carbohydrate.
FIGs. 8A-B show the results of a diffusion barrier experiment comparing control with an injectable biomaterial lacking a carbohydrate and an injectable biomaterial according to the present disclosure.
FIG. 9 shows an x-ray powder diffractogram of an injectable biomaterial, post setting and curing, according to the present disclosure.
FIG. 10 shows a Fourier Transform Infrared ("FT-IR") spectrograph of an injectable biomaterial according to the present disclosure.
FIGs. 11A-C show scanning electron microscopy ("SEM") images of an injectable biomaterial according to the present disclosure after setting and curing.
FIGs. 12A-B show micro-computed tomography ("micro CT") images from different planes taken 6 weeks after administration of an injectable biomaterial according to the present disclosure into degenerate bone generated in skeletally mature New Zealand White rabbits.
FIG. 13 shows an image of an injectable biomaterial according to the present disclosure injected into canine femoral condyle.
FIG. 14 shows and image of an injectable biomaterial according to the present disclosure injected into human cadaver bone.

### DETAILED DESCRIPTION OF THE INVENTION

The present disclosure relates to methods and compositions for the treatment of degenerate bone in a patient. In some embodiments, the methods and compositions disclosed herein are useful in the treatment, prevention, or in delaying the progression of a bone disease linked to bone degeneration, such as osteoarthritis ("OA"), rheumatoid arthritis, and avascular necrosis.

The inventors have surprisingly discovered that the addition of a carbohydrate to an injectable biomaterial provides for an injectable, intermixable, flowable, settable, curable, cohesive composition that adheres to bone. Further, the injectable biomaterials disclosed herein possess a low porosity and high dimensional stability that is desirable for use in the minimally invasive treatment of various bone diseases, a property absent in the biomaterials of the prior art.

Without wishing to be bound by theory, the inventors posit that a low porosity and high dimensional stability injectable biomaterial serves to arrest biochemical communication involving the joint space and the adjacent bone. The inventors posit that this biochemical communication is responsible for the onset of a number of symptoms of bone degeneration, including pain and fluid accumulation, and that continued bone degeneration can facilitate the progression of bone diseases linked to bone degeneration, such as OA, rheumatoid arthritis, and avascular necrosis. By way of example, as the cartilage and synovium become injured, whether from trauma or overuse, a milieu of inflammatory and/or non-inflammatory mediators is released from both tissues. These mediators enter the bone through channels in the cortical bone plane, stimulating pain through nociceptor activation, fluid accumulation, and degenerative changes in the affected area of bone (such as by the formation of a bone marrow lesion). The inventors hypothesize that, as a result of this biochemical communication between the joint space and the adjacent bone, the homeostasis of the affected area of bone is disturbed, resulting in dysregulation in the synthesis and degradation of bone proteins, and a degenerative positive feedback loop responsible for the progression of pathologies such as OA is formed. Blocking the effect of the inflammatory and/or non-inflammatory mediators causing bone degeneration is therefore essential to treating, preventing, and/or delaying the progression of bone disease.

Accordingly, the inventors set out to produce an injectable biomaterial with low porosity and high dimensional stability, but which were able to be prepared using lower solid-to-liquid ratios, allowing for their use in the minimally invasive treatment of bone disease linked to bone degeneration. During their research, the inventors found that existing techniques used to produce such injectable materials generally resulted in materials demonstrating poor cohesion, adhesion, setting and curing properties, rendering such materials unsuitable for use in the minimally invasive treatment of bone disease. Similarly, those prior techniques generally resulted in materials with high porosity that would be poorly suited to arresting biochemical communication between the affected area of bone and the adjacent joint space. *See, e.g.,* Eliaz, N.; Metoki, N. Materials 2017, 10, 334, at 13, the contents of which are incorporated by reference herein in their entirety. The inventors then surprisingly discovered that the addition of a carbohydrate to an injectable biomaterial allowed for a material that could be prepared with lower solid-to-liquid ratios but was nonetheless able to set, cure, maintain cohesiveness and adherency to bone, and that possessed low porosity and high dimensional stability as compared to injectable biomaterials prepared without an added carbohydrate. In other words, the inventors discovered that the addition of a carbohydrate provided for an injectable biomaterial having the critical combination of injectability, intermixability, flowability, settability and curability, while maintaining the desired properties of cohesivity, adhesivity, low porosity, and high dimensional stability. Accordingly, in one embodiment, the present disclosure provides injectable biomaterials that can be prepared using a lower solid-to-liquid ratio than previously possible while maintaining the cohesiveness, adherency to bone, low porosity, and high dimensional stability of materials traditionally made with a higher solid-to-liquid ratio. Accordingly, the disclosed injectable biomaterials are able to stem the flow and prevent ingress of inflammatory and non-inflammatory mediators into the affected area of bone from an adjacent joint space while not sacrificing the desirable ability to intermix, be injected, flow into, remain and set and cure in the area of degenerate bone to be treated without being cleared by the function of normal body fluid exchange. These materials provide this surprising and unique combination of properties and, contrary to the common knowledge in the art, which focused on the provision of high compressive strength and elastic modulus materials, unexpectedly allow for superior treatment of bone disease linked to bone degeneration as compared to prior compositions.

Without wishing to be bound by theory, the inventors posit that the injectable biomaterials disclosed herein serve to treat the underlying causes of bone disease linked to bone degeneration by eliminating and preventing recurrence of biochemical communication between the affected area of bone and the adjacent joint space. The injectable biomaterials disclosed herein provide a barrier between the affected area of bone and the adjacent joint space that prevents the ingress of inflammatory and/or non-inflammatory mediators from the joint space, arresting degradation mediated by, *e.g*., proteolytic enzymes and inflammatory cytokines, and allowing the bone to recover via normal dynamics (bone resorption). In contrast, prior injectable biomaterials addressed the symptoms, but not the underlying causes of bone disease linked to bone degeneration.

Furthermore, the disclosed injectable biomaterials possess lower compressive strength than the materials typically used in the prior art. Without wishing to be bound by theory, the inventors posit that, counter to the prevailing knowledge in the art, provision of an injectable biomaterial that does not biomechanically stabilize an affected area of bone provides for superior outcomes in the treatment, prevention, and slowing the progression of bone disease linked to bone degeneration. Conventional wisdom in the art indicated that injectable biomaterials (such as CPCs) with high compressive strength were required to properly treat joint pathologies by providing biomechanical stabilization. Contrary to this conventional wisdom, the inventors have surprisingly discovered that by providing a weaker injectable biomaterial that does not provide biomechanical support to the affected area, the disclosed methods and compositions do not artificially alter the biomechanical forces to which the joint is exposed, providing a superior methodology to address such pathologies. Without wishing to be bound by theory, the inventors posit that the provision of biomechanical support to an area of degenerate bone can be detrimental by shifting stress and strain to otherwise healthy tissue, potentially resulting in the spread of bone disease and/or its symptoms in accordance with Wolff's Law. The inventors posit that this is due the impossibility of exact recreation of healthy biomechanics, which necessarily relates on undue strain on other locations. For example, areas of high stress will become thicker and stiffer whereas areas of low stress will resorb according to Wolff's Law, which states that bone in a healthy person or animal will adapt to the loads under which it is placed. Based on Wolff s Law, if loading on a particular bone increases, the bone will remodel itself over time to become stronger to resist that sort of loading. However, these normal biological responses are not possible in bone disease because the degenerative biochemical feedback loop present in those conditions adversely affect, and can completely cease, normal bone remodeling processes. Accordingly, by not providing biomechanical support, the disclosed methods and compositions allow for physiological conditions that permit the affected area of bone to recover and rebuild through natural bone remodeling, thus slowing and/or reversing the progression of bone disease, and preventing the onset and progression of bone diseases such as symptomatic OA, rheumatoid arthritis, and avascular necrosis, as well as preventing spread of the disease or symptoms to adjacent healthy tissues.

In some embodiments, the strength of the fully set and cured injectable biomaterial is less than that provided by the injectable biomaterials of the prior art. For example, certain prior art injectable biomaterials possess a compressive strength of approximately 50 MPa, which is 4-10 times greater than the average 5-15 MPa compressive strength of healthy cancellous bone. *See, e.g.,* Norian® SRS® Tiabia plateau fractures, Synthes®, available at http://www.rch.org.au/uploadedFiles/Main/Content/ortho/Norian_SRS_Tibia_plateau_fractur es.pdf (last visited April 24, 2017), the contents of which are incorporated herein by reference in their entirety. In some embodiments, the strength of the fully set and cured injectable biomaterial is characterized by one or more of compressive strength and elastic modulus. In some embodiments, the fully set and cured injectable biomaterial has a compressive strength of less about 20 MPa. In some embodiments, the fully set and cured injectable biomaterial has a compressive strength of less about 15 MPa. In some embodiments, the fully set and cured injectable biomaterial has a compressive strength of less about 10 MPa. In some embodiments, the fully set and cured injectable biomaterial has a compressive strength of less about 9 MPa. In some embodiments, the fully set and cured injectable biomaterial has a compressive strength of less about 8 MPa. In some embodiments, the fully set and cured injectable biomaterial has a compressive strength of less about 7 MPa. In some embodiments, the fully set and cured injectable biomaterial has a compressive strength of less about 6 MPa. In some embodiments, the fully set and cured injectable biomaterial has a compressive strength of less about 5 MPa. In some embodiments, the fully set and cured injectable biomaterial has a compressive strength of less about 4 MPa. In some embodiments, the fully set and cured injectable biomaterial has a compressive strength of less about 3 MPa. In some embodiments, the fully set and cured injectable biomaterial has a compressive strength of less about 2 MPa. In some embodiments, the fully set and cured injectable biomaterial has a compressive strength of less about 1 MPa.

In further embodiments, the elastic modulus of the fully set and cured injectable biomaterial is less than that provided by the injectable biomaterials of the prior art. In further embodiments, the elastic modulus of the fully set and cured injectable biomaterial is close to that of healthy subchondral bone. Without wishing to be bound by theory, the inventors posit that provision of an injectable biomaterial having an elastic modulus similar to that of healthy subchondral bone reduces the risk of altering natural biomechanics and resulting in further bone degradation in accordance with Wolff's Law (*i.e*., stress shielding). *See, e.g.,* Eliaz, N.; Metoki, N. Materials 2017, 10, 334, at 3. For example, the elastic modulus of samples of human subchondral bone has been reported to be about 1.15 GPa. *See, e.g.,* Choi, K. et al. J. Biomech. 1990, 23(11), 1103-13; *see also* Brown, T. D.; Vrahas, M. S. J. Orthoped. Res. 1984, 2(1), 32-38 (reporting an apparent elastic modulus of 1.372 GPa for machined caps of subchondral bone); Mente, P. L.; Lewis, J. L. J. Orthoped. Res. 1994, 12(5), 637-47 (reporting an elastic modulus calculated from "pure" bovine subchondral bone beams of 2.3 ± 1.5 GPa), the contents of all of the foregoing of which are incorporated herein by reference in their entireties. In some embodiments, the fully set and cured injectable biomaterial has an elastic modulus of less than about 5 GPa. In some embodiments, the fully set and cured injectable biomaterial has an elastic modulus of less than about 4 GPa. In some embodiments, the fully set and cured injectable biomaterial has an elastic modulus of less than about 3 GPa. In some embodiments, the fully set and cured injectable biomaterial has an elastic modulus of less than about 2 GPa. In some embodiments, the fully set and cured injectable biomaterial has an elastic modulus of less than about 1 GPa. In some embodiments, the fully set and cured injectable biomaterial has an elastic modulus of less than about 0.5 GPa. In some embodiments, the fully set and cured injectable biomaterial has an elastic modulus of less than about 0.25 GPa.

Moreover, the inventors surprisingly discovered that the disclosed injectable biomaterials set and cure without significant gaseous emission. Without wishing to be bound by theory, the inventors posit that the absence of gaseous release during setting and curing post-administration results in an injectable biomaterial does not expand during setting and curing, thus reducing or eliminating post-operative pain as compared with prior art materials. Furthermore, the inventors posit that the absence of gaseous release during curing and setting post-administration facilitates the formation of a structure with the desired decreased porosity as compared with prior art materials. The inventors posit that the provision of an injectable biomaterial that does not comprise bicarbonate may serve to prevent gaseous release and thus results in a biomaterial with decreased porosity that does not cause post-operative pain.

### Definitions

The term "adherent to bone" as used herein with reference to an injectable biomaterial refers to the materials demonstration of sufficient affinity for bone such that it is not readily cleared away from the site of injection by bodily fluids.

The term "bone disease" as used herein refers to a disease, condition, or pathology in a patient that is linked to bone degeneration. For example, the bone disease can affect a joint adjacent to a degenerate bone.

The term "cohesive" as used herein with reference to an injectable biomaterial refers to the ability of a material to adhere to itself and be molded such that it non-transiently maintains its shape over a period of time and fills an affected area of degenerate bone after injection.

The term "cure" as used herein with reference to an injectable biomaterial refers to the process whereby the components of the injectable biomaterial chemically and physically react to form the final desired crystal structure. A material that is "cured" no longer undergoes appreciable changes in compressive strength or porosity. The terms "cure time" and "curing time" as used herein with reference to an injectable biomaterial refer to the time at which the injectable biomaterial is fully cured. In some embodiments, the injectable biomaterials disclosed herein cure to form an apatitic crystal structure.

The term "decompression" as used herein refers to a procedure to remove pressure on a structure.

The term "degenerate tissue" as used herein refers to tissue that has undergone a change to a lower or less functionally active form.

The term "degenerate bone" as used herein refers to an area of bone that has undergone a change to a lower or less functionally active form. In some embodiments, degenerate bone exhibits at least one change selected from (1) formation of higher volume fraction trabeculae relative to normal bone; (2) decreased mineral-to-matrix and carbonate-to-matrix values relative to normal bone; (3) increased intraosseous fluid accumulation relative to normal bone; and (4) increased infiltration into the marrow space of a fibrous collagen network relative to normal bone.

The term "dewater" as used herein with reference to an injectable biomaterial refers to separation of the solid component and the liquid component.

The term "flowable" as used herein with reference to an injectable biomaterial refers to any generally incompressible material which may be caused to flow under pressure or gravity.

The term "inflammatory mediator" as used herein refers to a biological component that induces an inflammatory response in an animal or a human. Inflammatory mediators include, but are not limited to, chemokines, such as C5, CCL1 (I-309), CCL11 (eotaxin), CCL13 (MCP-4), CCL15 (MIP-1d), CCL16 (HCC-4), CCL17 (TARC), CCL2 (MCP-1), CCL20 (MIP-3a), CCL22 (MDC), CCL23 (MPIF-1), CCL24 (MPIF-2, Eotaxin-2, MPIF-2, Eotaxin-2), CCL26 (eotaxin-3), CCL3 (MIP-1A), CCL4 (MIP-1B), CCL5 (RANTES), CCL7 (MCP-3), CCL8 (MCP-2), CX3CL1, CXCL1 (GRO1, GRO-alpha, SCYB1), CXCL 10 (INP10), CXCL11 (I-TAC, IP-9), CXCL 12 (SDF1), CXCL13, CXCL2 (GRO2, GRO-beta, SCYB2), CXCL3, CXCL5 (ENA-78, LIX), CXCL6 (GCP-2), CXCL9 (MIG); interleukins, such as IL13, IL15, IL16, IL17A, IL17C, IL17F, IL1A, IL1B, IL1RN, IL21, IL27, IL3, IL33, IL5, IL7, CXCL8, IL9; cytokines such as AIMP1 (SCYE1), BMP2, CD40LG (TNFSF5), CSF1 (MCSF), CSF2 (GM-CSF), CSF3 (GCSF), FASLG (TNFSF6), GM-CSF, IFNA2, IFNG, IL-1, IL-6, IL-8, IL-15, IL-16, IL-17, IL-18, IFN-γ, LTA (TNFB), LTB, MIF, NAMPT, OSM, SPP1, TGF-β, TNF, TNF-α, TNFSF10 (TRAIL), TNFSF11 (RANKL), TNFSF13, TNFSF13B, TNFSF4 (OX40L), VEGFA; and other inflammatory mediators, such as bradykinin, calcitonin gene related peptide (CGRP), histamine, lactic acid, nerve growth factor (NGF), prostaglandins, substance P, and vasoactive intestinal peptide; and mixtures thereof. Other inflammatory mediators are known to those skilled in the art.

The term "initial setting time" as used herein with reference to an injectable biomaterial refers to the shortest amount of time between (1) the mixing of the solid component and the liquid component and (2) the point where a 1 lb. (454 g) Gilmore Needle, having a tip diameter of 1/24 inch (1.06 mm) does not penetrate a sample of the injectable biomaterial of uniform thickness of 3/16 inch (5 mm), the length of the needle tip, in less than 1 minute. *See* ASTM C414-03 at 7.2, 8.2 (reapproved 2012), the contents of which are incorporated herein by reference in their entirety. The term "set" or "settable" as used herein with reference to an injectable biomaterial refers to the ability of the injectable material to transition from that state achieved at point (1) to point (2) described above.

The term "injectable" as used herein with reference to an injectable biomaterial refers to a material that is capable of being extruded from a syringe using no more than acceptable hand pressure applied to a plunger rod. Acceptable hand pressure is known to those of skill in the art. In some embodiments, the injectable biomaterial must be capable of being extruded from a syringe without the use of mechanical advantage, such as a screw-driven syringe. In some embodiments, the syringe is a 3 mL syringe. In some embodiments, the syringe is a 5 mL syringe. In some embodiments, the syringe is a 10 mL syringe. In some embodiments, the syringe is a 14 mL syringe. In some embodiments, an injectable biomaterial is able to be extruded from a syringe using no more than 15 lb. extrusion force at a rate of 6 mL/minute. In some embodiments, an injectable biomaterial is able to be extruded from a syringe using no more than 10 lb. extrusion force at a rate of 6 mL/minute. In some embodiments, an injectable biomaterial is able to be extruded from a syringe using no more than 7.5 lb. extrusion force at a rate of 6 mL/minute. In some embodiments, an injectable biomaterial is able to be extruded from a syringe using no more than 5 lb. extrusion force at a rate of 6 mL/minute. In some embodiments, the syringe is coupled to an 11 gauge cannula. In some embodiments, the syringe is coupled to an 14 gauge cannula. In some embodiments, the syringe is coupled to a 15 gauge cannula. In some embodiments, the syringe is coupled to an 18 gauge cannula. In some embodiments, the syringe is coupled to a 21 gauge cannula. In some embodiments, the syringe is coupled to a cannula and the injectable biomaterial must flow down the length of the cannula in its entirety without seizing or dewatering to be considered injectable. In some embodiments, the cannula is about 11 cm long. In some embodiments, the cannula is about 15 cm long.

The term "intermixable" as used herein with reference to an injectable biomaterial refers to the ability to achieve full mixing of the solid component and the liquid component when each is disposed in a 5 mL syringe, wherein the syringes are coupled and the contents extruded between the syringes for 1 minute using hand pressure with no visible seizing or dewatering.

The term "macroporous" as used herein with reference to an injectable biomaterial refers to a material that possess pores which are visible macroscopically.

The term "median pore diameter" as used herein refers to the pore diameter corresponding to 50% total intrusion volume from a cumulative intrusion volume vs. diameter plot. *See* Webb, P. An introduction to the physical characterization of materials by mercury intrusion porosimetry with emphasis on reduction and presentation of experimental data, Micromeritics Instrument Corp. (2001), the contents of which are incorporated herein by reference in their entirety.

The term "osteoconductive" as used herein with reference to an injectable biomaterial refers to the ability of an osteogenic material to serve as a substrate, scaffold or framework supporting new bone growth that is perpetuated by the native bone.

The term "osteogenic" as used herein with reference to an injectable biomaterial refers to the ability of a material to promote the growth of new bone tissue. Exemplary osteogenic materials include, but are not limited to, bone marrow aspirate, bone marrow aspirate concentrate, platelet-rich plasma, platelet-poor plasma, somatic cell autografts, stem cell autografts, stem cell allografts, and mixtures thereof. Other exemplary osteogenic materials are known to those skilled in the art.

The term "osteoinductive" as used herein with reference to an injectable biomaterial refers to the ability of an osteogenic material to recruit cells from the host that have the potential for forming new bone and repairing bone tissue. Osteoinductive injectable biomaterials according to the present disclosure stimulate osteoprogenitor cells to differentiate into osteoblasts that then begin new bone formation. Exemplary osteoinductive materials include, but are not limited to, BMP2, BMP7, BMP9, PDGF, and P15. *See, e.g.,* Neiva, R. F. et al. J. Periodontol. 2008, 79(2), 291-99, the contents of which are incorporated herein by reference in their entirety. Other osteoinductive materials are known to those skilled in the art.

The term "proteolytic enzymes" as used herein refers to enzymes capable of breaking down various proteins. Proteolytic enzymes include, but are not limited to, matrix metalloproteinases (MMPs), tissue inhibitors of metalloproteinases (TIMPs), a disintegrin and metalloproteinase with thrombospondin motifs (ADAM-TS), and mixtures thereof. Other proteolytic enzymes are known to those skilled in the art.

The term "patient" as used herein refers to humans and non-humans such as primates, pets and farm animals.

The term "resorbable" as used herein with reference to an injectable biomaterial refers to the ability of a material to be broken down and assimilated back into the body over time.

The term "self-setting" as used herein with reference to an injectable biomaterial refers to the ability of the material to form an apatitic crystal structure as a result of the mixing of the solid component and the liquid component. In contrast, certain prior art materials provide an apatitic crystal structure, *e.g*., by mixing pre-formed hydroxyapatite with a cement-forming material. Moreover, certain such cement-forming materials (*e.g*., swellable polymers) lack dimensional stability and are permeable, and are thus inappropriate for preventing ingress of inflammatory and/or non-inflammatory mediators from an adjacent joint space.

The term "seize" as used herein with reference to an injectable biomaterial refers to the rapid conversion of the injectable biomaterial to a material that is uninjectable.

The term "stable" as used herein with reference to an injectable biomaterial refers to the ability of the injectable biomaterial, or its precursors, to maintain sufficient physical and/or chemical properties such that it still functions for its intended purpose in accordance with the present disclosure after a period of time has elapsed. For example, stability includes, but is not limited to, the ability of the injectable biomaterial to mix, set, and/or cure.

The terms "subchondral bone plate" and "cortical bone plate" are used herein interchangeably, and refer to the thin cortical lamella lying immediately beneath the calcified cartilage.

The term "total porous area" as used herein refers to the total sum of all pore wall area derived from the volume of each incremental intrusion step. Assuming cylindrical pores, the wall area of each step *i* is A*ᵢ*= 4 V*ᵢ*/D*ᵢ*, where V*ᵢ*= pore volume and D*ᵢ* = pore diameter. *See* Webb, P. An introduction to the physical characterization of materials by mercury intrusion porosimetry with emphasis on reduction and presentation of experimental data, Micromeritics Instrument Corp. (2001), the contents of which are incorporated herein by reference in their entirety.

The terms "treatment," "treating," "treat," "therapy," "therapeutic," and the like are used herein to refer generally to attempting to obtain a desired pharmacological, biological, and/or physiological effect. The effect may be prophylactic in terms of completely or partially preventing or delaying the onset of a condition or symptom thereof and/or may be therapeutic in terms of a partial or complete stabilization, amelioration, or remedying of the condition or symptom.

The term "true density" as used herein refers to the mass divided by the solid volume or true skeletal volume. True density is usually determined after the substance has been reduced to a particle size so small that it accommodates no internal voids. *See* Webb, P. An introduction to the physical characterization of materials by mercury intrusion porosimetry with emphasis on reduction and presentation of experimental data, Micromeritics Instrument Corp. (2001), the contents of which are incorporated herein by reference in their entirety. True density can be measured, *e.g*., by helium pycnometry (such as by use of an AccuPyc II 1340 pycnometer).

The term "working time" as used herein with reference to an injectable biomaterial refers to the maximum amount of time between (1) the mixing of the solid component and the liquid component and (2) the point where the injectable biomaterial is no longer workable. The injectable biomaterial is workable if, after the elapsed time after mixing the solid component and the liquid component for one minute and injecting into an area of degenerate bone (or a substitute such as a sawbone model), the injectable biomaterial flows to fill the porosity of the area of administration prior to setting. The injectable biomaterial is no longer workable if, after the elapsed time after mixing the solid component and the liquid component for one minute and injecting into an area of degenerate bone (or a substitute such as a sawbone model, the injectable biomaterial sets prior to filling the porosity of the area of administration. Tests for working time are known to those skilled in the art. *See, e.g.,* ASTM C414-03 at 7.2, 8.2 (reapproved 2012), the contents of which are incorporated herein by reference in their entirety.

### Bone Structure

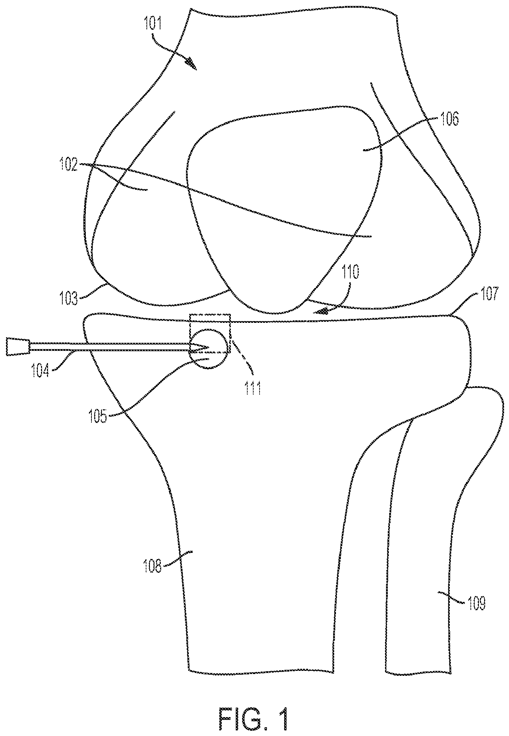

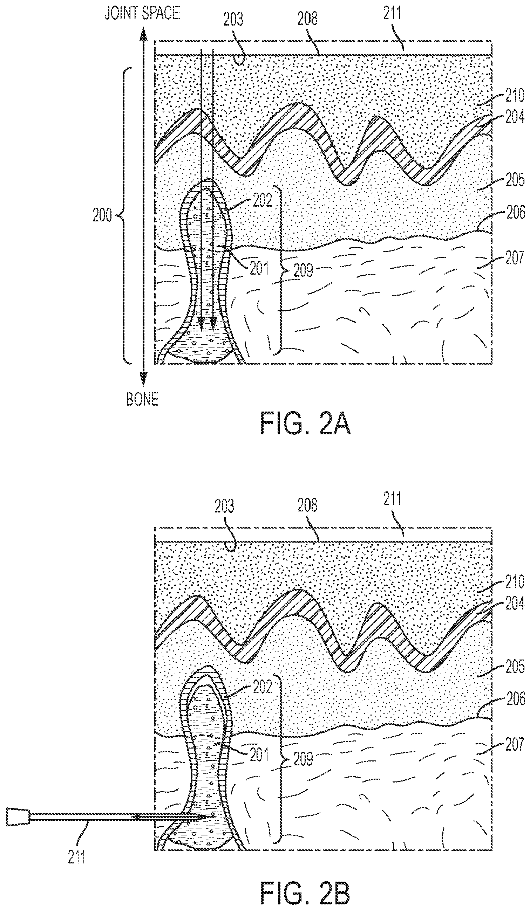

Bone disease often affects the whole of the adjacent joint. FIG. 1 shows a schematic of a joint affected by bone disease. Femur 101 comprises cartilage 102 and femoral articular surface 103, while patella 106 is shown above the joint space 110. The lower portion of FIG. 1 shows fibula 109 and tibia 108, the latter of which comprises an area of degenerate bone 105 into which a cannula 104 is inserted. A portion of tibial articular surface 107 is encompassed by callout 111, which is shown in greater detail in the schematics of FIGs. 2A-E, discussed in more detail as follows.

Subchondral bone plays a crucial role in the initiation and progression of bone diseases such as OA. The term "subchondral bone" as used herein refers to the bony components lying distal to calcified cartilage. Subchondral bone comprises the subchondral bone plate (aka cortical bone plate) and subchondral trabecular bone (aka cancellous bone). The subchondral bone plate and cancellous bone are not divided by a sharp border, but nonetheless are distinct anatomic entities.

As shown in FIGs. 2A-E, the subchondral bone plate is a thin cortical lamella, lying immediately beneath the calcified cartilage 205. This bone plate is not an impenetrable structure, but rather possesses a marked porosity. It is invaded by channels that provide a direct link between articular calcified cartilage and subchondral trabecular bone. A high number of arterial and venous vessels and/or nerves (collectively 202), penetrate through the channels and send branches into calcified cartilage 205. The distribution and intensity of the channels depend not only on the age of the tissues, but also on the magnitude of the compressive forces transmitting through calcified cartilage and subchondral bone within and between joints. These channels are preferentially concentrated in the heavily stressed areas of the joint. Channel shape and diameter also differs with the thickness of the cortical plate. Channels are narrower and form a tree-like mesh in regions where the plate is thicker, while they tend to be wider and resemble ampullae where the subchondral bone plate is thinner.

Arising from the subchondral bone plate is the supporting trabeculae, which are structures that are part of and support the trabecular bone, and also include deeper bone structure. Subchondral trabecular bone exerts important shock-absorbing and supportive functions in normal joints, and may also be important for calcified cartilage nutrient supply and metabolism. Relative to the subchondral bone plate 207, subchondral trabecular bone (not shown, but disposed distal the joint space from the subchondral bone plate 207) is more porous and metabolically active, containing blood vessels, sensory nerves, and bone marrow. Subchondral trabecular bone has an inhomogeneous structure that varies with the distance from the articular surface 203/208. It exhibits significant structural and mechanical anisotropy; that is, the bone trabeculae show preferential spatial orientation and parallelism.

Subchondral bone is a dynamic structure and is uniquely adapted to the mechanical forces imposed across the joint. In addition to bone density patterns and mechanical properties, subchondral bone also dynamically adjusts trabecular orientation and scale parameters in a precise relationship with principal stress. Mechanical stress also modifies the contour and shape of subchondral bone by means of bone modeling and remodeling. Subchondral bone and calcified cartilage are dynamic stress-bearing structures that play complementary roles in load-bearing of joints. Subchondral bone supports overlying articular cartilage 210 and distributes mechanical loads across joint surfaces with a gradual transition in stress and strain. Stiffened and less pliable subchondral bone tends to transmit increased loads to overlying cartilage, leading to secondary cartilage damage and degeneration. The load transmitted to underlying bone is substantially increased after articular cartilage damage or loss.

Articular cartilage overlies subchondral bone, and provides a vital function of maintaining homeostasis of the joint environment. It encompasses superficial non-calcified cartilage 210 and deeper calcified cartilage 205. Calcified cartilage 205 is permeable to small molecule transport, and plays an important role in the biochemical interaction between non-calcified cartilage 210 and subchondral bone. It is separated from non-calcified cartilage 210 by a boundary called the "tidemark" (204), a dynamic structure that appears as a basophilic line in histological sections. The tidemark 204 represents the mineralization front of calcified cartilage 205, and provides a gradual transition between the two dissimilar cartilage regions. Continuous collagen fibrils cross the tidemark, indicating the strong link between non-calcified cartilage 210 and calcified cartilage 205. There is also a sharp borderline between calcified cartilage 205 and subchondral bone, called the "cement line" (206). Unlike the tidemark 204, however, no continuous collagen fibrils cross the cement line.

Given the intimate contact between articular cartilage 210 and subchondral bone, they form a closely composited functional unit called the "osteochondral junction" (200). The osteochondral junction 200 is peculiarly complex, and consists of a layer of non-calcified cartilage 210, the tidemark 204, calcified cartilage 205, the cement line 206 and subchondral bone.

In some embodiments, the affected area of bone is disposed about 50 mm or less from the joint articular surface proximal to the affected area of bone. In some embodiments, the affected area of bone is disposed about 40 mm or less from the joint articular surface proximal to the affected area of bone. In some embodiments, the affected area of bone is disposed about 30 mm or less from the joint articular surface proximal to the affected area of bone. In some embodiments, the affected area of bone is disposed about 20 mm or less from the joint articular surface proximal to the affected area of bone. In some embodiments, the affected area of bone is disposed about 15 mm or less from the joint articular surface proximal to the affected area of bone. In some embodiments, the affected area of bone is disposed about 10 mm or less from the joint articular surface proximal to the affected area of bone. In some embodiments, the affected area of bone is disposed about 9 mm or less from the joint articular surface proximal to the affected area of bone. In some embodiments, the affected area of bone is disposed about 8 mm or less from the joint articular surface proximal to the affected area of bone. In some embodiments, the affected area of bone is disposed about 7 mm or less from the joint articular surface proximal to the affected area of bone. In some embodiments, the affected area of bone is disposed about 6 mm or less from the joint articular surface proximal to the affected area of bone. In some embodiments, the affected area of bone is disposed about 5 mm or less from the joint articular surface proximal to the affected area of bone. In some embodiments, the affected area of bone is disposed about 4 mm or less from the joint articular surface proximal to the affected area of bone. In some embodiments, the affected area of bone is disposed 3 mm or less from the joint articular surface proximal to the affected area of bone. In some embodiments, the affected area of bone is disposed about 2 mm or less from the joint articular surface proximal to the affected area of bone. In some embodiments, the affected area of bone is disposed about 1 mm or less from the joint articular surface proximal to the affected area of bone.

### Etiology of Bone Disease

Articular cartilage is both aneural and avascular. As such, cartilage is incapable of directly generating pain, stiffness (*e.g*., either the symptom of pain on moving a joint, the symptom of loss of range of motion, or the physical sign of reduced range of motion), or any of the symptoms that patients with bone disease typically describe. In contrast, the subchondral bone, periosteum, periarticular ligaments, periarticular muscle spasm, synovium and joint capsule are all richly innervated and can be the source of nociception in bone disease. Furthermore, cross-talk (*i.e*., biochemical communication) between subchondral bone, articular cartilage 210, and joint space 211 is crucial for the initiation and progression of bone disease linked to bone degeneration, in terms of pain, function and pathology. Alterations of any tissue will modulate the properties and functions of other parts of the osteochondral junction 200. There is intensive stress transfer and biochemical cross-talk across this region which plays a role in maintenance and degeneration of the joint.

The permeability of calcified cartilage 205 and subchondral bone plate 207 allows crossover communication, and provides connecting channels between subchondral bone and the joint space 211. *In vivo* studies showed that prostaglandins, leukotrienes and various growth factors released by osteoblasts during subchondral bone remodeling could reach overlying articular cartilage 210. Conversely, inflammatory and osteoclast stimulation factors released by articular cartilage 210 also leads to subchondral bone deterioration through increased bone remodeling in bone disease.

During bone disease linked to bone degeneration, functional units of joints comprising cartilage and subchondral bone can undergo uncontrolled catabolic and anabolic remodeling processes to adapt to local biochemical and biological signals. Changes in cartilage and subchondral bone are not merely secondary manifestations of bone disease but are active components of the disease, contributing to its severity. Increased vascularization and formation of microcracks in joints during bone disease have suggested the facilitation of movement of molecules from the joint space to subchondral bone and *vice versa* through the synovial and bone marrow fluids. Several biological factors and signaling molecules produced from both tissues may passage from one zone to another, affecting homeostasis of neighboring tissue. Secreted cytokines, growth factors and signaling molecules form cartilage-bone biochemical units play modulatory roles to alter pathophysiology of joints during bone disease through pathways such as WNT (wingless type), BMP (bone morphogenic protein), TGF-β (transforming growth factor β) and MAPK (mitogen-activated protein kinase) signaling. The close proximity of cartilage and subchondral bone provides an ample opportunity to induce physical and functional alteration in each other through molecular interaction.

As a result of the biochemical cross-talk between the joint space 211 and subchondral bone, a degenerative biochemical response is initiated which accelerates as biomechanical changes begin to manifest themselves in patients with bone disease. Both the subchondral cortical plate 207 and cancellous bone show distinct differences in their behavior during progression of bone disease and hence must be regarded as separate units to understand the joint deformation events. During progression of bone disease, subchondral bone turnover can be 20-fold increase compared to normal bone turnover. Subchondral bone in bone disease patients secrete high levels of alkaline phosphatase (ALP), osteocalcin, osteopontin, IL-6, IL-8, and progressive ankylosis protein homolog (ANKH), urokinase plasminogen activator (uPA), prostaglandin and growth factors, such as IGF-1, IGF-2 and TGF-β and Type 1 collagen compared to normal subchondral bone. These secreted biochemical factors contribute to bone formation, suggesting an enhanced bone anabolic activity of subchondral bone osteoblasts, exemplified by formation of osteophytes and the sclerosis observed in bone disease. However, the bone forming activity of subchondral bone is not necessarily accompanied by equivalent mineralization. Unmineralized immature new bone formation may lead to abundant osteoids in the subchondral bone (both at the level of the cortical plate and at the level of the trabecular bone) resulting in the opposite effect on tissue properties.

Inflammatory mediators present in synovial fluid also contribute to catabolic activities of chondrocytes leading to remodeling of the cartilage extracellular matrix. Chemokines, cytokines and proteases secreted from chondrocytes and present in the synovial fluid alter biochemical (*e.g*., catabolic) and functional abilities of cartilage. During bone disease, chondrocytes have been found to secrete TNF-α, IL-1, IL1β converting enzyme (caspase-1) and type 1 IL-1 receptor. The concentration at which IL-1 is synthesized by chondrocytes is capable of inducing the activation of proteolytic enzymes, such as matrix metalloproteinases (MMPs), aggrecanases, a disintegrin and metalloproteinase with thrombospondin motifs (ADAM-TS) and other catabolic genes in regions of matrix depletion in affected cartilage. Furthermore, under these conditions, chondrocytes are stimulated to express molecules that are associated with chondrocyte hypertrophy and terminal differentiation, like VEGF, runt-related transcription factor 2 (RUNX2) and MMP-13. Secretion of angiogenic factors such as VEGF increase vascularity within the deep layers of articular cartilage facilitating, together with the presence of microcracks, molecular transport of inflammatory and/or non-inflammatory mediators by diffusion from the joint space and into the articular cartilage and the subchondral bone. Fine, unmyelinated nerves (C-fibers and sympathetic nerves) accompany also these vessels and enervate normally aneural tissues, a source of bone disease pain. In addition, IL-6, in combination with other cytokines like IL1β, can switch osteoblasts from a normal phenotype to a sclerotic phenotype. All these actors potentiate and stimulate the process of bone remodeling, altering the physiology of subchondral bone.

These inflammatory and/or non-inflammatory mediators also affect nociception in the synovial tissue and the subchondral bone. Nociceptors encompass a broad range of receptors for ligands that change the properties of these neurons, such that they require lower thresholds to fire action potentials or even fire spontaneously when the receptors are engaged. These ligands include, but are not limited to, cytokines, chemokines, neuropeptides and prostaglandins, which in some embodiments all form part of the biochemical milieu in the affected joint. As a result of this peripheral sensitization, joint movement within the normal range becomes painful (a phenomenon known as mechanical allodynia).

Furthermore, inflammatory mediators such as bradykinin, histamine, prostaglandins, lactic acid, substance P, vasoactive intestinal peptide, nerve growth factor (NGF), and calcitonin gene related peptide (CGRP) are released into the joint from *e.g.,* synovial fibroblasts and migrate into the subchondral bone and synovium, activating the nociceptors, located in those regions. These mediators reduce the firing threshold of the nociceptors, making them more likely to respond to both non-noxious and noxious painful stimuli. As the disease progresses, more and more of these mediators accumulate in the joint migrate into the subchondral space and synovium, thereby triggering a self-perpetuating cycle of pain generation.

As the damage progresses, degeneration of the subchondral bone becomes radiographically visible. The severity of the joint damage on the radiograph may bear little relation to the severity of the pain experienced. However, utilizing imaging modalities such as magnetic resonance imaging ("MRI"), significant structural associations such as bone degeneration, sub-articular bone attrition, synovitis and effusion have been related to knee pain.

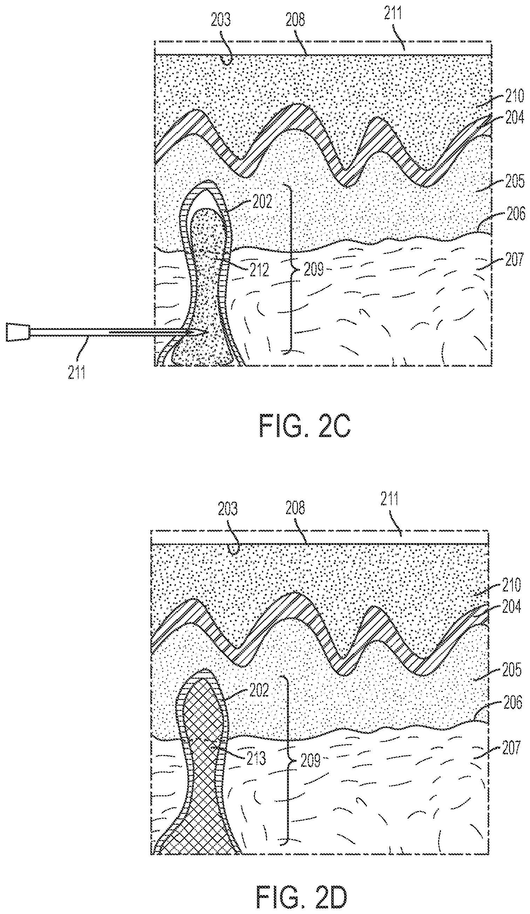

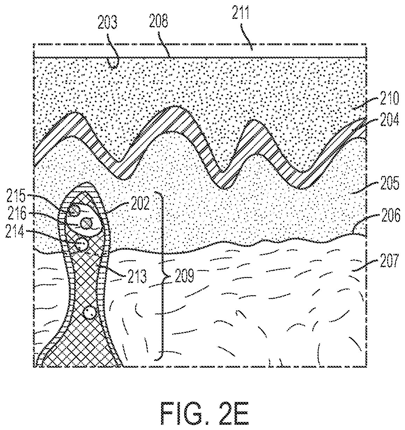

Without wishing to be bound by theory, the inventors posit that methods and compositions disclosed herein address these issues by breaking the biological communication between the joint space and the affected area of bone by optionally aspirating inflammatory and/or non-inflammatory mediators from the subchondral bone and subsequently filling the interconnected pores of the affected area with an injectable biomaterial. FIG. 2A shows a schematic of an exemplary embodiment of a diseased joint that can be treated according to the present disclosure. FIGs. 2A-E are a callout of the area of degenerate bone 105 shown in FIG. 1. Osteochondral junction 200 comprises an area of degenerate bone 209, surrounded by blood vessels/and or nerves 202. A biological fluid comprising inflammatory and/or non-inflammatory mediators 201 have permeated from the joint space 211 past the articular surface 203/208 through articular cartilage 210, tidemark 204, calcified cartilage 205 and have collected in degenerate area of bone 209. In an exemplary embodiment shown in FIG. 2B, cannula 211 is inserted into the area of degenerate bone 209 and the biological fluid 201 is aspirated through the cannula 211 and removed. As shown in FIG. 2C, injectable biomaterial 212 prepared according to the present disclosure fills the area of degenerate bone 209 in the affected area. As shown in FIG. 2D, the injectable biomaterial cures to form a low porosity, high dimensional stability material 213, thereby halting biochemical communication to and from the joint space and protecting the affected area and surrounding cells. Moreover, the injectable biomaterial remains in place for a period of time, preventing the re-infiltration of these inflammatory and/or non-inflammatory mediators. Accordingly, biochemical communication between the affected area of bone and the joint space is arrested by blocking the porosity connecting the affected area of bone and joint space, temporally breaking biochemical communication and allowing the affected area of bone to recover. By shielding the affected area of bone from these actors, arthritic degeneration of the joint is prevented. The latter results from the prevention of further biochemical degeneration of the bone and of the adjacent meniscal and cartilage tissues, and alleviation of the corresponding pain in the joint. Furthermore, certain injectable biomaterials according to the present disclosure provide for biomechanical repair of the affected area, such as by the provision of osteoconductive and/or osteoinductive surfaces to encourage natural bone healing processes, shifting the damage/repair equilibrium toward repair. This exemplary embodiment is shown in FIG. 2E, where osteoclasts 214 resorb the cured injectable biomaterial 213 and osteoblasts 215 begin to build new, healthy bone 216. Importantly, the compositions and methods disclosed herein achieve their intended effects, namely cessation of the underlying causes of bone disease linked to bone degeneration, without further altering the biomechanical stability of the joint being treated or causing significant pain post-operatively that can be associated with volume expansion from gas. They therefore offer several advantages over prior art treatments for bone disease.

### Identification of Bone for Treatment

In some embodiments, an affected area for treatment according to the compositions and methods disclosed herein is identified by the use of MRI. In further embodiments, the MRI is a knee MRI. In further embodiments, the MRI is an ankle MRI. In some embodiments, the MRI is weight-bearing MRI. In some embodiments, the MRI is open MRI. In some embodiments, the MIR is upright open MRI. In further embodiments, the MRI is low field strength MRI. In further embodiments, the MRI is an ultra-high field MRI. In further embodiments, the MRI is extremity MRI. In further embodiments, the MRI is whole body scanner MRI. In some embodiments, the affected area is identified by hyperintense signals on T2-weighted fat saturated MRI images. In some embodiments, MRI T1ρ value, an indicator of early cartilage degradation, is elevated in cartilage overlying bone marrow lesions, with the level of cartilage degradation proportional to T1ρ signal intensity in a bone marrow lesion. In further embodiments, the affected area is identified using Technetium-99 bone scans. In some embodiments, the affected area is identified using fluoroscopy.

Without wishing to be bound by theory, the inventors posit that affected areas of bone thought to be associated with arthritis are disposed less than 50 mm, 10 mm or 1 mm from the joint. Accordingly, in some embodiments, the methods and compositions disclosed herein are used to treat affected areas of bone which are disposed between about 0 mm to about 50 mm from the joint. In further embodiments, the affected area of bone is disposed between about 0 mm to about 10 mm from the joint. In still further embodiments, the affected area of bone is disposed between about 0 mm to about 1 mm from the joint. In some embodiments, the affected area of bone is disposed about 40 mm or less from the joint. In some embodiments, the affected area of bone is disposed about 20 mm or less from the joint. In some embodiments, the affected area of bone is disposed about 15 mm or less from the joint. In some embodiments, the affected area of bone is disposed about 10 mm or less from the joint. In some embodiments, the affected area of bone is disposed about 9 mm or less from the joint. In some embodiments, the affected area of bone is disposed about 8 mm or less from the joint. In some embodiments, the affected area of bone is disposed about 7 mm or less from the joint. In some embodiments, the affected area of bone is disposed about 6 mm or less from the joint. In some embodiments, the affected area of bone is disposed about 5 mm or less from the joint. In some embodiments, the affected area of bone is disposed about 4 mm or less from the joint. In some embodiments, the affected area of bone is disposed 3 mm or less from the joint. In some embodiments, the affected area of bone is disposed about 2 mm or less from the joint. In some embodiments, the affected area of bone is disposed about 1 mm or less from the joint.

In some embodiments, the affected area of bone comprises a bone marrow lesion. In further embodiments, the affected area of bone comprises degenerate cancellous bone space.

In some embodiments, the location of administration of injectable biomaterial is determined by studying a previously captured image of the affected area. In further embodiments, the location of administration of injectable biomaterial is determined using additional guidance during surgery. In some embodiments, the additional guidance comprises real-time fluoroscopic imaging. In further embodiments, the additional guidance comprises robotic devices. In further embodiments, the additional guidance comprises braces for maintaining the joint in a position consistent with previously captured images of the joint. In further embodiments, the additional guidance comprises the use of one or more labels. In some embodiments, the one or more labels comprise radioactive labels. In some embodiments, the radioactive labels comprise Technetium-99. In some embodiments, the one or more labels comprise radioactive label fiducial markers.

### Injectable Biomaterials

The injectable biomaterial disclosed herein is a physiologically compatible material that fills the porosity in the affected area of bone which is symptomatic of bone disease. Certain properties of the injectable biomaterial, namely that it is injectable, intermixable, flowable, is cohesive, and adherent to bone, allow the biomaterial to fill the porosity that exists in the affected area of bone without being readily cleared away by bodily fluids.

The injectable biomaterials disclosed herein comprise a solid component and a liquid component that comprises a carbohydrate. The injectable biomaterials disclosed herein can be prepared by mixing of the solid component and the liquid component. The injectable biomaterials disclosed herein set and cure to form an apatitic crystal structure after mixing of the solid component and the liquid component. The solid component provides a solid material, or mix of materials, that reacts when combined with the liquid component to form the apatitic crystal structure. The liquid component provides a medium for the components of the solid component to mix and react to form the apatitic crystal structure. In some embodiments, the solid component comprises a calcium phosphate. In some embodiments, the liquid component comprises water. In some embodiments, the solid component and/or the liquid component include additional components.

The liquid component of the injectable biomaterials disclosed herein includes a carbohydrate. While the carbohydrate itself may not be a liquid, when provided as a constituent of the liquid component, it is dissolved or suspended therein. In some embodiments, the liquid component is in the form of a gel or a hydrogel.

In some embodiments, the solid component and the liquid component are mixed at a particular ratio to achieve the desired injectable biomaterial. In some embodiments, the ratio of solid component to liquid component is about 3 to about 1 by mass. In some embodiments, the ratio of solid component to liquid component is about 2 to about 1 by mass. In some embodiments, the ratio of solid component to liquid component is about 1.5 to about 1 by mass. In some embodiments, the ratio of solid component to liquid component is about 1 to about 1 by mass. In some embodiments, these ratios of solid component to liquid component provide an injectable biomaterial that is injectable. While prior art materials comprising a solid component and a liquid component comprising a carbohydrate are disclosed, these materials utilize a higher powder-to-liquid ratio than is achievable according to the present disclosure. *See, e.g.,* Ahmadzadeh-Asl, S. et al. Adv. Applied Ceramics 2011, 110(6), 340-45, the contents of which are incorporated herein by reference in their entirety. Accordingly, these materials are not intermixable and require high extrusion forces to dispense the entirety of the material, resulting in compositions that cannot be readily administered in a minimally invasive fashion with the ease of the presently disclosed injectable biomaterials. In contrast, the injectable biomaterials of the present disclosure are readily intermixable, such that the solid component and liquid component can be provided in separate syringes, which are coupled to one another, the contents intermixed and then directly administered to an area of degenerate bone. This property not only provides for an injectable biomaterial with additional convenience, ease of mixing and use, but also reduced chance of contamination. By contrast, prior art materials made using higher powder-to-liquid ratios lack intermixability such that they must be manually mixed in a container and subsequently transferred to a syringe, such as by a spatula, increasing the changes for contamination and compromising of sterility. *See, e.g.,* Ahmadzadeh-Asl, S. et al. Adv. Applied Ceramics 2011, 110(6), 340-45, at 341, the contents of which are incorporated herein by reference in their entirety. The inventors have surprisingly discovered that, contrary to conventional wisdom, the reduced powder-to-liquid ratios achievable with the injectable biomaterials according to the present disclosure are still able to set in a commercially feasible time, remain cohesive, adhesive to bone, and provide the reduced strength desired by the inventors to prevent biomechanical stabilization. Moreover, prior art materials that utilized carbohydrates began with materials capable of setting, and added a carbohydrate to improve flowability and injectability. In contrast, the inventors surprisingly discovered that the addition of a carbohydrate to injectable biomaterials that were not capable of setting or remaining cohesive surprisingly were able to set and remain cohesive by virtue of the carbohydrate addition.

Exemplary injectable biomaterials according to the present disclosure can comprise calcium phosphate cements, settable polymers such as lysine diisocyanates, proteins, such as collagen, gelatin and their derivatives, and carbohydrates, such as hyaluronic acid, alginates, chitosan, cellulose, dextran and their derivatives. In further embodiments, the injectable biomaterial comprises bone, such as autografts, allografts, and artificial or synthetic bone substitutes. In certain embodiments, the injectable biomaterial comprises one or more of platelet-rich plasma ("PRP"), platelet-poor plasma ("PPP"), bone marrow aspirate ("BMA"), bone marrow aspirate concentrate ("BMAC"), or cell lysates. In further embodiments, the injectable biomaterial comprises at least one polymeric material.

In some embodiments, the injectable biomaterials disclosed herein are self-setting. In some embodiments, the self-setting injectable biomaterials disclosed herein set and cure to form an fully set and cured injectable biomaterial that has a major phase of hydroxyapatite. In further embodiments, the major phase is at least 95% hydroxyapatite. *See* ASTM F1185-03 (reapproved 2014), at 4.2; ISO 13175-3 (2012), at 4.2.2, the contents of all of the foregoing of which are incorporated herein by reference in their entireties.

In some embodiments, the injectable biomaterials disclosed herein are flowable. In contrast, prior art materials lack sufficient flowability to be able to be injected into an area of degenerate bone such that they flow into and substantially fill the area. While some prior art materials were capable of being injected, they cease flowing when backpressure ceases. Accordingly, these materials tend to stay resident at the immediate location of administration, rather than flowing to fill in the increased porosity present in the area of degenerate bone. Additionally, these materials tend to set prior to administration in full, and/or seize in the instrumentation. Accordingly, these materials were incapable of providing the requisite barrier to prevent influx of inflammatory and/or non-inflammatory mediators from the adjacent joint space.

In some embodiments, the solid component can comprise multiple constituents. In some embodiments, the constituents react to form an apatitic crystal structure. In some embodiments, the constituents are provided as solid powders. In some embodiments, the particle sizes of the powders of the constituents can be adjusted to effect the desired setting and curing times. For example, the particle size of constituents can be reduced to provide for faster reaction with other constituents, consequently shortening the initial setting time. Conversely, the particle size of constituents can be increased to provide for slower reaction with other constituents, consequently lengthening the initial setting time. *See, e.g.,* Bohner, M. et al. J. Mater. Chem. 2008, 18, 5669-75, the contents of which are incorporated herein by reference in their entirety. In some embodiments, the solid component is substantially free of bicarbonate.

In accordance with certain aspects of the present invention, the injectable material can be injected into bone to fill the affected area. In some embodiments, the injection volume of biomaterial is from about 1 to about 6 mL. In some embodiments, the injection volume of biomaterial is about 6 mL. In some embodiments, the injection volume of biomaterial is about 5 mL. In some embodiments, the injection volume of biomaterial is about 4 mL. In some embodiments, the injection volume of biomaterial is about 3 mL. In some embodiments, the injection volume of biomaterial is about 2 mL. In some embodiments, the injection volume of biomaterial is about 1 mL. Without wishing to be bound by theory, the inventors posit that the injectable biomaterial disclosed herein shields the affected area of bone from inflammatory and/or non-inflammatory mediators, thereby arresting, preventing and/or reversing degeneration of the j oint. These effects result from the prevention of further biochemical degeneration of the bone and of the adjacent meniscal and cartilage tissues, and alleviation of the corresponding pain in the joint. Furthermore, the injectable biomaterials disclosed herein provide for biomechanical repair of the affected area, such as by the provision of osteoconductive and/or osteoinductive surfaces to encourage natural bone healing processes, shifting the damage/repair equilibrium toward repair. Importantly, the injectable biomaterials disclosed herein achieve their attended effects, namely cessation of the underlying causes of bone disease, without further altering the biomechanical stability of the joint being treated or causing significant pain post-operatively that can be associated with volume expansion from gas.

In some embodiments, the injectable biomaterial has suitable viscosity such that it can be injected into the affected area from a syringe through an 10-21 gauge cannula. In some embodiments, the injectable biomaterial can be injected using pressure applied by no more than average hand and finger strength. Typical ranges of average hand and finger strength are known in the art, and are disclosed, for example, in DiDomenico, A.; Nussbaum, M. A. Ergonomics 2003, 46(15), 1531-1548, the contents of which are hereby incorporated by reference in their entirety. In some embodiments, an injectable biomaterial is able to be extruded from a syringe using no more than 15 lb. extrusion force at a rate of 6 mL/minute. In some embodiments, an injectable biomaterial is able to be extruded from a syringe using no more than 10 lb. extrusion force at a rate of 6 mL/minute. In some embodiments, an injectable biomaterial is able to be extruded from a syringe using no more than 5 lb. extrusion force at a rate of 6 mL/minute.

In some embodiments the flowability comprises sufficient flowability such that the injectable biomaterial flows into porosity in the bone in the affected area prior to initially setting and/or curing. In some embodiments, the injectable biomaterial flows into porosity in the bone as a result of hand pressure applied to the syringe from which it is expelled.

In some embodiments, the injectable biomaterial disclosed herein is cohesive. The cohesiveness of the injectable biomaterial manifests in the ability of the material to resist phase separation (*e.g*., dewatering) over the time required to prepare and inject the material into the affected area, while simultaneously maintaining sufficient flowability such that the material can still be injected and caused to flow through the porosity of the degenerate bone.

In some embodiments, the liquid component has a pH that can be modified to achieve the desired working, initial setting, and curing times. *See, e.g.,* Bohner, M. et al. J. Mater. Chem. 2008, 18, 5669-75, the contents of which are incorporated herein by reference in their entirety. For example, if lower working, initial setting, and curing times are desired, the pH of the liquid component may be lowered. In some embodiments, the pH of the liquid component is between about 3 and about 8. In some embodiments, the pH of the liquid component is between about 3 and about 7. In some embodiments, the pH of the liquid component is between about 3 and about 6. In some embodiments, the pH of the liquid component is between about 4 and about 6. In some embodiments, the pH of the liquid component is between about 5 and about 6. In some embodiments, the pH of the liquid components is about 6. In some embodiments, the pH of the liquid component is adjusted using a pH adjusting agent. In some embodiments, the pH adjusting agent is selected from an organic acid and an inorganic acid. In some embodiments, the pH adjusting agent is selected from the group consisting of citric acid, formic acid, acetic acid, and mixtures thereof. In some embodiments, the pH adjusting agent s selected from the group consisting of hydrochloric acid, phosphoric acid, nitric acid, and mixtures thereof. In some embodiments, the pH adjusting agent is citric acid.

In some embodiments, the liquid component comprises a salt whose concentration may also be modified to modify the desired working, initial setting, and curing times. For example, if lower working, initial setting, and curing times are desired, the salt concentration of the setting solution may be raised. In some embodiments, the liquid solution comprises a salt present at a concentration of about 0.01 to about 10 M. In further embodiments, the concentration of the salt is from about 0.1 to about 1 M. In further embodiments, the concentration of the salt is from about 0.2 to about 0.4 M. In further embodiments, the concentration of the salt is from about 0.3 M. In some embodiments, the salt is sodium phosphate dibasic, sodium silicate, sodium chloride, calcium hydroxide, or mixtures thereof. In some embodiments, the salt is sodium phosphate dibasic.

In some embodiments, the liquid component comprises water as the solvent.

In some embodiments, the injectable biomaterial cures to form a material having a molar calcium to phosphorus ("Ca/P") ratio of about 1 to about 2. In further embodiments, the material has a molar Ca/P ratio of about 1.3 to about 1.8. In further embodiments, the material has a molar Ca/P ratio of about 1.4 to about 1.7. In further embodiments, the material has a molar Ca/P ratio of about 1.5 to about 1.7. In further embodiments, the material has a molar Ca/P ratio of about 1.5 to about 1.667. Ca/P ratios can be determined according to methods known in the art. In some embodiments, the Ca/P ratio is calculated theoretically. In some embodiments, the Ca/P ratio is calculated using inductively-coupled plasma mass spectroscopy ("ICP-MS"). In some embodiments, the Ca/P ratio is calculated using ion chromatography.

In some embodiments, the injectable biomaterial disclosed herein is adherent to bone. In some embodiments, injectable biomaterial demonstrates sufficient adherence to bone such that it remains resident at the location of administration for sufficient time to prevent the re-infiltration of inflammatory and/or non-inflammatory mediators and to allow the damaged bone to heal. In some embodiments, the injectable biomaterial remains substantially resident at the location of administration for up to about 30 days. In some embodiments, the injectable biomaterial remains substantially resident at the location of administration for up to about 2 months. In some embodiments, the injectable biomaterial remains substantially resident at the location of administration for up to about 3 months. In some embodiments, the injectable biomaterial remains substantially resident at the location of administration for up to about 6 months. In some embodiments, the injectable biomaterial remains substantially resident at the location of administration for up to about one year. In some embodiments, the injectable biomaterial remains substantially resident at the location of administration for up to about 18 months. In some embodiments, the injectable biomaterial remains substantially resident at the location of administration for up to about 2 years. In some embodiments, the injectable biomaterial remains substantially resident at the location of administration for up to about 30 months. In some embodiments, the injectable biomaterial remains substantially resident at the location of administration for up to about 3 years. In some embodiments, the injectable biomaterial is resident at the location of administration until the injectable biomaterial is completely resorbed. Resorption is the process by which osteoclasts break down the injectable biomaterial and replace it with healthy bone. *See, e.g.,* Sheikh, Z. et al. Materials, 2015, 8, 7913-25. In some embodiments, the adherence of the injectable biomaterial to bone prevents the permeation of inflammatory and/or non-inflammatory mediators into the affected area, allowing the degenerate bone to heal and/or repair. In some embodiments, degenerate bone healing and/or repair prevents further cartilage damage.

In some embodiments, the injectable biomaterial is resorbable over time. In some embodiments, the injectable biomaterial is completely resorbed in about 30 days. In some embodiments, the injectable biomaterial is completely resorbed in about 2 months. In some embodiments, the injectable biomaterial is completely resorbed in about 3 months. In some embodiments, the injectable biomaterial is completely resorbed in about 6 months. In some embodiments, the injectable biomaterial is completely resorbed in about one year. In some embodiments, the injectable biomaterial is completely resorbed in about 18 months. In some embodiments, the injectable biomaterial is completely resorbed in about 2 years. In some embodiments, the injectable biomaterial is completely resorbed in about 30 months. In some embodiments, the injectable biomaterial is completely resorbed in about 3 years.

In some embodiments, the injectable biomaterial disclosed herein is macroporous when set or cured. Macroporosity allows for the infiltration of endogenous cells from the host. Without wishing to be bound by theory, the inventors posit that macroporosity allows endogenous cells to stimulate bone remodeling at the affected area.

In some embodiments, the injectable biomaterial possesses sufficient cohesion prior to setting and curing such that it remains in the location of administration, but lacks the compressive strength post-curing that would be required to substantially alter or support the existing biomechanics of the joint or biomechanically stabilize the affected area.

Consequently, an injection that changes the stress distribution within a bone will change the structure of the bone as well. Moreover, the provision of biomechanical support to an area of degenerate bone alone fails to address the underlying causes of bone disease and/or its symptoms and can lead to increases in biomechanical instability in other areas of the joint according to Wolff's law. Accordingly, the methods and compositions of the present disclosure do not directly prevent further degeneration of the bone by providing biomechanical support, but instead provide a biochemical environment to the affected area of bone which shields the bone from the effect of inflammatory and/or non-inflammatory actors and thus allows the bone to naturally heal and restore its original condition without Wolff's Law intervention.

In some embodiments, the injectable biomaterial disclosed herein includes an osteoinductive component. Osteoinductive injectable biomaterials according to the present disclosure have the ability to cause precursor cells (such as osteoprogenitors or mesenchymal stem cells) to differentiate into osteoblasts that then begin new bone formation in the affected area, thus facilitating rebuilding of healthy bone. Exemplary osteoinductive components suitable for use in the injectable biomaterials disclosed herein include, but are not limited to, bone morphogenetic proteins ("BMP," *e.g*., rhBMP-2), transforming growth factors (*e.g.,* transforming growth factor beta or "TGF-beta"), osteoblast cells, polymers such as hyaluronic acid, poly-hydroxyethylmethacrylate ("Poly-HEMA"); metals such as titanium; various forms of calcium phosphates including hydroxyapatite, tricalcium phosphate, natural ceramics such as hydroxyapatite and hydroxyapatite/calcium carbonate, including those derived from coral exoskeleton; synthetic non-sintered calcium phosphate ceramics such as tricalcium phosphate, dicalcium phosphate dihydrate, dicalcium phosphate anhydrous, hydroxyapatite, biphasic calcium phosphate, and octacalcium phosphate; synthetic sintered calcium phosphates such as pyrophosphate, hydroxyapatite, biphasic calcium phosphate, tricalcium phosphate, and carbonated apatite; other ceramics such as aluminum oxide, Bioglass^{®}, and Pyrex^{®}; composites such as hydroxyapatite/poly(D,L-lactide), and various other organic species known to induce bone formation by those of skill in the art. In some embodiments, the injectable biomaterial is prepared using a dilute suspension of type I collagen. In some embodiments, the osteoinductive component is BMP. In some embodiments, the osteoinductive component is TGF-beta. In some embodiments, the osteoinductive component is selected from PRP, PPP, BMA conditional media, BMAC, BMA lysate, cell lysates, and mixtures thereof.

In some embodiments, the injectable biomaterials disclosed herein including an osteoconductive component. Osteoconductive injectable biomaterials according to the present disclosure provide a scaffold or framework for new bone growth that is perpetuated by the native bone, thus facilitating rebuilding of healthy bone. Osteoblasts from native bone are supported by osteoconductive injectable biomaterials as they form new bone. Exemplary osteoconductive components suitable for use in the injectable biomaterials disclosed herein include, but are not limited to, demineralized bone matrix ("DBM"), collagen, autograft, allograft, synthetic scaffolds, and mixtures thereof.

In some embodiments, osteoconductive and/or osteoinductive properties are provided by bone marrow, blood plasma, morselized bone of the patient, or commercially available materials. In some embodiments, osteoconductive and/or osteoinductive properties are provided by hydroxyapatite, tricalcium phosphate, CaSO₄, and/or other materials known to those of skill in the art.

In some embodiments, the injectable biomaterials disclosed herein have a working time sufficient to allow a person skilled in the art to administer the injectable biomaterial to an affected area in a patient after mixing of the solid component and the liquid component prior to the transition of the injectable biomaterial to a material that it is no longer injectable.

In some embodiments, the properties of the injectable biomaterial disclosed herein are obtained after injection. For example, in some embodiments the injectable biomaterial is less adherent to bone prior to injection, but becomes more adherent to bone after injection into the affected area. In some embodiments, the injectable biomaterial becomes more adherent to bone after initially setting. In some embodiments, the injectable biomaterial becomes more adherent to bone after curing.

In some embodiments, the setting or curing of the injectable biomaterial is not significantly exothermic. In some embodiments, the setting or curing of the injectable biomaterial is isothermic. Release of heat during the setting and/or curing of the injectable biomaterial *in situ* can result in damage to the surrounding tissues, and thus an injectable biomaterial that sets and/or cures isothermically can prevent damage to these tissues.

In some embodiments, the carbohydrate is selected from the group consisting of dextran, alginate, carboxymethylcellulose, and hyaluronic acid. In some embodiments, the carbohydrate is hyaluronic acid. In some embodiments, inclusion of hyaluronic acid in the injectable biomaterial improves the intermixability, flowability and cohesion of the injectable biomaterial, while providing a material that sets and cures to form an apatitic crystal structure. In some embodiments, the inclusion of hyaluronic acid in the injectable biomaterial weakens the injectable biomaterial to prevent biomechanical stabilization. In some embodiments, the injectable biomaterial including hyaluronic acid disclosed herein exhibit anti-inflammatory properties, which function to dampen the inflammatory milieu causing destruction of the subchondral bone.

In some embodiments, the injectable biomaterial bonds to the bone. In further embodiments, the injectable biomaterial attaches to the bone. In further embodiments, the injectable biomaterial adheres to the bone. In some embodiments, the bonds are formed by biological processes *in situ.*

In certain embodiments, the injectable biomaterial is in the form of a fluid. In further embodiments, the injectable biomaterial is in the form of a viscous liquid having a viscosity between about 5 Pa s and about 30 Pa s at room temperature. In some embodiments, the viscosity is between about 5 Pa s to about 25 Pa s. In some embodiments, the viscosity is between about 5 Pa·s to about 24 Pa·s. In some embodiments, the viscosity is between about 5 Pa s to about 23 Pa s. In some embodiments, the viscosity is between about 5 Pa s to about 22 Pa s. In some embodiments, the viscosity is between about 5 Pa s to about 21 Pa s. In some embodiments, the viscosity is between about 5 Pa s to about 20 Pa s. In some embodiments, the viscosity is between about 5 Pa·s to about 19 Pa·s. In some embodiments, the viscosity is between about 5 Pa s to about 18 Pa s. In some embodiments, the viscosity is between about 5 Pa·s to about 17 Pa·s. In some embodiments, the viscosity is between about 5 Pa·s to about 16 Pa·s. In some embodiments, the viscosity is between about 5 Pa·s to about 15 Pa·s. In some embodiments, the viscosity is between about 5 Pa·s to about 14 Pa s. In some embodiments, the viscosity is between about 5 Pa s to about 13 Pa s. In some embodiments, the viscosity is between about 5 Pa s to about 12 Pa s. In some embodiments, the viscosity is between about 5 Pa·s to about 11 Pa·s. In some embodiments, the viscosity is between about 5 Pa s to about 10 Pa·s. In some embodiments, viscosity is measured immediately after the mixing of the solid component and the liquid component. In further embodiments, the injectable biomaterial is in the form of a semi-solid. In further embodiments, the injectable biomaterial is in the form of a gel. In further embodiments, the injectable biomaterial is in the form of a hydrogel. In further embodiments, the injectable biomaterial is in the form of a dispersion. In further embodiments, the injectable biomaterial is in the form of a slurry.

In some embodiments, the injectable biomaterial remains in its originally-injected state after preparation and/or injection. In further embodiments, the injectable biomaterial initially sets to a less fluid state after preparation and/or injection.

In some embodiments, the injectable biomaterial converts from a liquid to form a semi-solid after preparation and/or injection. In some embodiments, the injectable biomaterial converts from a liquid to form a semi-solid after preparation and/or injection over a working time. In some embodiments, the injectable biomaterial converts from a liquid to form a semi-solid after preparation and/or injection over an initial setting time. In some embodiments, the injectable biomaterial converts from a liquid to form a semi-solid after preparation and/or injection over a curing time. In some embodiments, the injectable biomaterial converts from a liquid to form a gel after preparation and/or injection. In some embodiments, the injectable biomaterial converts from a liquid to form a gel after preparation and/or injection over a working time. In some embodiments, the injectable biomaterial converts from a liquid to form a gel after preparation and/or injection over an initial setting time. In some embodiments, the injectable biomaterial converts from a liquid to form a gel after preparation and/or injection over a curing time. In some embodiments, the injectable biomaterial converts from a liquid to form a solid after preparation and/or injection. In some embodiments, the injectable biomaterial converts from a liquid to form a solid after preparation and/or injection over a working time. In some embodiments, the injectable biomaterial converts from a liquid to form a solid after preparation and/or injection over an initial setting time. In some embodiments, the injectable biomaterial converts from a liquid to form a solid after preparation and/or injection over a curing time.

In some embodiments, the injectable biomaterial is provided in a syringe. In some embodiments, the injectable biomaterial is provided in a syringe that is coupled to a cannula. In some embodiments, the injectable biomaterial is injected into the bone so as to form an injectable biomaterial *in situ.* In some embodiments, an opening is created in the bone prior to injection of the injectable biomaterial.

In some embodiments, the injectable biomaterial is formed in a syringe. In some embodiments, the solid component is disposed in a first syringe. In some embodiments, the liquid component is disposed in a second syringe. In some embodiments, at least one of the first and the second syringes comprises an integrated mixing system. In some embodiments, the injectable biomaterial is provided by injecting the contents of the second syringe into the first syringe, thereby combining the solid component and the liquid component. In some embodiments, the solid component and the liquid component are mixed by repeated extrusion between the first and second syringes. In some embodiments, the solid component and the liquid component are mixed by use of the integrating mixing system. Integrated mixing systems are known in the art, such as the Medmix^{®} P-System and F-system. *See, e.g.,* Bone-Cement Delivery System (P-System), available at http://www.medmix.ch/portofolio-item/bone-cement-delivery-system-p-system/ (last visited April 20, 2017). In some embodiments, the first syringe is coupled to the second syringe. In some embodiments, the coupling is by Luer lock. In some embodiments, the Luer-Lock is then disconnected and the first syringe is capped with an end cap. In some embodiments, the mixture in the first syringe is then mixed using the integrated mixing system to form the injectable biomaterial.

In some embodiments, the injectable biomaterial and/or the containers in which it or its precursors are stored are sterile. In some embodiments, the sterility comprises a condition in which an object has a sterility assurance level (SAL) of 10⁻³ or less. In further embodiments, the sterility comprises a condition in which an object has a SAL of 10⁻⁶ or less. In some embodiments, the SAL is determined in accordance with current FDA guidelines for medical devices. In some embodiments, the injectable material is sterile for up to up to 5 years.

In some embodiments, the injectable biomaterial has a shelf life of at least about 3 months. In some embodiments, the injectable biomaterial has a shelf life of at least about 6 months. In some embodiments, the injectable biomaterial has a shelf life of at least about 1 year. In some embodiments, the injectable biomaterial has a shelf life of at least about 18 months. In some embodiments, the injectable biomaterial has a shelf life of at least about 2 years. In some embodiments, the injectable biomaterial has a shelf life of at least about 3 years. In some embodiments, the injectable biomaterial has a shelf life of at least about 4 years. In some embodiments, the injectable biomaterial has a shelf life of at least about 5 years.

### Methods of Treatment

The compositions and methods disclosed herein are useful for the treatment of degenerate bone in a patient. In some embodiments, the degenerate bone is disposed in an affected area of bone. In some embodiments, the affected area or bone is a region of bone that exhibits inflammatory and/or degradative changes as a result of inflammatory and/or non-inflammatory mediators. In some embodiments, the methods and compositions disclosed herein are useful for the treatment of bone disease in a patient.

In some embodiments, the methods and compositions disclosed herein are useful for the treatment of j oint pain in an affected area. In some embodiments, the methods and compositions disclosed herein are useful for the treatment of bone pain in an affected area. In some embodiments, the methods and compositions disclosed herein are useful for the treatment of arthritic pain in an affected area. In some embodiments, the affected area is a knee. In further embodiments, the affected area is a hip. In further embodiments, the affected area is a shoulder. In further embodiments, the affected area is an ankle. In further embodiments, the affected area is a wrist. In further embodiments, the affected area is an elbow. In further embodiments, the affected area is a vertebrae. In further embodiments, the affected area is a hand.

In some embodiments, the methods and compositions disclosed herein are useful for the treatment of arthritis in an affected joint. In some embodiments, the arthritis is OA. In some embodiments, the arthritis is rheumatoid arthritis. In some embodiments, the affected joint is a knee. In further embodiments, the affected joint is a hip. In further embodiments, the affected joint is a shoulder. In further embodiments, the affected joint is an ankle. In further embodiments, the affected joint is a wrist. In further embodiments, the affected joint is an elbow. In further embodiments, the affected joint is a vertebrae. In further embodiments, the affected joint is a join proximal to a hand.

In some embodiments, the methods and compositions disclosed herein are useful for the treatment of avascular necrosis. In some embodiments, the affected joint is a knee. In further embodiments, the affected joint is a hip. In further embodiments, the affected joint is a shoulder. In further embodiments, the affected joint is an ankle. In further embodiments, the affected joint is a wrist. In further embodiments, the affected joint is an elbow. In further embodiments, the affected joint is a vertebrae. In further embodiments, the affected joint is a joint proximal to a hand.

In some embodiments, the methods and compositions disclosed herein are useful for the treatment of focal osteochondral defects in an affected bone. In some embodiments, the affected bone is a femoral condyle. In some embodiments, the affected bone is a humeral head. In some embodiments, the affected bone is a talus. In some embodiments, the affected bone is a capitellum of the humerus. In some embodiments, the affected bone is an elbow. In some embodiments, the affected bone is a wrist. In some embodiments, the affected bone is a hand bone. In some embodiments, the affected bone is a toe. In some embodiments, the methods and compositions disclosed herein are useful for the treatment of a femoral head. In some embodiments, the methods and compositions disclosed herein are useful for the treatment of an acetabulum. In some embodiments, the methods and compositions disclosed herein are useful for the treatment of a tibial plateau.

In some embodiments, the affected area is a tight, pressure-filled environment. Accordingly, in some embodiments, the methods disclosed herein comprise the step of decompressing or aspirating the affected area. In some embodiments, the step of obtaining access to the affected area provides for decompression of the affected area.

In some embodiments, the compositions and methods disclosed herein are used to fill bony voids or gaps that are not intrinsic to the stability of the bony structure. Typically, these bony voids or gaps are not regions with poor biomechanical integrity.

In some embodiments, the methods disclosed herein break the biochemical communication between the joint space and the affected area of bone. In some embodiments, the methods disclosed herein provide a protective coating around the affected area of bone against inflammatory and/or non-inflammatory mediators. In some embodiments, the methods disclosed herein decrease pain associated with arthritis. In some embodiments, the methods disclosed herein slow the progression of arthritis. In some embodiments, the methods disclosed herein stop the progression of arthritis.

In some embodiments, the injectable biomaterial is administered to the affected area via a cannula. In some embodiments, the cannula is between 10 and 21 gauge. In some embodiments, the cannula has an integrated trocar to allow for penetration of the cortical bone and the formation of a fluid-tight seal in the affected area. In some embodiments, the cannula is fenestrated to allow for directional injection of the injectable biomaterial. In some embodiments, the cannula is non-fenestrated. In some cases, the cannula is designed to facilitate either directional injection of the biomaterial through a fenestrations located in the wall of the cannula or through an opening at the distal end of the cannula. In accordance with this type of system, an outer cannula and two inner cannulas are provided. The outer cannula is provided with fenestrations in the wall of the cannula and an open distal tip. The first inner cannula is provided with fenestrations in the wall of the cannula and a closed distal tip. The second inner cannula includes an opening in the distal tip and no fenestrations in the side wall of the cannula. The user can then select the first inner cannula for insertion into the outer cannula, whereby when the first inner cannula is coupled to a syringe and the contents extruded, directional injection is achieved. Alternatively, the user can select the second inner cannula for insertion into the outer cannula, whereby when the second inner cannula is coupled to a syringe and the contents extruded, injection through the distal tip is achieved. Fenestrated and non-fenestrated cannulas, such as the Ranfac Bone Marrow Aspiration and Access Needles are known in the art. In some embodiments, the cannula is steerable to minimize surgical damage due to the need to create more intrusive access into affected areas that are in difficult-to-access places. Steerable cannulas, such as the Osseon^{®} Osseoflex^{®} SN, are known in the art. In some embodiments, after injection the syringe is removed from the cannula and a rod is used to push the remaining injectable biomaterial disposed in the cannula into the affected area.

In some embodiments, the injectable biomaterial is injected without increasing post-operative intra-osseous pressure, resulting in no or minimal post-operative pain that can be associated with volume expansion from gas.

In accordance with certain aspects, the procedure produces short term pain reduction in ≤ 7 days and continue to reduce pain and prevent total joint replacement for ≥ 2 years, as measured by visual analog scored (VAS) or any other clinical accepted measure for pain and/or function.

In some embodiments, patients are required to maintain partial weight bearing and use ambulatory aids post-operatively. In some embodiments, full weight bearing is permitted post-operatively. In some embodiments, post-intervention physical therapy is required. In some embodiments, patients require routine post intervention care, observation and followup.

In some embodiments, the methods disclosed herein further comprise the application of electrical stimulation to the bone to promote bone healing.

### Kits

In another aspect, the present disclosure provides a kit comprising an injectable biomaterial and instructions for use of the same.

In some embodiments, the kit comprises two syringes. In some embodiments, a solid component is disposed in a first syringe and a liquid component is disposed in a second syringe. When mixed together, the solid component and liquid component form the injectable biomaterial. In some embodiments, at least one of the syringes in the kit comprises an integrated mixing device for *in situ* mixing of premeasured portions of ingredients from each of the first and second syringes, wherein the ingredients form the injectable biomaterial upon combination. In some embodiments, the kit comprises a Luer lock. In some embodiments, the kit comprises an end cap. In some embodiments the kit comprises a cannula. In some embodiment, the cannula comprises and inner cannula and an outer cannula. In some embodiments, at least one of the syringes is disposed in a sealed pouch to protect the contents from moisture. In some embodiments, the pouch is constructed of foil reinforced with nylon.

In some embodiments, the kit comprises bone tools. In some embodiments, the bone tools are adapted to provide a channel in the bone into which the injectable biomaterial is injected. In some embodiments, the kit comprises a bone filler to seal the open end of the channel in the bone in which the injectable biomaterial is injected. In some embodiments, the kit comprising the bone tools is distinct from the kit comprising the solid component and the liquid component.

In some embodiments, at least a portion of the kit and its contents are sterile. In some embodiments, the sterility comprises a condition in which an object has a sterility assurance level (SAL) of 10⁻³ or less. In further embodiments, the sterility comprises a condition in which an object has a SAL of 10⁻⁶ or less. In some embodiments, the SAL is determined in accordance with current FDA guidelines for medical devices. In some embodiments, the syringes are sterile.

### EXAMPLES

The following examples further describe and demonstrate embodiments within the scope of the present disclosure. The examples are given solely for the purpose of illustration and are not to be construed as limitations of the present disclosure, as many variations thereof are possible without departing from the spirit and scope of the disclosure.

### Example 1: Diagnosis of a Patient in Need of Treatment

Described herein is an exemplary diagnosis of a patient in need of treatment for bone disease according to the present disclosure.

A patient presents with pain in a joint, for example a knee joint. Pain and activity are evaluated using a clinical score such as KOOS, IKDC, and/or Tegner Lysholm Activity Scale, which reveals increased pain and decreased function relative to an unaffected joint. *See, e.g.,* Collins, N. J. et al. Arthritis Care Res. (Hoboken) 2011, 63(011), S208-228, the contents of which are incorporated herein by reference in their entirety. Conventional radiography does not reveal an obvious cause thereof. Accordingly, the patient undergoes T2 MRI to identify an area of bone degeneration, visible as an intense white area in the MRI output.

### Example 2: Method of Treating a Patient

Described herein is an exemplary method of treatment for bone disease in a patient in need thereof according to the present disclosure.

The surgical area is draped and cleaned using standard surgical protocols. The leg of the patient is abducted and a mini-fluoroscopy unit is placed so that appropriate anteroposterior and lateral views of the knee can be obtained. The appropriate starting site is identified based on the location of the affected area of bone. Cannula trajectory is determined and an incision is made in the skin of the patient at a location that allows for access to the affected area of bone.

A trocar of a bone marrow aspiration needle is used to access an area adjacent to the affected area and the tip of the bone marrow aspiration needle is inserted and punched through the remaining cortex and into or adjacent the affected area of bone. Optionally, fluoroscopy is present in the operating room to allow for verification of instrument location. Optionally, a K-wire is used to drill through the cortex to the site of injection and a cannula is placed over the K-wire. The trocar is removed from the cannula and the contents of the affected area of bone are optionally aspirated, such as by suction. Optionally, an outer cannula and two inner cannulas are provided. The outer cannula is provided with fenestrations in the wall of the cannula and an open distal tip. The first inner cannula is provided with fenestrations in the wall of the cannula and a closed distal tip. The second inner cannula includes an opening in the distal tip and no fenestrations in the side wall of the cannula. The user can then select the first inner cannula for insertion into the outer cannula, whereby when the first inner cannula is coupled to a syringe and the contents extruded, directional injection is achieved. Alternatively, the user can select the second inner cannula for insertion into the outer cannula, whereby when the second inner cannula is coupled to a syringe and the contents extruded, injection through the distal tip is achieved.

Next, a syringe comprising an injectable biomaterial according to the present disclosure is prepared and coupled to the cannula. Pressure is applied to the syringe causing the injectable biomaterial to be injected through the cannula in an amount sufficient to fill the degenerate area of bone in the affected area. Administration can be perpendicular relative to the long axis of the bone or at an angle relative to the long axis of the bone. The injectable biomaterial is optionally allowed to sit in the affected area for 5-30 minutes before removal of the cannula. During this time, the working time and/or initial setting time of the injectable biomaterial can expire. Initial setting of the injectable biomaterial can reduce clearance of the injectable biomaterial from the affected area by fluids, such as bodily fluids or fluids from surgical irrigation. During removal, additional injectable biomaterial can optionally be extruded into the space to fill the space in which the cannula was resident.

Final fluoroscopic images are obtained to confirm the appropriate location of the injected biomaterial and an arthroscope is inserted into the knee to verify that no injectable biomaterial has extravasated into the capsule (e.g., that no extrusion of the injectable biomaterial occurs into the joint space post-implantation, such as via the microcracks).

Arthroscopy is optionally used to address other correctable issues (e.g., meniscal tears, osteophytes, etc.) during the procedure. The cannula is removed from the patient and the incision is closed, such as by simple sutures.

After clinical evaluation after a period of one month, the patient shows an improvement in clinical scores, such as KOOS, IKDC, and/or Tegner Lysholm Activity Scale.

### Example 3: Exemplary Solid Components

Described herein are exemplary solid components according to the present disclosure.

### Solid Component 1

A 98.5 g batch of solid component was made as follows. Separate amounts of 83.0 g of alpha tricalcium phosphate ("α-TCP," Ca₃(PO₄)₂), 14.5 g calcium carbonate (CaCO₃), and 1.00 g calcium phosphate monobasic monohydrate ("monocalcium phosphate monohydrate," Ca(H₂PO₄)₂ H₂O) were weighed out as powders and separately dried at a temperature of at least 165 °C overnight, for at least 12 hours. The dried powders were then combined in ajar and mixed by hand shaking for 10 minutes to produce a 98.5 g batch of Solid Component 1 containing 84.3% alpha tricalcium phosphate, 14.7% calcium carbonate, and 1.02% calcium phosphate monobasic monohydrate (mass/mass).

Aliquots of the resulting solid component were then dispensed into sterile syringes comprising integrated mixing devices (Medmix Systems AG, Rotkreuz, Switzerland). Into 3 mL sterile syringes were dispensed 1.50 g of Solid Component 1. Into 14 mL sterile syringes were dispensed 4.00 g of Solid Component 1.

Particle size analysis was conducted on the component powders using a Malvern MasterSizer 2000 to ensure compliance with a set of exemplary particle size specifications as detailed in Table 1 below.

**Table 1: Exemplary Measurements of Particle Size of Solid Component Constituents**

| **Component** | **D₁₀ (µM)** | **D₉₀ (µM)** |
|---|---|---|
| α-TCP | 1.85 | 5.27 |
| CaCO₃ | 2.01 | 5.73 |
| Ca(H₂PO₄)₂ | 13.17 | 75.88 |

Particle size of alpha-TCP and calcium carbonate were measured via laser diffraction of a water dispersion. Particle size of calcium phosphate monobasic monohydrate were measured by laser diffraction of an isopropanol dispersion. Particle size measurements were conducted in accordance with USP <429>, the contents of which are incorporated herein by reference in the entirety. Particle size analysis was performed using MasterSizer 2000 software. The particle sizes measured were found to be in the acceptable range for each component.

### Solid Component 2

A 100. g batch of solid component was made as follows. Separate amounts of 83.0 g of alpha tricalcium phosphate ("α-TCP," Ca₃(PO₄)₂), 16.0 g calcium carbonate (CaCO₃), and 1.00 g calcium phosphate monobasic monohydrate ("monocalcium phosphate monohydrate," Ca(H₂PO₄)₂ H₂O) were weighed out as powders and separately dried at a temperature of at least 165 °C overnight, for at least 12 hours. The dried powders were then combined in ajar and mixed by hand shaking for 10 minutes to produce a 100. g batch of Solid Component 2 containing 83.0% alpha tricalcium phosphate, 16.0% calcium carbonate, and 1.00% calcium phosphate monobasic monohydrate (mass/mass).

Aliquots of the resulting solid component were then dispensed into sterile syringes comprising integrated mixing devices (Medmix Systems AG, Rotkreuz, Switzerland). Into 3 mL sterile syringes were dispensed 1.50 g of Solid Component 2. Into 14 mL sterile syringes were dispensed 4.00 g of Solid Component 2.

### Example 4: Exemplary Liquid Components

Described herein are exemplary liquid components according to the present disclosure.

### Control Liquid Component

A control liquid component lacking a carbohydrate was prepared by dissolving sodium phosphate dibasic in sterile water for injection to a concentration of 0.30 M. Once fully dissolved, the pH of the solution was adjusted to about pH 6 using citric acid.

Aliquots of the resulting liquid component were then dispensed into sterile syringes (Becton Dickinson, Franklin Lakes, NJ). Into 3 mL sterile syringes were dispensed 1.50 mL of the Control Liquid Component. Into 5 mL sterile syringes were dispensed 4.00 mL of the Control Liquid Component.

### Liquid Component 1

A liquid component comprising hyaluronic acid was prepared by dissolving sodium phosphate dibasic in sterile water for injection to a concentration of 0.30 M. Once fully dissolved, the pH of the solution was adjusted to about pH 6 using citric acid. Sodium hyaluronate having an average molecular weight of 0.90 × 10⁶ was added to a final concentration of 6.0 mg/mL.

Aliquots of the resulting liquid component were then dispensed into sterile syringes (Becton Dickinson, Franklin Lakes, NJ). Into 3 mL sterile syringes was dispensed 1.50 mL of the liquid component. Into 5 mL sterile syringes was dispensed 4.00 mL of the liquid component.

### Liquid Component 2

A liquid component comprising hyaluronic acid was prepared by dissolving sodium phosphate dibasic in sterile water for injection to a concentration of 0.30 M. Once fully dissolved, the pH of the solution was adjusted to about pH 6 using citric acid. Sodium hyaluronate having an average molecular weight of 1.7 × 10⁶ was added to a final concentration of 6.0 mg/mL.

Aliquots of the resulting liquid component were then dispensed into sterile syringes (Becton Dickinson, Franklin Lakes, NJ). Into 3 mL sterile syringes was dispensed 1.50 mL of the liquid component. Into 5 mL sterile syringes was dispensed 4.00 mL of the liquid component.

### Liquid Component 3

A liquid component comprising hyaluronic acid was prepared by dissolving sodium phosphate dibasic in sterile water for injection to a concentration of 0.30 M. Once fully dissolved, the pH of the solution was adjusted to about pH 6 using citric acid. Sodium hyaluronate having an average molecular weight of 2.6 × 10⁶ was added to a final concentration of 6.0 mg/mL.

Aliquots of the resulting liquid component were then dispensed into sterile syringes (Becton Dickinson, Franklin Lakes, NJ). Into 3 mL sterile syringes was dispensed 1.50 mL of the liquid component. Into 5 mL sterile syringes was dispensed 4.00 mL of the liquid component.

### Liquid Component 4

A liquid component comprising alginic acid is prepared by dissolving sodium phosphate dibasic in sterile water for injection to a concentration of 0.30 M. Once fully dissolved, the pH of the solution is adjusted to about pH 6 using citric acid. Sodium alginate (Sigma-Aldrich, St. Louis, MO) is added to a final concentration of 6.0 mg/mL.

Aliquots of the resulting liquid component are then dispensed into sterile syringes (Becton Dickinson, Franklin Lakes, NJ). Into 3 mL sterile syringes is dispensed 1.50 mL of the liquid component. Into 5 mL sterile syringes is dispensed 4.00 mL of the liquid component.

### Liquid Component 5

A liquid component comprising chitosan is prepared by dissolving sodium phosphate dibasic in sterile water for injection to a concentration of 0.30 M. Once fully dissolved, the pH of the solution is adjusted to about pH 6 using citric acid. Medium molecular weight chitosan (Sigma-Aldrich, St. Louis, MO) is added to a final concentration of 6.0 mg/mL.

Aliquots of the resulting liquid component are then dispensed into sterile syringes (Becton Dickinson, Franklin Lakes, NJ). Into 3 mL sterile syringes is dispensed 1.50 mL of the liquid component. Into 5 mL sterile syringes is dispensed 4.00 mL of the liquid component.

### Liquid Component 6

A liquid component comprising cellulose is prepared by dissolving sodium phosphate dibasic in sterile water for injection to a concentration of 0.30 M. Once fully dissolved, the pH of the solution is adjusted to about pH 6 using citric acid. Microcrystalline cellulose (Sigma-Aldrich, St. Louis, MO) is added to a final concentration of 6.0 mg/mL.

Aliquots of the resulting liquid component are then dispensed into sterile syringes (Becton Dickinson, Franklin Lakes, NJ). Into 3 mL sterile syringes is dispensed 1.50 mL of the liquid component. Into 5 mL sterile syringes is dispensed 4.00 mL of the liquid component.

### Liquid Component 7

A liquid component comprising dextran is prepared by dissolving sodium phosphate dibasic in sterile water for injection to a concentration of 0.30 M. Once fully dissolved, the pH of the solution is adjusted to about pH 6 using citric acid. Dextran from *Leuconostoc spp.* with a relative molecular weight of 450,000-650,000 (Sigma-Aldrich, St. Louis, MO) is added to a final concentration of 6.0 mg/mL.

Aliquots of the resulting liquid component are then dispensed into sterile syringes (Becton Dickinson, Franklin Lakes, NJ). Into 3 mL sterile syringes is dispensed 1.50 mL of the liquid component. Into 5 mL sterile syringes is dispensed 4.00 mL of the liquid component.

### Example 5: Preparation of Injectable Biomaterials

Described herein is the preparation of exemplary injectable biomaterials according to the present disclosure.

The injectable biomaterials indicated in Table 2 below were prepared according to the following procedure. A first syringe containing the indicated solid component described in Example 3 and comprising an integrated mixing device was coupled via Luer lock to a second syringe containing the indicated liquid component described in Example 4. The contents of the first syringe were expelled into the second syringe. The Luer lock and the second syringe were removed and an end cap was coupled to the first syringe. The integrated mixing device was actuated and the contents of the first syringe were mixed for one minute. The resultant injectable biomaterial can then be dispensed by removal of the end cap and extrusion of the contents, either directly or via a cannula or syringe coupled to the first syringe.

**Table 2: Exemplary Injectable Biomaterials**

| **Name** | **Solid Component (amount)** | **Liquid Component (amount)** |
|---|---|---|
| Control Injectable Biomaterial | 1 (1.50 g) | Control (1.50 mL) |
| Injectable Biomaterial 1 | 1 (1.50 g) | 1 (1.50 mL) |
| Injectable Biomaterial 2 | 1 (1.50 g) | 2 (1.50 mL) |
| Injectable Biomaterial 3 | 1 (1.50 g) | 3 (1.50 mL) |
| Injectable Biomaterial 4 | 1 (4.00 g) | 1 (4.00 mL) |
| Injectable Biomaterial 5 | 1 (4.00 g) | 2 (4.00 mL) |
| Injectable Biomaterial 6 | 1 (4.00 g) | 3 (4.00 mL) |

### Example 6: Comparison of Injectable Biomaterials Including and Lacking a Carbohydrate

Described herein is a comparison of exemplary injectable biomaterials according to the present disclosure with materials lacking a carbohydrate.

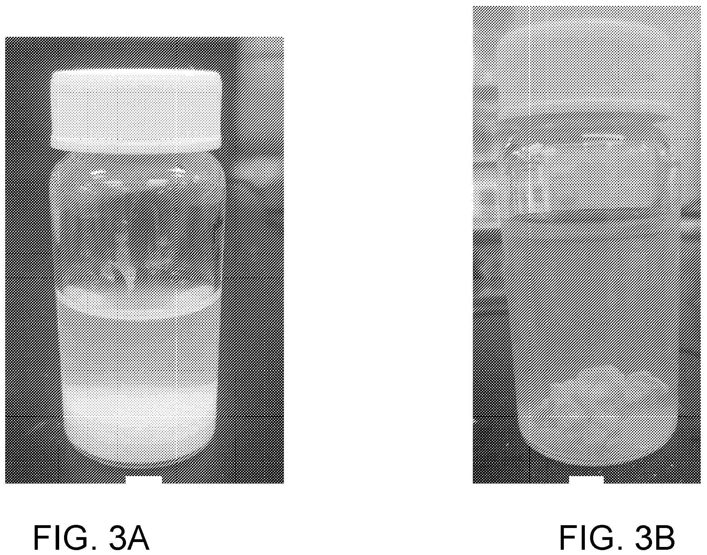

Samples of the Control Injectable Biomaterial and Injectable Biomaterial 3 were prepared as indicated in Example 5. Immediately after preparation, the syringes containing each composition were coupled to 18 gauge needles. The contents of the respective syringes were each expelled into separate vials, each containing 10.0 mL phosphate buffered saline ("PBS") at 37 °C. The vials were placed on a shaker plate at 125 rpm for 15 minutes. After removal from the shaker plate, the vials were immediately photographed as shown in FIGs. 3A-B. As shown in FIG. 3A, the Control Injectable Biomaterial (i.e., lacking a carbohydrate) produced an un-set powder mixture that was partly suspended in solution and evenly settled at the bottom of the vial. The uniform settling of the material indicates a lack of setting and cohesiveness, and indicates this material is unsuitable for treatment of degenerate bone as disclosed herein. In contrast, FIG. 3B demonstrates that an injectable biomaterial according to the present disclosure (*i.e*., Injectable Biomaterial 3) is cohesive and sets to form a material suitable for the treatment of degenerate bone as disclosed herein.

### Example 7: Second Comparison of Injectable Biomaterials Including and Lacking a Carbohydrate

Described herein is a comparison of exemplary injectable biomaterials according to the present disclosure with materials lacking a carbohydrate.

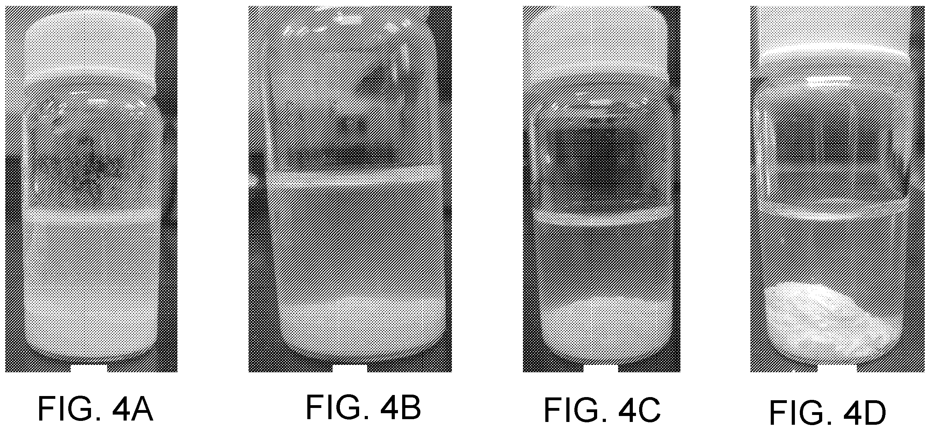

In another example comparing the properties of injectable biomaterials according to the present disclosure made using carbohydrates of varying molecular weights with materials lacking a carbohydrate, compositions of the Control Injectable Biomaterial, Injectable Biomaterial 1, Injectable Biomaterial 2, and Injectable Biomaterial 3 were separately prepared as indicated in Example 5. Immediately after preparation, the syringes contain each composition were coupled to 18 gauge needles. The contents of the respective syringes were each expelled into separate vials, each containing 10.0 mL PBS at 37 °C. The vials were placed on a shaker plate at 125 rpm for 15 minutes. After removal from the shaker plate, the vials were immediately photographed as shown in FIGs. 4A-D. As shown in FIG. 4A, the Control Injectable Biomaterial (*i.e*., lacking a carbohydrate) produces an un-set powder mixture partly suspended in solution and evenly settled at the bottom of the vial. The uniform settling of the material indicates a lack of setting and cohesiveness, and indicates this material is unsuitable for treatment of degenerate bone as disclosed herein. In contrast, FIG. 4B (Injectable Biomaterial 1), FIG. 4C (Injectable Biomaterial 2), and FIG. 4D (Injectable Biomaterial 3) demonstrate that injectable biomaterials according to the present disclosure made utilizing carbohydrates having a variety of molecular weights are cohesive and set to form materials suitable for the treatment of degenerate bone as disclosed herein.

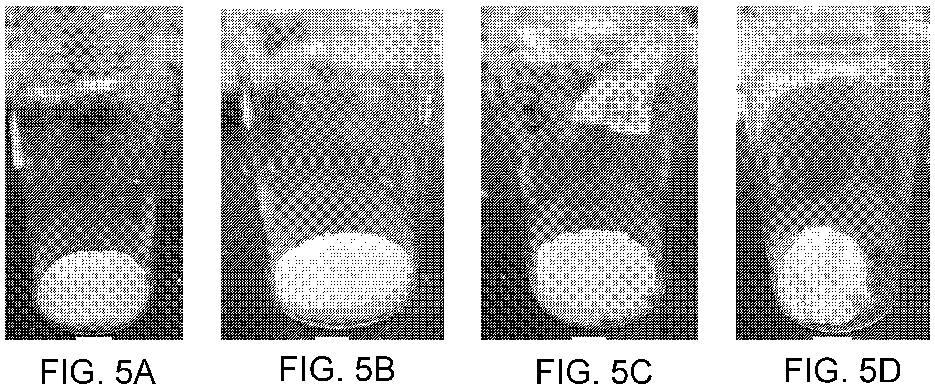

FIGs. 5A-D show the same materials as FIGs. 4A-D after removal of excess PBS by pipette, washing of the material with additional PBS (3 × 10 mL) and drying (100 °C, 3 hours). As shown in FIG. 5A, the Control Injectable Biomaterial (*i.e*., lacking a carbohydrate) produces an un-set loose powder mixture evenly distributed over the bottom of the vial. The uniform settling of the material indicates a lack of setting and cohesiveness, and indicates this material is unsuitable for treatment of degenerate bone as disclosed herein. In contrast, FIGs. 5B-D demonstrate that injectable biomaterials according to the present disclosure made utilizing carbohydrates having a range of molecular weights are cohesive and set to form materials suitable for the treatment of degenerate bone as disclosed herein.

Mass of the injectable biomaterials shown in FIGs. 4A-D and FIGs. 5A-D were measured prior to and after removal of excess liquid. The Control Injectable Biomaterial (*i.e.,* lacking a carbohydrate) shown in FIG. 4A and FIG. 5A retained only 54% of its mass, representing a considerable loss in the amount of material formed. In contrast, the injectable biomaterials according to the present disclosure shown in FIGs. 4B-D and FIGs. 5B-D retained 92%, 95% and 88% of their masses, respectively, demonstrating a much higher yield of the desired material.

### Example 8: Evaluation of Injectable Biomaterials in Sawbone

Described herein is the evaluation of exemplary injectable biomaterials according to the present disclosure when injected into sawbone as compared with materials lacking a carbohydrate. Sawbone provides a useful model for the evaluation of the performance of injectable biomaterials in patient bone as it mimics the porosity of cancellous bone. *See, e.g.,* Patel, P. S. D. et al. BMC Musculoskeletal Disorders 2008, 9, 137, the contents of which are incorporated herein in their entirety.

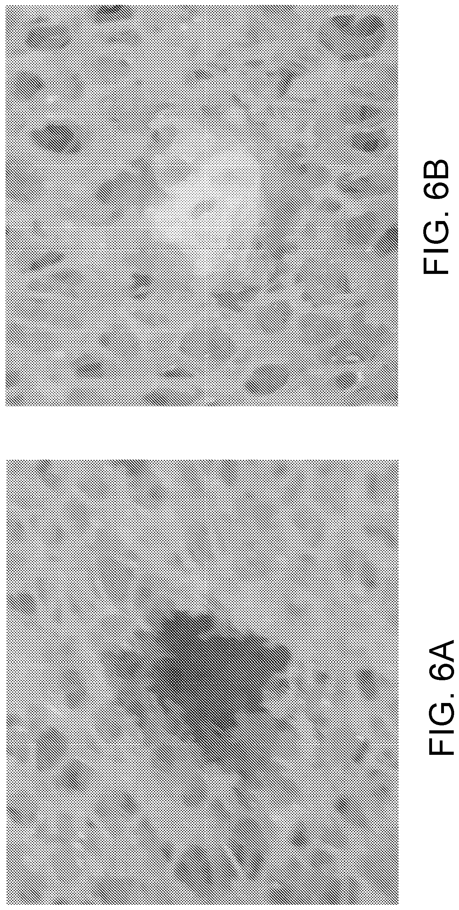

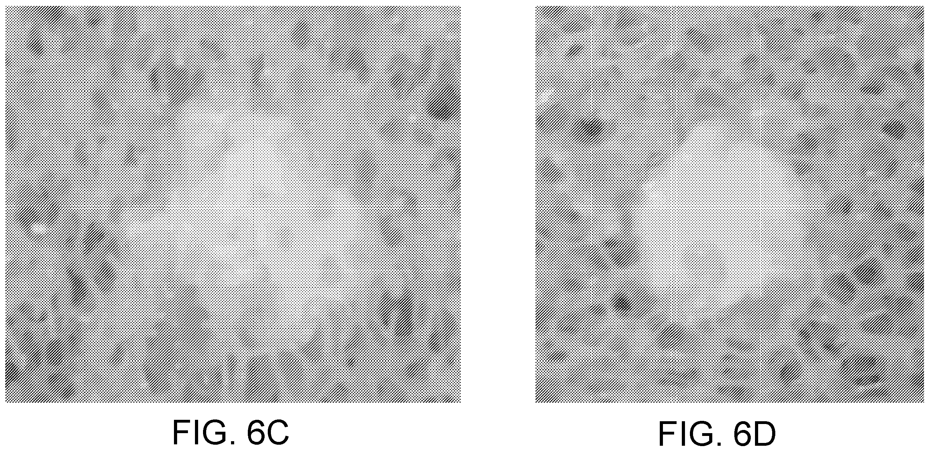

Sawbones Open Cell Block 15 PCF ( 1522-524, Pacific Research Laboratories, Vashon Island, WA) was separated into several sample blocks using a hacksaw. On a level, flat face of each sample block was drilled a hole approximately 6 mm in diameter and 12 mm deep to simulate a degenerate area of bone. Each sample block was submerged into PBS at 37 °C. 18 gauge needles were coupled to syringes containing compositions of the Control Injectable Biomaterial, Injectable Biomaterial 1, Injectable Biomaterial 2, and Injectable Biomaterial 3, separately prepared as indicated in Example 5. Each needle was inserted into the side of a sample block and into the simulated degenerate area of bone, and the compositions were injected. After sitting for 15 minutes in the heated PBS solution, the sample blocks were removed from the medium and washed with deionized water to remove cement debris, in part to mimic normal function of bodily fluids. The blocks were shaken to remove excess water, dried using compressed air and photographed as shown in FIGs. 6A-D. As shown in FIG. 6A, no perceptible amount of the Control Injectable Biomaterial (*i.e*., lacking a carbohydrate) remained and set in the defect, leaving the defect substantially unaffected. In contrast, as shown in FIGs. 6B-D, Injectable Biomaterial 1, Injectable Biomaterial 2, and Injectable Biomaterial 3 having various molecular weights demonstrated cohesion and set to substantially fill the defects in the sample blocks of sawbone. These results demonstrate the utility of the injectable biomaterials according to the present disclosure in retaining cohesiveness and adhering to a bone-like substance to effectively fill and protect a material that mimics an area of degenerate bone in a patient.

### Example 9: Second Evaluation of Injectable Biomaterials in Sawbone

Described herein is the evaluation of exemplary injectable biomaterials according to the present disclosure when injected into sawbone as compared with materials lacking a carbohydrate.

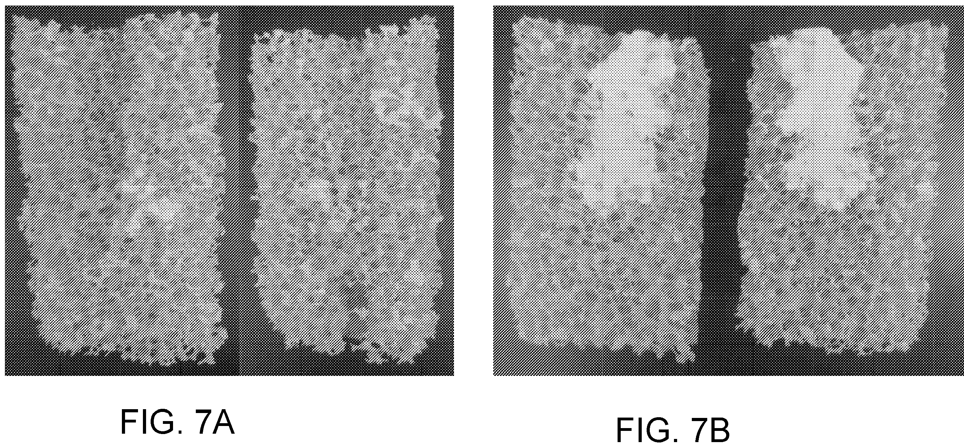

In another example comparing the properties of injectable biomaterials according to the present disclosure with materials lacking a carbohydrate, compositions of the Control Injectable Biomaterial and Injectable Biomaterial 3 were separately prepared as indicated in Example 5. Two roughly cylindrical samples of Sawbones Open Cell Block 15 PCF ( 1522-524, Pacific Research Laboratories, Vashon Island, WA) were prepared using a hacksaw. On a level, flat face of each sample block was drilled a hole approximately 6 mm in diameter and 12 mm deep to simulate a degenerate area of bone. Each sample block was submerged into PBS at 37 °C. 18 gauge needles were coupled to syringes containing compositions of the Control Injectable Biomaterial and Injectable Biomaterial 3. Each needle was inserted into the side of a sample block and into the simulated degenerate area of bone, and the compositions were injected. After sitting for 15 minutes in the heated PBS solution, the sample blocks were removed from the medium and washed with deionized water to remove cement debris, in part to mimic normal function of bodily fluids. The blocks were shaken to remove excess water, dried using compressed air, cut cross-sectionally through the simulated defect using a hacksaw, photographed as shown in FIGs. 7A-B. As shown in FIG. 7A, little of the Control Injectable Biomaterial (*i.e*., lacking a carbohydrate) remained and set in the defect, leaving the defect substantially unaffected. In contrast, as shown in FIG. 7B, Injectable Biomaterial 3, prepared according to the present disclosure, was cohesive and set to substantially fill the defect in sawbone. These results demonstrate the utility of the injectable biomaterials according to the present disclosure in retaining cohesiveness and adhering to a bone-like substance to effectively fill and protect a material that mimics an area of degenerate bone in a patient.

### Example 10: Evaluation of Diffusional Permeability of Injectable Biomaterials

Described herein is the evaluation of the diffusional permeability properties of exemplary injectable biomaterials according to the present disclosure as compared with, *inter alia,* a control composition lacking a carbohydrate.

In an *in vitro* experiment, the diffusional permeability of injectable biomaterials according to the present disclosure was compared to injectable biomaterials lacking a carbohydrate. This experiment utilized Transwells^{®} (Corning Inc., Corning, NY), two-part trays assembly that comprise a lower compartment with multiple receiving wells that couple with an upper compartment that includes corresponding wells that engage with the receiving wells but include a membrane at their base. *See* Transwell® Permeable Supports Selection and Use Guide, available at http://csmedia2.coming.com/LifeSciences/Media/pdf/transwel_guide.pdf (last visited April 24, 2017). Transwells^{®} are useful to measure the permeability test materials deposited on the membranes by filling the receiving wells with a control liquid, affixing the upper tray to the lower tray, and placing a liquid containing a solute (*e.g*., a color indicator) on top of the test materials. The amount of solute that penetrates the test materials and membranes to diffuse into the control liquid in the lower wells can then be assessed, qualitatively or quantitatively (such as by absorption spectroscopy).

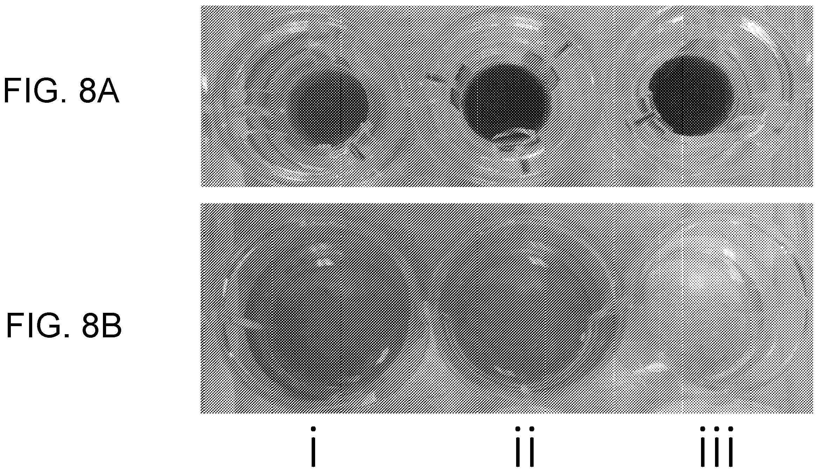

In this experiment, three permeability tests were conducted: (1) a control membrane including no injectable biomaterial (shown in column (i) of FIGs. 8A-B), (2) a membrane treated with Control Injectable Biomaterial (*i.e*., lacking a carbohydrate) (shown in column (ii) of FIGs. 8A-B), and (3) a membrane treated with Injectable Biomaterial 3 (shown in column (iii) of FIGs. 8A-B). The lower well was filled with PBS and the upper tray affixed to the lower tray. The Control Injectable Biomaterial and Injectable Biomaterial 3 were prepared as disclosed in Example 5. These materials were extruded from the syringe onto each membrane as applicable, and 1 mL of a 0.026 M (0.25 g/40 mL) solution of alizarin red was then added on top of the material in each of the upper tray wells. The tray assembly was then incubated overnight at 37 °C and photographed as shown in FIG. 8A. The upper tray was then removed and the results photographed in FIG. 8B. As shown in in FIGs. 8A-B, the dyed liquid was able to penetrate the membrane in column (i) as well as the membrane treated with Control Injectable Biomaterial in column (ii), but was substantially blocked from penetrating the membrane treated with Injectable Biomaterial 3 according to the present disclosure shown in column (iii). These results demonstrate the effectiveness of the diffusional barrier provided by the injectable biomaterials according to the present disclosure as compared with control compositions lacking a carbohydrate.

Quantification of this experiment is conducted by preparing stock solutions of alizarin red and creating a calibration curve by measuring the absorption of various dilutions at a wavelength of 450 nm. Final solutions passed through Transwells^{®} are then measured at 450 nm and correlated to a specific absorbance based on extrapolation. Results expressed as percentage permeated are expected to results demonstrate the effectiveness of the diffusional barrier provided by the injectable biomaterials according to the present disclosure as compared with control compositions lacking a carbohydrate.

### Example 11: Methods for Testing Working Time and Injectability

Described herein are tests for working time and injectability of exemplary injectable biomaterials according to the present disclosure.

Working time and injectability are assessed using procedures detailed in ASTM C414-03 at 7.2, 8.2 (reapproved 2012), the contents of which are incorporated herein by reference in their entirety as disclosed above. Briefly, approximately 0.5 oz (15 g) portions of the injectable biomaterial are extruded at desired intervals and troweled onto smooth, clean and dry horizontal surfaces. The injectable biomaterial is considered workable if it stays in the applied position without following the trowel, or without curling behind the trowel while spreading. The injectable biomaterial is no longer workable when it fails to stay in the applied position while spreading.

Working time and injectability were also tested using a 14 mL syringe of Injectable Biomaterial 6, prepared as disclosed in Example 5. After preparation, the syringe was allowed to sit for 5 minutes. The syringe was coupled to a 15 gauge cannula and the contents were able to be expelled using normal hand pressure. Required pressure for injectability is also measured using an Instron 3342.

Working time was also tested on a sample of Injectable Biomaterial 3, prepared as indicated in Example 5. The entire volume of the injectable biomaterial was extruded on a clean surface and formed into a ball. The material was considered workable when the surface was tacky to the touch of a dry gloved hand, as indicated by a visible residue present on the finger of the glove. The material was no longer considered workable when the surface was no longer tacky to the touch of a dry gloved hand, as indicated by no visible residue present on the finger of the glove. Testing for tackiness was repeated every 15 seconds. The working time was determined to be approximately 6 minutes, 15 seconds.

### Example 12: Methods for Testing Viscosity

Described herein are tests for viscosity of exemplary injectable biomaterials according to the present disclosure.

A sample of Injectable Biomaterial 1 was prepared as indicated in Example 5. The resulting material was extruded directly onto a Model AR 1000 rheometer at 25 °C. A 60 mm 1-degree stainless steel plate was used with a truncation gap of 28 µm at a sheer rate of 1 s⁻¹. Once stabilized after 10 seconds, the viscosity was recorded. The viscosity is reported as 18.69 Pa·s.

### Example 13: Methods for Testing Cohesion

Described herein is a test for cohesion of exemplary injectable biomaterials according to the present disclosure.

A sample of Injectable Biomaterial 3 was prepared as indicated in Example 5. Immediately after preparation, a syringe containing the composition was coupled to an 18 gauge needle. The contents of the syringe was expelled into a vial containing 10.0 mL PBS at 37 °C. The material was visually cohesive and did not break apart in solution.

### Example 14: Methods for Testing Initial Setting Time

Described herein is a test for initial setting time of exemplary injectable biomaterials according to the present disclosure.

Injectable Biomaterial 3 was made according to Example 5. A 14 gauge cannula was coupled to the syringe and the contents expelled onto an aluminum dish. The surface was struck off evenly using a straight-edge spatula. The remainder of the material was spread out in the mixing pan to a uniform thickness of 3/16 inch (5 mm). The pan was submerged in PBS at 37 °C for 15 minutes and then removed. After removal, a 1 lb. (454 g) Gilmore Needle, having a tip diameter of 1/24 inch (1.06 mm) penetrated the sample the length of the needle tip, in greater than 1 minute, establishing an initial setting time of no more than 15 minutes. *See* ASTM C414-03 at 7.2, 8.2 (reapproved 2012), the contents of which are incorporated herein by reference in their entirety.

Additional testing at 15 second intervals provides a more refined assessment of initial setting time.

### Example 15: Methods for Testing Extrusion Force

Described herein is a test extrusion force for exemplary injectable biomaterials according to the present disclosure.

An injectable biomaterial according to the present disclosure is prepared as disclosed herein. The material is loaded into a 10 mL syringe with an internal tip diameter of 800 mm, and the syringe is loaded into a computer-controlled extrusion force testing machine, such as a Zwick/Roell-HCr 25/400, set at a crosshead speed of 5 mm min-1. The injectable biomaterial is extruded by a compressive load vertically mounted on top of the plunger. The injectable biomaterials according to the present disclosure are expected to be completely extruded using a peak force less than about 150 N.

### Example 16: X-Ray Diffraction Testing of Phase Composition

Described herein are methods for testing the phase of exemplary injectable biomaterials according to the present disclosure. ASTM and ISO requirements mandate a minimum hydroxyapatite content of 95% of the crystalline phases and a maximum mass fraction of calcium oxide of 1% of the crystalline phases. *See* ASTM F 1185-03 (reapproved 2014), at 4.2; ISO 13175-3 (2012), at 4.2.2, the contents of all of the foregoing of which are incorporated herein by reference in their entireties.

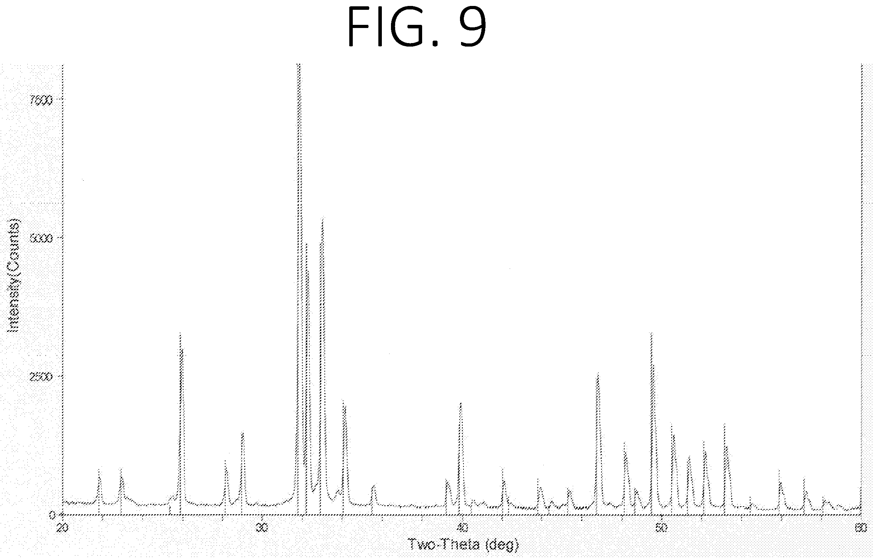

Injectable Biomaterial 3 was made according to Example 5. The sample was injected into PBS at 37 °C and allowed to sit for approximately 16 hours. The resulting solid was collected, ground in a zirconia mortar and pestle, and sintered (1 hour, 1,100 °C) before being allowed to cool to room temperature and being subjected to analysis by x-ray diffraction ("XRD") at 1.2°/min with a scan range of °2θ using a Rigaku Mini flex II desktop X-ray diffraction machine model 2005H302. As shown in FIG. 9, only crystalline phases were detected, and an absence of amorphous material. Additionally, characteristic peaks for hydroxyapatite were identified and the percentage hydroxyapatite was determined to be greater than 99%. *See* International Center for Diffraction Data ("ICDD") 9-342. The amount of calcium oxide was found to be less than 1%. *See* ICDD 4-777, the contents of which are incorporated herein by reference in their entirety.

### Example 17: FT-IR Testing of Phase Composition

Described herein are methods for testing the phase of exemplary injectable biomaterials according to the present disclosure.

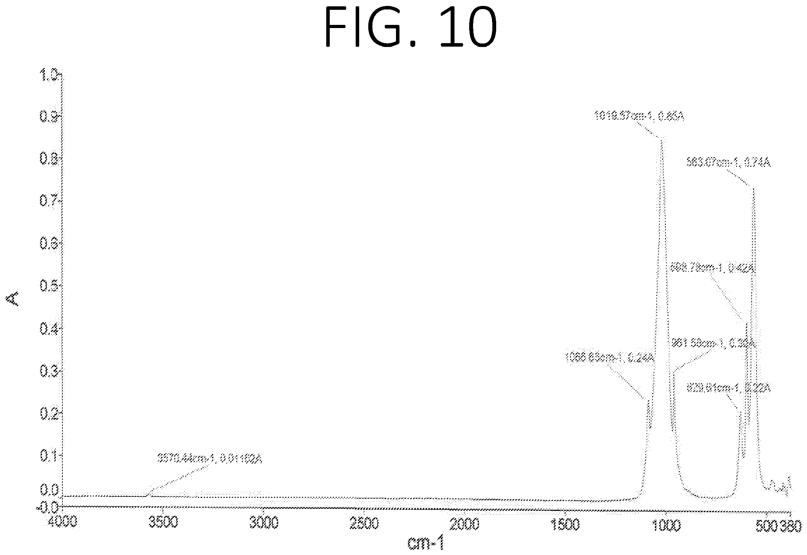

Injectable Biomaterial 3 was made according to Example 5. The sample was injected into PBS at 37 °C and allowed to sit for approximately 16 hours. The resulting solid was collected, ground in a zirconia mortar and pestle, and sintered (1 hour, 1,100 °C) before being allowed to cool to room temperature and being and subjected to FTIR. As shown in FIG. 10, characteristic bands were observed for hydroxyapatite as follows: PO₄³⁻ absorption bands at 563, 598, 961, 1019, and 1086 cm⁻¹; OH⁻ bands are present at 629 and 3570 cm⁻¹. No other bands were observed. This spectrum indicated that hydroxyapatite was formed with no other mineral phases present.

### Example 18: Scanning Electron Microscopy of Injectable Biomaterial

Described herein are scanning electron micrographs of injectable biomaterials according to the present disclosure.

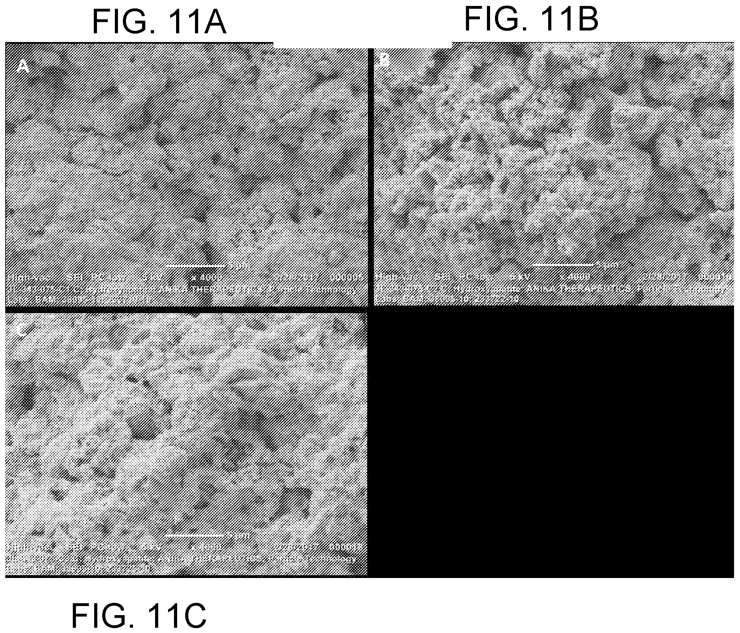

A sample of Injectable Biomaterial 3 was prepared as indicated in Example 5. The resulting material was injected into PBS at 37 °C and allowed to sit for approximately 16 hours. After drying (100 °C for 3 hours), the samples were fractured and imaged by scanning electron microscopy ("SEM"). As shown in FIGs. 11A-C, representative SEM images show nano-sized, interlocking, interconnected crystalline structures typical to that of a hydroxyapatite crystal structure.

### Example 19: Methods for Elemental Analysis

Described herein are methods for testing the elemental composition of exemplary injectable biomaterials according to the present disclosure. ASTM and ISO requirements mandate maximum content of certain elements for compositions used for bone cement. *See* ASTM F1185-03 (reapproved 2014), at 4.3; ISO 13175-3 (2012), at 4.1, the contents of all of the foregoing of which are incorporated herein by reference in their entireties.

Injectable Biomaterial 3 was made according to Example 5. The sample was injected into PBS at 37 °C and allowed to sit for approximately 16 hours. The resulting solid was collected and ground in a zirconia mortar and pestle. The material was then divided, with Sample 1 being sintered (1 hour, 1,100 °C) before being allowed to cool to room temperature, and Sample 2 not being sintered. Both samples were then subjected to analysis by ICP-MS. Triplicate tests were run for each sample to confirm the results. Results were substantially the same, and in compliance with specification, both with and without sintering. Table 3 provides the results of these experiments.

**Table 3: Results of ICP-MS Elemental Analysis of Injectable Biomaterial 3 With and Without Sintering**

| **Element** | **Specification (upper limit, ppm)** | **Sample 1 (ppm)** | | | **Sample 2 (ppm)** | | |
|---|---|---|---|---|---|---|---|
| | | **Run 1** | **Run 2** | **Run 3** | **Run 1** | **Run 2** | **Run 3** |
| Pb | 30 | 0.1 | 0.1 | 0.1 | 0.2 | 0.1 | 0.1 |
| Hg | 5 | 0.03 | 0.05 | 0.05 | 0.4 | 0.4 | 0.4 |
| As | 3 | 0.04 | 0.05 | 0.04 | 0.1 | 0.1 | 0.1 |
| Cd | 5 | 0.02 | 0.03 | 0.03 | <0.3 | <0.3 | <0.3 |

### Example 20: Methods for Testing Setting Reaction Temperature

Described herein are exemplary test methods for measuring the setting reaction temperature of injectable biomaterials according to the present disclosure.

The energetic characteristics of the setting reaction are tested on an injectable biomaterial prepared in accordance with Example 5. The resulting material is extruded into an exothermic heat mold made of polytetrafluoroethylene (PTFE), poly(ethyleneterephthalate), polyoxymethylene, high density polyethylene, or ultra-high molecular weight polyethylene (UHMWPE) and equipped with a No. 24 gage wire thermocouple, or similar device, positioned with its junction in the center of the mold at a height of 3.0 mm in the internal cavity. The plunger is immediately seated with a C-clamp or suitable press to produce the 6.0 mm specimen height. Upon producing plunger seating, excess material and the C-clamp or press are removed for the remainder of the procedure. The temperature is continuously recorded with respect to time from the onset of mixing the liquid component and the solid component until cooling is observed. The maximum temperature recorded is reported to the nearest 1°C.
At least three independent samples are tested. *See* ASTM 451-16, at 7.6, the contents of which are incorporated herein by reference in their entirety. The results are expected to indicate that the setting reaction is substantially isothermic and does not significantly change the temperature in the immediate vicinity of setting material.

### Example 21: Methods for Testing Compressive Strength

Described herein are exemplary methods for testing the compressive strength of injectable biomaterials according to the present disclosure.

The compressive strength of Injectable Biomaterial 3 prepared in accordance with Example 5 was tested. A 14 gauge cannula was coupled to the syringe and the mixture was expelled into a mold submerged in 37 °C PBS using hand pressure to produce test specimens in the shape of cylinders approximately 12 mm high and 6 mm in diameter. After 24 hours, the samples were removed and filed so that the ends of each sample were plane parallel. The samples were then subjected to compressive strength testing using an Omega Force Gauge Model DFG35-100 used to read the force of failure at 12.7 mm/minute, which provided the compressive strength. *See* ASTM F451-16, at 7.9, the contents of which are incorporated herein by reference in their entirety. A total of three compressive strength readings were recorded for each sample. The results indicated a compressive strength of 5.7 ± 1 MPa.

### Example 22: Methods for Testing Dimensional Stability

Described herein are exemplary methods for testing the dimensional stability of injectable biomaterials according to the present disclosure.

An injectable biomaterial is produced according to the Examples disclosed herein. After five minutes from the initiation of mixing have elapsed, an 14 gauge cannula is coupled to the syringe and the mixture is expelled into a mold approximately 12 mm high and 6 mm in diameter submerged in 37 °C PBS using hand pressure. After 15 minutes, the samples are ejected from the mold and their height and diameter measured using digital calipers. The samples are then submerged in 37 °C PBS for a further 24 hours after which their height and diameter is re-measured. The sample is then dried in an oven (60 °C, 24 hours). A total of three readings are recorded for each sample during the first, second, and third measurements. The results are expected to indicate no significant change in height or diameter between the first, second, and third readings, indicating high dimensional stability. The maximum change in any dimension over the three tests is expected to be less than 10%, less than 7.5%, less than 5%, or less than 3%.

### Example 23: Methods of Testing Bone Formation In Vivo

Described herein are *in vivo* tests of bone formation in rabbits using exemplary injectable biomaterials according to the present disclosure.

The skin of skeletally mature New Zealand White rabbits was opened and the periosteum reflected using a periosteal elevator in the medial aspect of the distal femur. Bilateral critical size defects (6 mm in diameter and 10 mm deep) were created using a burr with a 6 mm flat drill and controlled with a depth indicator. The medial epicondyle was used as an anatomical landmark. The defects were prepared under saline irrigation to minimize thermal damage. The defects were flushed with sterile saline during preparation and at completion to remove residual bone. The defects were filled with approximately 0.3 mL Injectable Biomaterial 6 (prepared according to Example 5) using a spatula to the height of the original cortex. The skin was closed using 3-0 Dexon. Animals were given post-operative analgesia and returned to their holding cages. The animals were free to mobilize and weight-bear post-operatively as tolerated. Animals were monitored daily, to include changes in skin, fur, eyes, and mucous membranes, as well as behavior pattern and central nervous system and somatomotor activity.

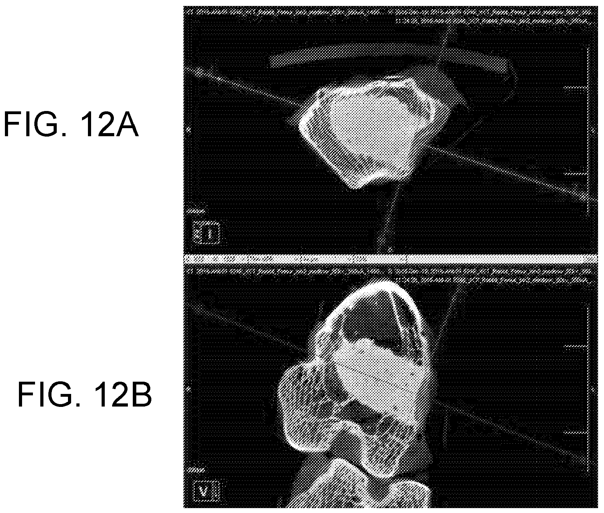

At 6-12 weeks post-implantation the animals were euthanized and the right and left femora harvested and photographed with a digital camera. The general integrity of the skin incision was examined along with the macroscopic and underlining subcutaneous tissues. Internal organs (*e.g*., heart, liver, lungs, and spleen) were excised, photographed, and preserved in 10% neutral buffered formalin (NBF) until further processing. After fixation, the internal organs were embedded, sectioned and stained with hematoxylin and eosin (H&E). The harvested femora were radiographed in the anteroposterior and lateral planes using an HP Faxitron and high resolution mammography film at 24 kV for 45 seconds. Micro-computed tomography ("micro CT") slices were also taken for representative animals using an Inveon *in vivo* micro-computer tomography scanner in order to obtain high resolution images of bone formation and test article resorption. The distal femurs were scanned and the raw images reconstructed to DICOM data using Siemens software. Images were examined in the axial, sagittal, and coronal planes to assess the overall quality of the healing sites and any local reactions. As shown in FIGs. 12A-B, the injectable biomaterial (shown in solid white) remains resident at the site of administration 6 weeks post-implantation.

Additional analysis is conducted at 6, 12, 18 and 26 weeks post-implantation to determine if new bone formation is detected by micro-computed tomography.

### Example 24: Administration of Injectable Biomaterials to Canines

Described herein are *in vivo* tests of exemplary injectable biomaterials according to the present disclosure in canines.

Animals were anesthetized by intramuscular administration of Acepromazine Maleate Injection as a pre-medication at a dose of 0.5 mg/lb. Subsequently, animals were administered Telazol^{®} (Tiletamine HCl and Zolazepam HCl, Zoetis) intramuscularly as an anesthetic at a dose of 4.5 mg/lb. to allow endotracheal tube insertion prior to isoflurane anesthesia at a rate of 1.5-2%. Finally, animals were administered buprenorphine injection intramuscularly at a dose of 1-3 µg/lb. to ensure pain free surgery. All hair around the stifle joint was shaved, and extra hair was removed from the surgical site using alcohol soaked gauze. Final surgical site cleaning was achieved using a 2% chlorhexidine/70% isopropyl preparation stick with tint to ensure coverage, starting in the middle of the surgical site and applied clockwise ever expanding circles until entire site was clean.

Two medial or lateral incisions were made to expose the fascia over the distal femur and proximal tibia. Through these incisions, and one at a time, a 15 gauge, four inch cannula was driven into the bone of either the distal femur or proximal tibia by hand pressure. Live fluoroscopic imaging confirmed appropriate placement. The trocar was removed from the cannula and a syringe containing Injectable Biomaterial 3 prepared as described in Example 5 was coupled to the cannula, followed by extrusion of the material from the syringe, through the cannula, and into the site of administration. Injection was monitored by fluoroscopy successful injection was achieved.

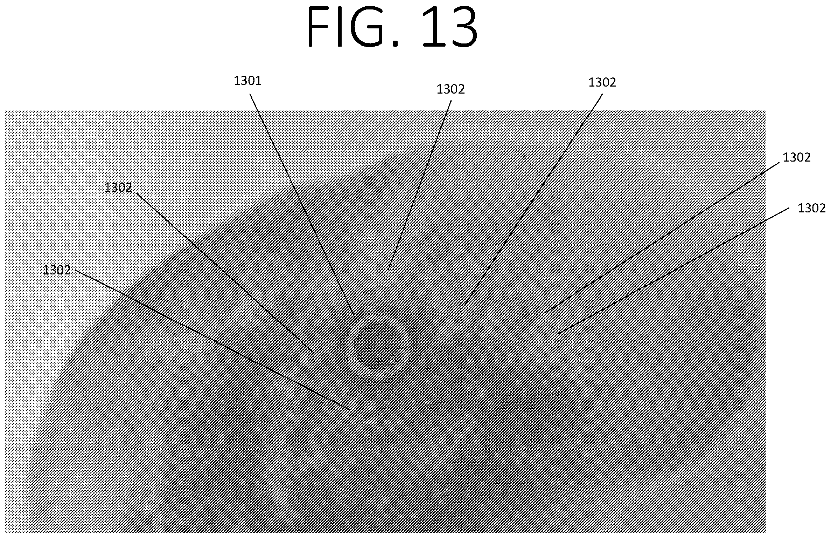

The injectable biomaterial was allowed to set for 15 minutes post-initiation of mixing. The cannula was left in place. Animals were euthanized using intravenous Euthasol^{®} at a dose of 0.1 mL/lb. The fascia were then dissected to expose the bone. Bone was be removed from the animal by sawing above and below the involved joint. The resultant specimens were set in epoxy and sectioned along the sagittal plane. As shown in FIG. 13, the cured injectable material 1302 intruded into the existing porosity of the cancellous bone in the femoral condyle. The cannula 1301 is also shown still resident at the site of administration.

### Example 25: Administration of Injectable Biomaterials to Human Cadaver Bones

Described herein are *in vivo* tests of exemplary injectable biomaterials according to the present disclosure in human cadaver bones.

Surgeries were performed on the knee joint of human cadavers. Specimens were positioned appropriately and an incision was made on the medial and lateral sides to allow insertion of a cannula into either the distal femur or tibial plateau. An 11 gauge outer cannula was driven into the cancellous bone, the trocar removed, and an inner 15 gauge cannula was inserted into the outer cannula to allow for either injection through an open distal tip or a lateral fenestration. An arthroscope was positioned in the knee to monitor for extravasation of the cement into the joint space and a fluoroscope was positioned to allow visualization of the positioning of the surgical instrumentation and injectable biomaterial.

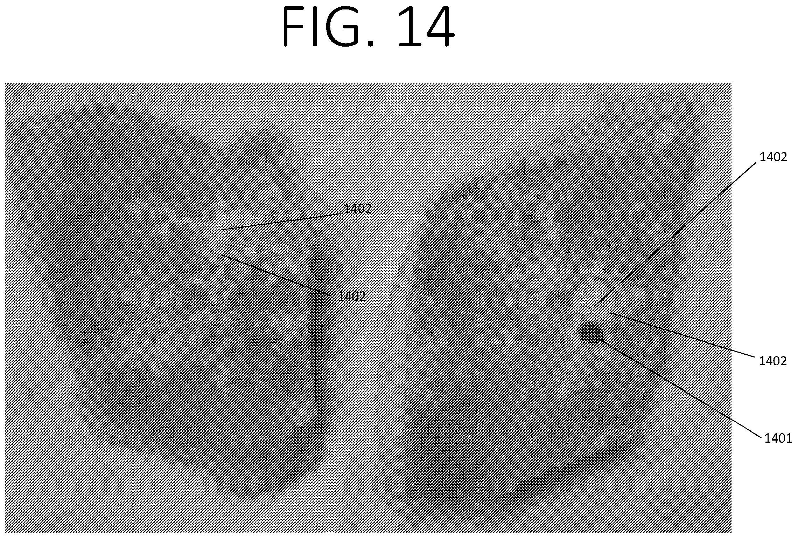

A syringe containing Injectable Biomaterial 3 prepared as described in Example 5 was coupled to the inner cannula, followed by extrusion of the material from the syringe, through the cannula, and into the site of administration using normal hand strength, with no back pressure hindering injection. No leakage of the injectable material into the joint space or from the surgical instrumentation was noted. Dissection into the cancellous space post-injection was performed. As shown in FIG. 14, the cured injectable biomaterial 1402 intruded into the existing porosity of the cancellous bone of human distal femur cadaver bone. Void 1401 shows the location in which the cannula was placed.

### INCORPORATION BY REFERENCE

The entire disclosure of each of the patent documents, including certificates of correction, patent application documents, scientific articles, governmental reports, websites, and other references referred to herein is incorporated by reference in its entirety for all purposes.

### COMBINATIONS

It is appreciated that certain features of the present disclosure, which are, for clarity, described in the context of separate embodiments, may also be provided in combination in a single embodiment.

Conversely, various features of the present disclosure, which are, for brevity, described in the context of a single embodiment, may also be provided separately or in any suitable sub-combination. All combinations of the embodiments are specifically embraced by the present disclosure and are disclosed herein just as if each and every combination was individually and explicitly disclosed. In addition, all sub-combinations listed in the embodiments describing such variables are also specifically embraced by the present disclosure and are disclosed herein just as if each and every such sub-combination of factors was individually and explicitly disclosed herein.

### EQUIVALENTS

As will be apparent to one of ordinary skill in the art from a reading of this disclosure, the present disclosure can be embodied in forms other than those specifically disclosed above without departing from the spirit or essential characteristics thereof. The particular embodiments described above are, therefore, to be considered as illustrative and not restrictive or limiting of the present disclosure. Those skilled in the art will recognize, or be able to ascertain, using no more than routine experimentation, numerous equivalents to the specific embodiments described herein. The scope of the disclosure is as set forth in the appended claims and equivalents thereof. All changes that come within the meaning and range of equivalency of the claims are intended to be embraced therein, rather than being limited to the examples contained in the foregoing description.

Aspects and features of the present disclosure are set out in the following numbered clauses which contain the subject matter of the claims of the PCT application as filed:
1. An injectable biomaterial comprising:
   (a) a solid component; and
   (b) a liquid component comprising a carbohydrate;
   wherein the injectable biomaterial sets and cures to form an apatitic crystal structure after mixing of the solid component and the liquid component.
2. A method for making the injectable biomaterial of clause 1, the method comprising:
   (a) creating the liquid component by:
      (i) providing a liquid solution;
      (ii) adjusting the pH of the liquid solution with a pH adjusting agent; and
      (iii) dissolving the carbohydrate in the liquid solution to form a the liquid component;
   (b) providing the solid component; and
   (c) mixing the liquid component and the solid component to form the injectable biomaterial.
3. The injectable biomaterial or method of any preceding clause, wherein the injectable biomaterial sets over a period of time.
4. The injectable biomaterial or method of any preceding clause, wherein the injectable biomaterial cures over a period of time.
5. The injectable biomaterial or method of any preceding clause, wherein the injectable biomaterial sets prior to completely curing.
6. The injectable biomaterial or method of any preceding clause, wherein the solid component comprises at least one of a metal phosphate and a metal carbonate.
7. The injectable biomaterial or method of any preceding clause, wherein the solid component comprises a reactive calcium phosphate.
8. The injectable biomaterial or method of any preceding clause, wherein the solid component comprises at least one of α-tricalcium phosphate (Ca₃(PO₄)₂), calcium carbonate (CaCO₃), and monocalcium phosphate monohydrate (Ca(H₂PO₄)₂ H₂O).
9. The injectable biomaterial or method of any preceding clause, wherein the solid component comprises 70-90% alpha tricalcium phosphate, 10-20% calcium carbonate, and 0.5-2% calcium phosphate monobasic monohydrate (mass/mass).
10. The injectable biomaterial or method of any preceding clause, wherein the solid component comprises 80-89% alpha tricalcium phosphate, 11-19% calcium carbonate, and 0.75-1.5% calcium phosphate monobasic monohydrate (mass/mass).
11. The injectable biomaterial or method of any preceding clause, wherein the solid component comprises 82-86% alpha tricalcium phosphate, 13-16% calcium carbonate, and 0.9-1.2% calcium phosphate monobasic monohydrate (mass/mass).
12. The injectable biomaterial or method of any preceding clause, wherein the solid component comprises 84.3% alpha tricalcium phosphate, 14.7% calcium carbonate, and 1.02% calcium phosphate monobasic monohydrate (mass/mass).
13. The injectable biomaterial or method of any preceding clause, wherein the solid component further comprises one or more ionic compound of one or more oligoelement occurring naturally in a human body.
14. The injectable biomaterial or method of clause 13, wherein the at least one ionic compound comprises a cation selected from the group consisting of Na⁺, K⁺, Mg²⁺, Ca²⁺, Sr²⁺, H⁺, and mixtures thereof.
15. The injectable biomaterial or method of clause 13, wherein the at least one ionic compound comprises an anion selected from the group consisting of PO₄³⁻, HPO₄²⁻, H₂PO₄⁻, P₂O₇⁴⁻, CO₃²⁻, HCO₃⁻, SO₄²⁻, HSO₄⁻, Cl⁻, OH⁻, F⁻, SiO₄⁴⁻, and mixtures hereof.
16. The injectable biomaterial or method of any preceding clause, wherein the liquid component further comprises a salt.
17. The injectable biomaterial or method of clause 16, wherein the salt is a metal salt.
18. The injectable biomaterial or method of any one of clauses 16-17, wherein the salt is selected from a phosphate salt, a silicate salt, a chloride salt, a hydroxide salt, and mixtures thereof.
19. The injectable biomaterial or method of any one of clauses 16-18, wherein the salt comprises at least one of sodium phosphate dibasic, sodium silicate, sodium chloride, and calcium hydroxide.
20. The injectable biomaterial or method of any preceding clause, wherein the carbohydrate is selected from the group consisting of dextran, alginate, carboxymethylcellulose, and hyaluronic acid.
21. The injectable biomaterial or method of any preceding clause, wherein the carbohydrate is hyaluronic acid, or an ester, acylurea, acyl isourea, disulfide, or amide thereof.
22. The injectable biomaterial or method of clause 21, wherein the hyaluronic acid is selected from the group consisting of hyaluronan, sodium hyaluronate, potassium hyaluronate, magnesium hyaluronate, calcium hyaluronate, ammonium hyaluronate, and combinations thereof.
23. The injectable biomaterial or method of any one of clauses 21-22, wherein the hyaluronic acid comprises at least one cross-link.
24. The injectable biomaterial or method of any one of clauses 21-23, wherein the hyaluronic acid is derived from bacteria or animals.
25. The injectable biomaterial or method of any one of clauses 21-24, wherein the hyaluronic acid comprises a sulfated hyaluronic acid, or ester, acylurea, acyl isourea, carbomer, disulfide, or amide thereof.
26. The injectable biomaterial or method of clause 25, wherein the hyaluronic acid comprises an N-sulfated hyaluronic acid, or ester, acylurea, acyl isourea, carbomer, disulfide, or amide thereof.
27. The injectable biomaterial or method of any one of clauses 21-26, wherein the hyaluronic acid comprises a hyaluronic ester.
28. The injectable biomaterial or method of clause 27, wherein the hyaluronic ester is a esterified in an amount from about 20 to 100%.
29. The injectable biomaterial or method of clause 28, wherein the non-esterified hyaluronic acid is salified with an organic or an inorganic base.
30. The injectable biomaterial or method of any preceding clause, wherein the carbohydrate is water-soluble.
31. The injectable biomaterial or method of any preceding clause, wherein the liquid component is in the form of a hydrogel.
32. The injectable biomaterial or method of any preceding clause, wherein the carbohydrate is present in the injectable biomaterial at a concentration of about 0.1 to about 100 mg/mL.
33. The injectable biomaterial or method of clause 32, wherein the carbohydrate is present in the injectable biomaterial at a concentration of about 0.1 to about 50 mg/mL.
34. The injectable biomaterial or method of clause 32, wherein the carbohydrate is present in the injectable biomaterial at a concentration of about 0.1 to about 10 mg/mL.
35. The injectable biomaterial or method of clause 32, wherein the carbohydrate is present in the injectable biomaterial at a concentration of about 1 to about 10 mg/mL.
36. The injectable biomaterial or method of clause 32, wherein the carbohydrate is present in the injectable biomaterial at a concentration of about 2 to about 10 mg/mL.
37. The injectable biomaterial or method of clause 32, wherein the carbohydrate is present in the injectable biomaterial at a concentration of about 4 to about 8 mg/mL.
38. The injectable biomaterial or method of clause 32, wherein the carbohydrate is present in the injectable biomaterial at a concentration of about 5 to about 7 mg/mL.
39. The injectable biomaterial or method of any preceding clause, wherein the carbohydrate has a molecular weight of from about 0.90 × 10⁶ Da to about 1.0 × 10⁷ Da.
40. The injectable biomaterial or method of clause 39, wherein the carbohydrate has a molecular weight of from about 0.90 × 10⁶ Da to about 5.0 × 10⁶ Da.
41. The injectable biomaterial or method of clause 39, wherein the carbohydrate has a molecular weight of from about 0.90 × 10⁶ Da to about 4.0 × 10⁶ Da.
42. The injectable biomaterial or method of clause 39, wherein the carbohydrate has a molecular weight of from about 0.90 × 10⁶ Da to about 3.0 × 10⁶ Da.
43. The injectable biomaterial or method of clause 39, wherein the carbohydrate has a molecular weight of from about 1.5 × 10⁶ Da to about 3.0 ×10⁶ Da.
44. The injectable biomaterial or method of clause 39, wherein the carbohydrate has a molecular weight of from about 1.7 × 10⁶ Da to about 2.5 × 10⁶ Da.
45. The injectable biomaterial or method of any preceding clause, wherein the carbohydrate is hyaluronic acid having a molecular weight of about 0.90 × 10⁶ Da and is present at a concentration of about 6.0 mg/mL.
46. The injectable biomaterial or method of any preceding clause, wherein the carbohydrate is hyaluronic acid having a molecular weight of about 1.7 × 10⁶ Da and is present at a concentration of about 6.0 mg/mL.
47. The injectable biomaterial or method of any preceding clause, wherein the carbohydrate is hyaluronic acid having a molecular weight of about 2.6 × 10⁶ Da and is present at a concentration of about 6.0 mg/mL.
48. The injectable biomaterial or method of any preceding clause, wherein the molecular weight of the carbohydrate is stable for at least 3 months.
49. The injectable biomaterial or method of any preceding clause, wherein the molecular weight of the carbohydrate is stable for at least 6 months.
50. The injectable biomaterial or method of any preceding clause, wherein the molecular weight of the carbohydrate is stable for at least 1 year.
51. The injectable biomaterial or method of any preceding clause, wherein the molecular weight of the carbohydrate is stable for at least 2 years.
52. The injectable biomaterial or method of any preceding clause, wherein the molecular weight of the carbohydrate is stable for at least 3 years.
53. The injectable biomaterial or method of any preceding clause, wherein the molecular weight of the carbohydrate is stable for at least 4 years.
54. The injectable biomaterial or method of any preceding clause, wherein the molecular weight of the carbohydrate is stable for at least 5 years.
55. The injectable biomaterial or method of any preceding clause, wherein the ratio of solid component to liquid component is about 3 to about 1 by mass.
56. The injectable biomaterial or method of clause 55, wherein the ratio of solid component to liquid component is about 2 to about 1 by mass.
57. The injectable biomaterial or method of clause 55, wherein the ratio of solid component to liquid component is about 1.5 to about 1 by mass.
58. The injectable biomaterial or method of clause 55, wherein the ratio of solid component to liquid component is about 1 to about 1 by mass.
59. The injectable biomaterial or method of any preceding clause, wherein the injectable biomaterial is injectable through a needle or cannula prior to initially setting.
60. The injectable biomaterial or method of clause 59, wherein the needle or cannula has a size of at least 21 gauge.
61. The injectable biomaterial or method of clause 59, wherein the needle or cannula has a size of at least 20 gauge.
62. The injectable biomaterial or method of clause 59, wherein the needle or cannula has a size of at least 18 gauge.
63. The injectable biomaterial or method of clause 59, wherein the needle or cannula has a size of at least 16 gauge.
64. The injectable biomaterial or method of clause 59, wherein the needle or cannula has a size of at least 15 gauge.
65. The injectable biomaterial or method of clause 59, wherein the needle or cannula has a size of at least 14 gauge.
66. The injectable biomaterial or method of clause 59, wherein the needle or cannula has a size of at least 12 gauge.
67. The injectable biomaterial or method of clause 59, wherein the needle or cannula has a size of at least 10 gauge.
68. The injectable biomaterial or method of any preceding clause, wherein the injectable biomaterial does not dewater when being dispensed through a needle or cannula.
69. The injectable biomaterial or method of any preceding clause, wherein the injectable biomaterial does not seize when being dispensed through a needle or cannula.
70. The injectable biomaterial or method of any preceding clause, wherein the injectable biomaterial is cohesive.
71. The injectable biomaterial or method of any preceding clause, wherein the injectable biomaterial remains cohesive during its initial setting time.
72. The injectable biomaterial or method of any preceding clause, wherein the injectable biomaterial adheres to bone.
73. The injectable biomaterial or method of any preceding clause, wherein the injectable biomaterial remains adhesive to the bone during its initial setting time.
74. The injectable biomaterial or method of any preceding clause, wherein the injectable biomaterial is workable for less than about 60 minutes after the mixing of the solid component and the liquid component.
75. The injectable biomaterial or method of clause 74, wherein the injectable biomaterial is workable for less than about 50 minutes after the mixing of the solid component and the liquid component.
76. The injectable biomaterial or method of clause 74, wherein the injectable biomaterial is workable for less than about 40 minutes after the mixing of the solid component and the liquid component.
77. The injectable biomaterial or method of clause 74, wherein the injectable biomaterial is workable for less than about 30 minutes after the mixing of the solid component and the liquid component.
78. The injectable biomaterial or method of clause 74, wherein the injectable biomaterial is workable for less than about 20 minutes after the mixing of the solid component and the liquid component.
79. The injectable biomaterial or method of clause 74, wherein the injectable biomaterial is workable for less than about 10 minutes after the mixing of the solid component and the liquid component.
80. The injectable biomaterial or method of clause 74, wherein the injectable biomaterial is workable for less than about 5 minutes after the mixing of the solid component and the liquid component.
81. The injectable biomaterial or method of clause 74, wherein the injectable biomaterial is workable for less than about 4 minutes after the mixing of the solid component and the liquid component.
82. The injectable biomaterial or method of clause 74, wherein the injectable biomaterial is workable for less than about 3 minutes after the mixing of the solid component and the liquid component.
83. The injectable biomaterial or method of clause 74, wherein the injectable biomaterial is workable for less than about 2 minutes after the mixing of the solid component and the liquid component.
84. The injectable biomaterial or method of clause 74, wherein the injectable biomaterial is workable for less than about 1 minute after the mixing of the solid component and the liquid component.
85. The injectable biomaterial or method of any preceding clause, wherein the injectable biomaterial initially sets in less than about 60 minutes after mixing the solid component and the liquid component.
86. The injectable biomaterial or method of clause 85, wherein the injectable biomaterial initially sets in less than in less than about 50 minutes after mixing the solid component and the liquid component.
87. The injectable biomaterial or method of clause 85, wherein the injectable biomaterial initially sets in less than in less than about 40 minutes after mixing the solid component and the liquid component.
88. The injectable biomaterial or method of clause 85, wherein the injectable biomaterial initially sets in less than in less than about 30 minutes after mixing the solid component and the liquid component.
89. The injectable biomaterial or method of clause 85, wherein the injectable biomaterial initially sets in less than in less than about 20 minutes after mixing the solid component and the liquid component.
90. The injectable biomaterial or method of clause 85, wherein the injectable biomaterial initially sets in less than in less than about 10 minutes after mixing the solid component and the liquid component.
91. The injectable biomaterial or method of clause 85, wherein the injectable biomaterial initially sets in less than in less than about 5 minutes after mixing the solid component and the liquid component.
92. The injectable biomaterial or method of clause 85, wherein the injectable biomaterial initially sets in less than in less than about 4 minutes after mixing the solid component and the liquid component.
93. The injectable biomaterial or method of clause 85, wherein the injectable biomaterial initially sets in less than in less than about 3 minutes after mixing the solid component and the liquid component.
94. The injectable biomaterial or method of clause 85, wherein the injectable biomaterial initially sets in less than in less than about 2 minutes after mixing the solid component and the liquid component.
95. The injectable biomaterial or method of clause 85, wherein the injectable biomaterial initially sets in less than in less than about 1 minute after mixing the solid component and the liquid component.
96. The injectable biomaterial or method of any preceding clause, wherein the injectable biomaterial cures completely in less than about 96 hours after the mixing of the solid component and the liquid component.
97. The injectable biomaterial or method of clause 96, wherein the injectable biomaterial cures completely in less than about 72 hours after the mixing of the solid component and the liquid component.
98. The injectable biomaterial or method of clause 96, wherein the injectable biomaterial cures completely in less than about 48 hours after the mixing of the solid component and the liquid component.
99. The injectable biomaterial or method of clause 96, wherein the injectable biomaterial cures completely in less than about 24 hours after the mixing of the solid component and the liquid component.
100. The injectable biomaterial or method of clause 96, wherein the injectable biomaterial cures completely in less than about 12 hours after the mixing of the solid component and the liquid component.
101. The injectable biomaterial or method of clause 96, wherein the injectable biomaterial cures completely in less than about 6 hours after the mixing of the solid component and the liquid component.
102. The injectable biomaterial or method of clause 96, wherein the injectable biomaterial cures completely in less than about 5 hours after the mixing of the solid component and the liquid component.
103. The injectable biomaterial or method of clause 96, wherein the injectable biomaterial cures completely in less than about 4 hours after the mixing of the solid component and the liquid component.
104. The injectable biomaterial or method of clause 96, wherein the injectable biomaterial cures completely in less than about 3 hours after the mixing of the solid component and the liquid component.
105. The injectable biomaterial or method of clause 96, wherein the injectable biomaterial cures completely in less than about 2 hours after the mixing of the solid component and the liquid component.
106. The injectable biomaterial or method of clause 96, wherein the injectable biomaterial cures completely in less than about 1 hour after the mixing of the solid component and the liquid component.
107. The injectable biomaterial or method of any preceding clause, wherein the initial setting and curing of the injectable biomaterial does not result in a gaseous release.
108. The injectable biomaterial or method of any preceding clause, wherein the injectable biomaterial does not significantly alter the pH of the adjacent fluids when disposed in a patient.
109. The injectable biomaterial or method of any preceding clause, wherein the initial setting curing of the injectable biomaterial does not significantly alter the temperature of the adjacent fluids when disposed in a patient.
110. The injectable biomaterial or method of any preceding clause, wherein the curing of the injectable biomaterial yields an apatitic crystal structure substantially consistent with that of hydroxyapatite.
111. The injectable biomaterial or method of any preceding clause, wherein the curing of the injectable biomaterial yields an apatitic crystal structure that is at least about 90% hydroxyapatite.
112. The injectable biomaterial or method of clause 111, wherein the curing of the injectable biomaterial yields an apatitic crystal structure that is at least about 95% hydroxyapatite.
113. The injectable biomaterial or method of clause 111, wherein the curing of the injectable biomaterial yields an apatitic crystal structure that is at least about 96% hydroxyapatite.
114. The injectable biomaterial or method of clause 111, wherein the curing of the injectable biomaterial yields an apatitic crystal structure that is at least about 97% hydroxyapatite.
115. The injectable biomaterial or method of clause 111, wherein the curing of the injectable biomaterial yields an apatitic crystal structure that is at least about 98% hydroxyapatite.
116. The injectable biomaterial or method of clause 111, wherein the curing of the injectable biomaterial yields an apatitic crystal structure that is at least about 99% hydroxyapatite.
117. The injectable biomaterial or method of clause 111, wherein the curing of the injectable biomaterial yields an apatitic crystal structure that is greater than about 99% hydroxyapatite.
118. The injectable biomaterial or method of any preceding clause, wherein the fully set and cured injectable biomaterial has a molar Ca/P ratio of about 1 to about 2.
119. The injectable biomaterial or method of clause 117, wherein the fully set and cured injectable biomaterial has a molar Ca/P ratio of about 1.3 to about 1.8.
120. The injectable biomaterial or method of clause 117, wherein the fully set and cured injectable biomaterial has a molar Ca/P ratio of about 1.4 to about 1.7.
121. The injectable biomaterial or method of clause 117, wherein the fully set and cured injectable biomaterial has a molar Ca/P ratio of about 1.5 to about 1.7.
122. The injectable biomaterial or method of clause 117, wherein the fully set and cured injectable biomaterial has a molar Ca/P ratio of about 1.5 to about 1.667.
123. The injectable biomaterial or method of any preceding clause, wherein the fully set and cured injectable biomaterial has a compressive strength of less about 20 MPa.
124. The injectable biomaterial or method of clause 123, wherein the fully set and cured injectable biomaterial has a compressive strength of less about 15 MPa.
125. The injectable biomaterial or method of clause 123, wherein the fully set and cured injectable biomaterial has a compressive strength of less about 10 MPa.
126. The injectable biomaterial or method of clause 123, wherein the fully set and cured injectable biomaterial has a compressive strength of less about 9 MPa.
127. The injectable biomaterial or method of clause 123, wherein the fully set and cured injectable biomaterial has a compressive strength of less about 8 MPa.
128. The injectable biomaterial or method of clause 123, wherein the fully set and cured injectable biomaterial has a compressive strength of less about 7 MPa.
129. The injectable biomaterial or method of clause 123, wherein the fully set and cured injectable biomaterial has a compressive strength of less about 6 MPa.
130. The injectable biomaterial or method of clause 123, wherein the fully set and cured injectable biomaterial has a compressive strength of less about 5 MPa.
131. The injectable biomaterial or method of clause 123, wherein the fully set and cured injectable biomaterial has a compressive strength of less about 4 MPa.
132. The injectable biomaterial or method of clause 123, wherein the fully set and cured injectable biomaterial has a compressive strength of less about 3 MPa.
133. The injectable biomaterial or method of clause 123, wherein the fully set and cured injectable biomaterial has a compressive strength of less about 2 MPa.
134. The injectable biomaterial or method of clause 123, wherein the fully set and cured injectable biomaterial has a compressive strength of less about 1 MPa.
135. The injectable biomaterial or method of any preceding clause, wherein the fully set and cured injectable biomaterial has an elastic modulus of less than about 5 GPa.
136. The injectable biomaterial or method of clause 133, wherein the fully set and cured injectable biomaterial has an elastic modulus of less than about 4 GPa.
137. The injectable biomaterial or method of clause 133, wherein the fully set and cured injectable biomaterial has an elastic modulus of less than about 3 GPa.
138. The injectable biomaterial or method of clause 133, wherein the fully set and cured injectable biomaterial has an elastic modulus of less than about 2 GPa.
139. The injectable biomaterial or method of clause 133, wherein the fully set and cured injectable biomaterial has an elastic modulus of less than about 1 GPa.
140. The injectable biomaterial or method of clause 133, wherein the fully set and cured injectable biomaterial has an elastic modulus of less than about 0.5 GPa.
141. The injectable biomaterial or method of clause 133, wherein the fully set and cured injectable biomaterial has an elastic modulus of less than about 0.25 GPa.
142. The injectable biomaterial of any preceding clause, wherein the injectable biomaterial has a viscosity of about 5 Pa·s and about 30 Pa·s immediately after mixing the solid component and the liquid component, when measured at room temperature.
143. The injectable biomaterial of any preceding clause, wherein the injectable biomaterial has a viscosity of about 5 Pa s and about 20 Pa s immediately after mixing the solid component and the liquid component, when measured at room temperature.
144. The injectable biomaterial of any preceding clause, wherein the injectable biomaterial has a viscosity of about 5 Pa·s and about 18 Pa·s immediately after mixing the solid component and the liquid component, when measured at room temperature.
145. The injectable biomaterial or method of any preceding clause, wherein the injectable biomaterial does not biomechanically stabilize bone.
146. The injectable biomaterial or method of any preceding clause, wherein the fully set and cured injectable biomaterial has a true density of about 1 g/cm³ to about 4 g/cm³.
147. The injectable biomaterial or method of any preceding clause, wherein the fully set and cured injectable biomaterial has a true density of about 1.5 g/cm³ to about 3.5 g/cm³.
148. The injectable biomaterial or method of any preceding clause, wherein the fully set and cured injectable biomaterial has a true density of about 1.83 g/cm³ to about 3.14 g/cm³.
149. The injectable biomaterial or method of any preceding clause, wherein the fully set and cured injectable biomaterial has a true density of about 2 g/cm³ to about 3 g/cm³.
150. The injectable biomaterial or method of any preceding clause, wherein the fully set and cured injectable biomaterial comprises a median pore diameter of less than about 1 µm.
151. The injectable biomaterial or method of any preceding clause, wherein the fully set and cured injectable biomaterial comprises a median pore diameter of less than about 0.8 µm.
152. The injectable biomaterial or method of any preceding clause, wherein the fully set and cured injectable biomaterial comprises a median pore diameter of less than about 0.6 µm.
153. The injectable biomaterial or method of any preceding clause, wherein the fully set and cured injectable biomaterial comprises a median pore diameter of less than about 0.5 µm.
154. The injectable biomaterial or method of any preceding clause, wherein the fully set and cured injectable biomaterial comprises a median pore diameter of less than about 0.4 µm.
155. The injectable biomaterial or method of any preceding clause, wherein the fully set and cured injectable biomaterial comprises a median pore diameter of less than about 0.2 µm.
156. The injectable biomaterial or method of any preceding clause, wherein the fully set and cured injectable biomaterial comprises a median pore diameter of less than about 0.15 µm.
157. The injectable biomaterial or method of any preceding clause, wherein the fully set and cured injectable biomaterial comprises a total porous area of less than about 4 m²/g.
158. The injectable biomaterial or method of any preceding clause, wherein the fully set and cured injectable biomaterial comprises a total porous area of less than about 3 m²/g.
159. The injectable biomaterial or method of any preceding clause, wherein the fully set and cured injectable biomaterial comprises a total porous area of less than about 2 m²/g.
160. The injectable biomaterial or method of any preceding clause, wherein the fully set and cured injectable biomaterial comprises a porosity sufficient to prevent diffusional passage of at least one of inflammatory mediators and non-inflammatory mediators.
161. The injectable biomaterial or method of any preceding clause, wherein the fully set and cured injectable biomaterial is osteoinductive.
162. The injectable biomaterial or method of any preceding clause, wherein the fully set and cured injectable biomaterial is osteoconductive.
163. The injectable biomaterial or method of any preceding clause wherein the fully set and cured injectable biomaterial is resorbable.
164. The injectable biomaterial or method of any preceding clause, wherein the curing of the injectable biomaterial yields less than about 5% calcium oxide.
165. The injectable biomaterial or method of clause 164, wherein the curing of the injectable biomaterial yields less than about 4% calcium oxide.
166. The injectable biomaterial or method of clause 164, wherein the curing of the injectable biomaterial yields less than about 3% calcium oxide.
167. The injectable biomaterial or method of clause 164, wherein the curing of the injectable biomaterial yields less than about 2% calcium oxide.
168. The injectable biomaterial or method of clause 164, wherein the curing of the injectable biomaterial yields less than about 1% calcium oxide.
169. The injectable biomaterial or method of any preceding clause, wherein the liquid component is sterile.
170. The injectable biomaterial or method of any preceding clause, wherein the solid component is sterile.
171. The injectable biomaterial or method of any preceding clause, wherein the injectable biomaterial is intermixable.
172. The method of any one of clauses 2-170, wherein the pH adjusting agent is selected from an organic acid and an inorganic acid.
173. The method of any one of clauses 2-172, wherein the pH adjusting agent is selected from the group consisting of citric acid, formic acid, acetic acid, and mixtures thereof.
174. The method of any one of clauses 2-173, wherein the pH adjusting agent s selected from the group consisting of hydrochloric acid, phosphoric acid, nitric acid, and mixtures thereof.
175. The method of any one of clauses 2-174, wherein providing the solid component further comprises drying the solid component.
176. The method of clause 175, wherein the drying comprises exposing the solid component to heat over a period of time.
177. The method of clause 176, wherein the heat comprises at least about 165 °C.
178. The method of clause 176, wherein the period of time comprises at least about 12 hours.
179. A method of treating an affected area of a bone in a patient in need thereof, the method comprising:
   a) identifying the affected area in the bone of the patient;
   b) creating in the bone an incision through a cortical wall of the bone to provide access to a degenerate cancellous space in the affected area of the bone;
   c) administering a volume of an injectable biomaterial of any preceding clause through the incision through the cortical wall of the bone and into the degenerate cancellous space.
180. The method of clause 179, wherein the affected area of bone is adjacent to a joint of the patient in which the patient is experiencing a joint pathology.
181. The method of any clause 179-180, wherein the joint pathology is a pathology of the knee, shoulder, ankle, elbow, wrist, hand, spine, or hip.
182. The method of any one of clauses 179-181, wherein the joint pathology is selected from the group consisting of pain, osteoarthritis, rheumatoid arthritis, avascular necrosis, and combinations thereof.
183. The method of any one of clauses 179-182, wherein the method is for the treatment of osteoarthritis in a joint of the patient.
184. The method of clause 183, wherein the osteoarthritis has a Kellgren Lawrence (KL) grade of 1-3.
185. The method of any one of clauses 179-184, wherein the joint pathology is not related to joint instability.
186. The method of any one of clauses 179-185, wherein the affected area exhibits at least one of inflammatory or degradative changes as a result of at least one of inflammatory mediators and non-inflammatory mediators.
187. The method of clause 186, wherein the at least one inflammatory mediator comprises at least one of bradykinin, histamine, prostaglandins, lactic acid, substance P, vasoactive intestinal peptide, nerve growth factor (NGF), calcitonin gene related peptide (CGRP), and mixtures thereof.
188. The method of clause 186, wherein the at least one inflammatory mediator comprises an inflammatory cytokine.
189. The method of clause 188, wherein the inflammatory cytokine is selected from the group consisting of AIMP1 (SCYE1), BMP2, CD40LG (TNFSF5), CSF1 (MCSF), CSF2 (GM-CSF), CSF3 (GCSF), FASLG (TNFSF6), GM-CSF, IFNA2, IFNG, IL-1, IL-6, IL-8, IL-15, IL-16, IL-17, IL-18, IFN-γ, LTA (TNFB), LTB, MIF, NAMPT, OSM, SPP1, TGF-β, TNF, TNF-α, TNFSF10 (TRAIL), TNFSF11 (RANKL), TNFSF13, TNFSF13B, TNFSF4 (OX40L), VEGFA, and mixtures thereof.
190. The method of clause 186, wherein the at least one non-inflammatory mediator comprises a proteolytic enzyme.
191. The method of clause 190, wherein the proteolytic enzyme is selected from the group consisting of matrix metalloproteinases (MMPs), tissue inhibitors of metalloproteinases (TIMPs), a disintegrin and metalloproteinase with thrombospondin motifs (ADAM-TS), and mixtures thereof.
192. The method of clause 186, wherein the inflammatory mediator comprises an inflammatory chemokine.
193. The method of clause 190, where the inflammatory chemokine is selected from the group consisting of C5, CCL1 (I-309), CCL11 (eotaxin), CCL13 (MCP-4), CCL15 (MIP-1d), CCL16 (HCC-4), CCL17 (TARC), CCL2 (MCP-1), CCL20 (MIP-3a), CCL22 (MDC), CCL23 (MPIF-1), CCL24 (MPIF-2, Eotaxin-2, MPIF-2, Eotaxin-2), CCL26 (eotaxin-3), CCL3 (MIP-1A), CCL4 (MIP-1B), CCL5 (RANTES), CCL7 (MCP-3), CCL8 (MCP-2), CX3CL1, CXCL1 (GRO1, GRO-alpha, SCYB1), CXCL10 (INP10), CXCL11 (I-TAC, IP-9), CXCL12 (SDF1), CXCL13, CXCL2 (GRO2, GRO-beta, SCYB2), CXCL3, CXCL5 (ENA-78, LIX), CXCL6 (GCP-2), CXCL9 (MIG), and mixtures thereof.
194. The method of clause 186, wherein the inflammatory mediator comprises an interleukin.
195. The method of clause 194, wherein the interleukin is selected from the group consisting of IL13, IL15, IL16, IL17A, IL17C, IL17F, IL1A, IL1B, IL1RN, IL21, IL27, IL3, IL33, IL5, IL7, CXCL8, IL9, and mixtures thereof.
196. The method of clause 186, wherein the inflammatory mediator comprises an inflammatory selected from the group consisting of bradykinin, calcitonin gene related peptide (CGRP), histamine, lactic acid, nerve growth factor (NGF), prostaglandins, substance P, vasoactive intestinal peptide, and mixtures thereof.
197. The method of any one of clauses 179-191, wherein the inflammatory or degradative changes are identified by MRI.
198. The method of clause 197, wherein the MRI is a T2 MRI.
199. The method of any one of clauses 186-198, wherein the inflammatory or degradative changes are disposed in cancellous bone.
200. The method of any one of clauses 179-199, wherein the affected area is disposed between about 0 inches and about 5 inches from the joint of the patient.
201. The method of any one of clauses 179-200, wherein the affected area is disposed between about 0 inches and about 4 inches from the joint of the patient.
202. The method of any one of clauses 179-201, wherein the affected area is disposed between about 0 inches and about 3 inches from the joint of the patient.
203. The method of any one of clauses 179-202, wherein the affected area is disposed between about 0 inches and about 2 inches from the joint of the patient.
204. The method of any one of clauses 179-203, wherein the affected area is disposed between about 0 inches and about 1 inch from the joint of the patient.
205. The method of any one of clauses 179-204, wherein the affected area is disposed between about 0 inches and about 20 mm from the joint of the patient.
206. The method of any one of clauses 179-205, wherein the affected area is disposed between about 0 mm and about 10 mm from the joint of the patient.
207. The method of any one of clauses 179-206, wherein the affected area is disposed between about 0 mm and about 5 mm from the joint of the patient.
208. The method of any one of clauses 179-207, wherein the affected area is disposed between about 0 mm and about 1 mm from the joint of the patient.
209. The method of any one of clauses 179-208, wherein the incision is percutaneous.
210. The method of any one of clauses 179-209, wherein providing the access to the cancellous space comprises creating a channel in the bone of the patient to couple the incision in the cortical wall of the bone to the cancellous space comprising the affected area.
211. The method of clause 210, wherein the channel is perpendicular to the long axis of the bone.
212. The method of clause 210, wherein the channel is not perpendicular to the long axis of the bone.
213. The method of any one of clauses 210-212, wherein the channel is within about 5 inches from the proximal subchondral plate.
214. The method of any one of clauses 210-213, wherein the channel is within about 4 inches from the proximal subchondral plate.
215. The method of any one of clauses 210-214, wherein the channel is within about 3 inches from the proximal subchondral plate.
216. The method of any one of clauses 210-215, wherein the channel is within about 2 inches from the proximal subchondral plate.
217. The method of any one of clauses 210-216, wherein the channel is within about 1 inches from the proximal subchondral plate.
218. The method of any one of clauses 210-217, wherein the channel is within about 20 mm of the proximal subchondral plate.
219. The method of any one of clauses 210-218, wherein the channel is within about 10 mm of the proximal subchondral plate.
220. The method of any one of clauses 210-219, wherein the channel is within about 5 mm of the proximal subchondral plate.
221. The method of any one of clauses 210-220, wherein the channel is within about 1 mm of the proximal subchondral plate.
222. The method of any one of clauses 210-221, wherein the channel is accessed by a cannula that is positioned and inserted without the need for additional targeting instrumentation.
223. The method of any one of clauses 179-222, further comprising decompressing and aspirating the contents of the affected area prior to administration of the injectable biomaterial to the affected area.
224. The method of clause 223, wherein the decompression and aspiration reduces localized inflammation in the affected area.
225. The method of clause 223, wherein the decompression and aspiration reduces intraosseous pressure in the affected area.
226. The method of clause 223, wherein the contents comprise a fluid.
227. The method of clause 223, wherein the fluid comprises at least one of inflammatory mediators and non-inflammatory mediators.
228. The method of clause 227, wherein the at least one inflammatory mediator comprises at least one of bradykinin, histamine, nerve growth factor (NGF), prostaglandins, lactic acid, substance P, vasoactive intestinal peptide, calcitonin gene related peptide (CGRP), and mixtures thereof.
229. The method of clause 227, wherein the at least one inflammatory mediator comprises an inflammatory cytokine.
230. The method of clause 229, wherein the inflammatory cytokine is selected from the group consisting of AIMP1 (SCYE1), BMP2, CD40LG (TNFSF5), CSF1 (MCSF), CSF2 (GM-CSF), CSF3 (GCSF), FASLG (TNFSF6), GM-CSF, IFNA2, IFNG, IL-1, IL-6, IL-8, IL-15, IL-16, IL-17, IL-18, IFN-γ, LTA (TNFB), LTB, MIF, NAMPT, OSM, SPP1, TGF-β, TNF, TNF-α, TNFSF10 (TRAIL), TNFSF11 (RANKL), TNFSF13, TNFSF13B, TNFSF4 (OX40L), VEGFA, and mixtures thereof.
231. The method of clause 227, wherein the at least one non-inflammatory mediator comprises a proteolytic enzyme.
232. The method of clause 231, wherein the proteolytic enzyme is selected from the group consisting of matrix metalloproteinases (MMPs), tissue inhibitors of metalloproteinases (TIMPs), a disintegrin and metalloproteinase with thrombospondin motifs (ADAM-TS), and mixtures thereof.
233. The method of clause 227, wherein the inflammatory mediator comprises an inflammatory chemokine.
234. The method of clause 233, where the inflammatory chemokine is selected from the group consisting of C5, CCL1 (I-309), CCL11 (eotaxin), CCL13 (MCP-4), CCL15 (MIP-1d), CCL16 (HCC-4), CCL17 (TARC), CCL2 (MCP-1), CCL20 (MIP-3a), CCL22 (MDC), CCL23 (MPIF-1), CCL24 (MPIF-2, Eotaxin-2, MPIF-2, Eotaxin-2), CCL26 (eotaxin-3), CCL3 (MIP-1A), CCL4 (MIP-1B), CCL5 (RANTES), CCL7 (MCP-3), CCL8 (MCP-2), CX3CL1, CXCL1 (GRO1, GRO-alpha, SCYB1), CXCL10 (INP10), CXCL11 (I-TAC, IP-9), CXCL12 (SDF1), CXCL13, CXCL2 (GRO2, GRO-beta, SCYB2), CXCL3, CXCL5 (ENA-78, LIX), CXCL6 (GCP-2), CXCL9 (MIG), and mixtures thereof.
235. The method of clause 227, wherein the inflammatory mediator comprises an interleukin.
236. The method of clause 235, wherein the interleukin is selected from the group consisting of IL13, IL15, IL16, IL17A, IL17C, IL17F, IL1A, IL1B, IL1RN, IL21, IL27, IL3, IL33, IL5, IL7, CXCL8, IL9, and mixtures thereof.
237. The method of any one of clauses 179-236 wherein the injectable biomaterial is administered through a cannula or needle.
238. The method of clause 237, wherein the needle or cannula has a size of at least 21 gauge.
239. The method of clause 237, wherein the needle or cannula has a size of at least 20 gauge.
240. The method of clause 237, wherein the needle or cannula has a size of at least 18 gauge.
241. The method of clause 237, wherein the needle or cannula has a size of at least 16 gauge.
242. The method of clause 237, wherein the needle or cannula has a size of at least 15 gauge.
243. The method of clause 237, wherein the needle or cannula has a size of at least 14 gauge.
244. The method of clause 237, wherein the needle or cannula has a size of at least 12 gauge.
245. The method of clause 237, wherein the needle or cannula has a size of at least 10 gauge.
246. The method of clause 237, wherein the injectable biomaterial does not dewater when being dispensed through the needle or cannula.
247. The method of clause 237, wherein the injectable biomaterial does not seize when being dispensed through the needle or cannula.
248. The method of clause 237, wherein the injectable biomaterial is administered through a steerable cannula to minimize surgical damage.
249. The method of any one of clauses 179-248, wherein the injectable biomaterial is injected into the affected area while minimally disrupting the subchondral plate.
250. The method of any one of clauses 179-249, wherein the injectable biomaterial is injected into a layer between about 0 mm and about 20 mm above or below the affected area while minimally disrupting the subchondral plate.
251. The method of any one of clauses 179-250, wherein the injectable biomaterial is injected into a layer between about 0 mm and about 10 mm above or below the affected area while minimally disrupting the subchondral plate.
252. The method of any one of clauses 179-251, wherein the injectable biomaterial is injected into a layer between about 0 mm and about 5 mm above or below the affected area while minimally disrupting the subchondral plate.
253. The method of any one of clauses 179-252, wherein the injectable biomaterial is injected into a layer between about 0 mm and about 1 mm above or below the affected area while minimally disrupting the subchondral plate.
254. The method of any one of clauses 179-253, wherein the injectable biomaterial is administered to an area that is not intrinsic to the structural stability of the bone.
255. The method of any one of clauses 179-254, further comprising arthroscopically examining the joint space post-injection to ensure an absence of the injectable biomaterial in the joint.
256. The method of any one of clauses 179-255, wherein the injectable biomaterial flows into the porosity of cancellous bone during administration into the affected area.
257. The method of any one of clauses 179-256, wherein the injectable biomaterial remains cohesive and substantially fills bone voids during administration into the affected area.
258. The method of any one of clauses 179-257, wherein the injectable biomaterial at least partially coats the interface between the cancellous space and an adj acent joint to provide a protective layer upon setting.
259. The method of any one of clauses 179-258, wherein the injectable biomaterial prevents diffusional passage of at least one of inflammatory mediators and non-inflammatory mediators from the adj acent joint space into the affected area.
260. The method of clause 258, wherein the protective layer provides a sacrificial layer for osteoclasts to consume during bone remodeling.
261. The method of any one of clauses 179-260, wherein the administration of the injectable biomaterial does not cause stress shielding resulting in the weakening of the unloaded bone.
262. The method of any one of clauses 179-261, wherein the method does not cause substantial post-operative pain.
263. The method of any one of clauses 179-262, wherein the method decreases pain in the joint.
264. The method of any one of clauses 179-263, wherein the method slows the progression of osteoarthritis in the joint.
265. The method of any one of clauses 179-264, wherein the method is for the treatment of rheumatoid arthritis in a joint of the patient.
266. The method of any one of clauses 179-265, wherein the method slows the progression of rheumatoid arthritis in the joint.
267. The method of any one of clauses 179-266, wherein the method slows the progression of avascular necrosis in the joint.
268. A kit comprising:
   (a) the solid component and the liquid component for preparing the injectable biomaterial of any one of clauses 1-178; and
   (b) instructions for use of the same.
269. The kit of clause 268, wherein the instructions are for a method of treating an affected area of a bone in a patient in need thereof.
270. The kit of clause 268, wherein the treatment is for pain, osteoarthritis, rheumatoid arthritis, avascular necrosis, or combinations thereof.
271. The kit of any one of clauses 268-270, wherein the solid component and the liquid component are disposed in separate sterile containers.
272. The kit of any one of clauses 268-271, wherein the packaging configuration allows the solid component and the liquid component to remain stable at 2 °C - 25 °C for at least about three months.
273. The kit of any one of clauses 268-272, wherein the packaging configuration allows the solid component and the liquid component to remain stable at 2 °C - 25 °C for at least about six months.
274. The kit of clause 272, wherein the packaging configuration allows the solid component and the liquid component to remain stable at 2 °C - 25 °C for at least about one year.
275. The kit of clause 272, wherein the packaging configuration allows the solid component and the liquid component to remain stable at 2 °C - 25 °C for at least about two years.
276. The kit of clause 272, wherein the packaging configuration allows the solid component and the liquid component to remain stable at 2 °C - 25 °C for at least about three years.
277. The kit of clause 272, wherein the packaging configuration allows the solid component and the liquid component to remain stable at 2 °C - 25 °C for at least about four years.
278. The kit of clause 272, wherein the packaging configuration allows the solid component and the liquid component to remain stable at 2 °C - 25 °C for at least about five years.
279. The kit of any one of clauses 268-278, wherein the liquid component is disposed in a sterile syringe.
280. The kit of any one of clauses 268-279, wherein the solid component is disposed in a syringe possessing an integrated mixing device for *in situ* mixing of premeasured portions of the solid component and the liquid component to form the injectable biomaterial.
281. The kit of clause 280, wherein the syringe is sterile.
282. The kit of any one of clauses 268-281, further comprising a Luer-Lock.
283. The kit of any one of clauses 268-282, further comprising an end cap.

## Claims

1. An injectable biomaterial comprising:
a. a solid component; and
b. a liquid component comprising a carbohydrate;
wherein the injectable biomaterial sets and cures to form an apatitic crystal structure after mixing of the solid component and the liquid component;
wherein, the ratio of solid component to liquid component is i) about 1 to about 1 by mass, ii) about 1.5 to about 1 by mass, iii) about 2 to about 1 by mass, or iv) about 3 to about 1 by mass; and
wherein the fully set and cured injectable biomaterial comprises a median pore diameter of less than about 1 µm.

2. A method for making the injectable biomaterial of claim 1, the method comprising:
a. creating the liquid component by:
i. providing a liquid solution;
ii. adjusting the pH of the liquid solution with a pH adjusting agent; and
iii. dissolving the carbohydrate in the liquid solution to form the liquid component;
b. providing the solid component; and
c. mixing the liquid component and the solid component to form the injectable biomaterial.

3. The injectable biomaterial or method of any preceding claim, wherein the solid component comprises at least one of a metal phosphate and a metal carbonate.

4. The injectable biomaterial or method of any preceding claim, wherein the solid component comprises a reactive calcium phosphate.

5. The injectable biomaterial or method of any preceding claim, wherein the solid component comprises at least one of α-tricalcium phosphate (Ca₃(PO₄)₂), calcium carbonate (CaCO₃), and monocalcium phosphate monohydrate (Ca(H₂PO₄)₂ H₂O).

6. The injectable biomaterial or method of any preceding claim, wherein the solid component comprises 70-90% alpha tricalcium phosphate, 10-20% calcium carbonate, and 0.5-2% calcium phosphate monobasic monohydrate (mass/mass).

7. The injectable biomaterial or method of any preceding claim, wherein the carbohydrate is hyaluronic acid, or an ester, acylurea, acyl isourea, disulfide, or amide thereof.

8. The injectable biomaterial or method of any preceding claim, wherein the carbohydrate is sodium hyaluronate.

9. The injectable biomaterial or method of any preceding claim, wherein the fully set and cured injectable biomaterial has a molar Ca/P ratio of about 1 to about 2, about 1.3 to about 1.8, about 1.4 to about 1.7, about 1.5 to about 1.7, or about 1.5 to about 1.667.

10. The injectable biomaterial or method of any preceding claim, wherein the fully set and cured injectable biomaterial has a compressive strength of less about 20 MPa, 10 MPA, 5MPa, or 1 MPa.

11. The injectable biomaterial or method of any preceding claim, wherein the carbohydrate is present in the injectable biomaterial at a concentration of about 0.1 to about 100 mg/mL, about 0.1 to about 50 mg/mL, about 0.1 to about 10 mg/mL, about 1 to about 10 mg/mL, about 2 to about 10 mg/mL, about 4 to about 8 mg/mL, or about 5 to about 7 mg/mL.

12. The injectable biomaterial or method of any preceding claim, wherein the carbohydrate has a molecular weight of from about 0.90 × 10⁶ Da to about 1.0 × 10⁷ Da, from about 0.90 × 10⁶ Da to about 5.0 × 10⁶ Da, from about 0.90 × 10⁶ Da to about 4.0 × 10⁶ Da, from about 0.90 × 10⁶ Da to about 3.0 × 10⁶ Da, from about 0.90 × 10⁶ Da to about 3.0 × 10⁶ Da, from about 1.7 × 10⁶ Da to about 2.50 × 10⁶ Da.

13. The injectable biomaterial or method of any preceding claim, wherein the carbohydrate is hyaluronic acid having a molecular weight of about 0.90 × 10⁶ Da and is present at a concentration of about 6.0 mg/mL, of about 1.7 × 10⁶ Da and is present at a concentration of about 6.0 mg/mL, of about 2.6 × 10⁶ Da and is present at a concentration of about 6.0 mg/mL.

14. The injectable biomaterial or method of any preceding claim, wherein the injectable biomaterial is injectable through a needle or cannula prior to initially setting, wherein the needle or cannula has a size of at least 21 gauge, at least 20 gauge, at least 15 gauge, at least 12 gauge, or at least 10 gauge.

15. A kit comprising:
a. The solid component and the liquid component for preparing the injectable biomaterial of any one of claims 1 to 14; and
b. instructions for use of the same.
